(19) Europäisches Patentamt
European Patent Office
Office européen des brevets

(11) **EP 4 061 768 B1**

(12) **EUROPÄISCHE PATENTSCHRIFT**

(45) Veröffentlichungstag und Bekanntmachung des Hinweises auf die Patenterteilung:
**06.08.2025 Patentblatt 2025/32**

(21) Anmeldenummer: **20824091.1**

(22) Anmeldetag: **23.11.2020**

(51) Internationale Patentklassifikation (IPC):
***C01B 25/41*** (2006.01) ***A23L 31/15*** (2016.01)
***C12N 1/18*** (2006.01) ***A23L 33/16*** (2016.01)
***C12N 1/06*** (2006.01) ***C12P 3/00*** (2006.01)

(52) Gemeinsame Patentklassifikation (CPC):
**C01B 25/41; A23L 31/15; A23L 33/16; C12N 1/063; C12N 1/18; C12P 3/00**

(86) Internationale Anmeldenummer:
**PCT/EP2020/083067**

(87) Internationale Veröffentlichungsnummer:
**WO 2021/099636 (27.05.2021 Gazette 2021/21)**

(54) **ZUSAMMENSETZUNG, ENTHALTEND GETROCKNETES POLYPHOSPHAT UND VERFAHREN ZUR GEWINNUNG VON POLYPHOSPHAT AUS POLYPHOSPHAT-HALTIGEN HEFEZELLEN DAZU**

COMPOSITION CONTAINING DRIED POLYPHOSPHATE AND METHODS FOR OBTAINING POLYPHOSPHATE FROM POLYPHOSPHATE-CONTAINING YEAST CELLS

COMPOSITION CONTENANT DU POLYPHOSPHATE SÉCHÉ ET PROCÉDÉS DE PRODUCTION DE POLYPHOSPHATE À PARTIR DE CELLULES DE LEVURE CONTENANT DU POLYPHOSPHATE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.11.2019 DE 102019131561**

(43) Veröffentlichungstag der Anmeldung:
**28.09.2022 Patentblatt 2022/39**

(73) Patentinhaber: **Rheinisch-Westfälische Technische Hochschule (RWTH) Aachen
52062 Aachen (DE)**

(72) Erfinder:
- **CHRIST, Jonas Johannes
52074 Aachen (DE)**
- **BLANK, Lars Mathias
44227 Dortmund (DE)**

(74) Vertreter: **Schiweck Weinzierl Koch Patentanwälte Partnerschaft mbB
Ganghoferstraße 68 B
80339 München (DE)**

(56) Entgegenhaltungen:
**WO-A2-2007/076444 GB-A- 1 161 693
US-A- 4 581 057**

- **E. LISS ET AL: "Versuche zur Polyphosphat-Überkompensation in Hefezellen nach Phosphatverarmung", ARCHIV FÜR MIKROBIOLOGIE, vol. 41, no. 4, 1 December 1962 (1962-12-01), DE, pages 383 - 392, XP055670007, ISSN: 0003-9276, DOI: 10.1007/BF00422195**
- **JONAS JOHANNES CHRIST ET AL: "Saccharomyces cerevisiae containing 28% polyphosphate and production of a polyphosphate-rich yeast extract thereof", FEMS YEAST RESEARCH, vol. 19, no. 3, 1 May 2019 (2019-05-01), GB, NL, pages 1 - 13, XP055669733, ISSN: 1567-1356, DOI: 10.1093/femsyr/foz011**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



**(Forts. nächste Seite)**

- **JONAS JOHANNES CHRIST ET AL: "Biotechnological synthesis of water-soluble food-grade polyphosphate with Saccharomyces cerevisiae", BIOTECHNOLOGY AND BIOENGINEERING, vol. 117, no. 7, 4 April 2020 (2020-04-04), US, pages 2089 - 2099, XP055771646, ISSN: 0006-3592, DOI: 10.1002/bit.27337**

## Beschreibung

### Technisches Gebiet

Die vorliegende Erfindung bezieht sich auf Verfahren zur Gewinnung von Polyphosphat aus polyphosphathaltigen Hefezellen sowie auf Zusammensetzung, enthaltend getrocknetes Polyphosphat. Insbesondere bezieht sich die vorliegende Erfindung auf neuartige Verfahren zur biotechnologischen Herstellung von wasserlöslichem, lebensmitteltauglichem Polyphosphat mit hohem Reinheitsgrad, enthaltend eine Reinigung des Bio-Polyphosphats aus den Zellen der Backhefe (z.B., *S. cerevisiae,* insbesondere Hefemasse mit hohem Polyphosphatanteil), die das Orthophosphat ansammeln und es intrazellulär zu Polyphosphat polymerisieren. Zudem umfasst die vorliegende Erfindung getrocknetes Polyphosphat (insbesondere in Pulverform), erhältlich durch die erfindungsgemäßen Verfahren.

Des Weiteren bezieht sich die vorliegende Erfindung auf ein Verfahren zur Herstellung einer Hefemasse mit hohem Polyphosphatanteil von mindestens etwa 5 Gew%, vorzugsweise von mindestens 25 Gew% bezogen auf die Trockenzellmasse (hier auch "Zelltrockengewicht" genannt), umfassend folgende Schritte: (1) Fermentieren von Hefe in einem Orthophosphat-Mangelmedium für etwa 4 bis 9 h bei etwa 23 bis 32 °C bei einem pH von etwa 4 bis 6; (2) Trocknung der Zellen; (3) optional Lagerung der Zellen bis etwa 24 h bei etwa 2 bis 10 °C; (4) Überführung der Zellen in ein Orthophosphat-haltiges Medium und Fermentieren für etwa 1,0 bis 3,5 h bei etwa 23 bis 32 °C bei einem pH von etwa 4 bis 7, und (5) Trocknung der Zellen, wobei das Polyphosphat (PolyP) als $KPO_3$ gemessen wird, vorzugsweise enthält das Orthophosphat-Mangelmedium: etwa 30 bis 300 mM Glucose, etwa 5 bis 50 mM $(NH_4)SO_4$, etwa 5 bis 30 mM KCl, etwa 5 bis 30 mM $Na_2$Succinat, etwa 2 bis 10 mM $CaCl_2$, Vitaminlösung umfassend ein oder mehrere Vitamine ausgewählt aus der Gruppe bestehend aus Myo-inositol, Nicotinsäure, Pyridoxalhydrochlorid, Thiaminhydrochlorid, Ca-D-pantothenat, P-Aminobenzoesäure, und D-Biotin und Spurenelementelösung umfassend Spurenelemente ausgewählt aus der Gruppe bestehend aus $Na_2EDTA$, $CaCl_2$, $H_3BO_3$, $ZnSO_4$, $FeSO_4$, $MnCl_2$, $Na_2MoO_4$, $CoCl_2$, $CuSO_4$, und KI; noch mehr bevorzugt enthält das Orthophosphat-Mangelmedium: etwa 133 mM Glucose, etwa 10 mM $(NH_4)SO_4$, etwa 13 mM KCl, etwa 12,5 mM $Na_2$Succinat, etwa 4 mM $CaCl_2$, Vitaminlösung umfassend Myo-inositol, Nicotinsäure, Pyridoxalhydrochlorid, Thiaminhydrochlorid, Ca-D-pantothenat, P-Aminobenzoesäure, und D-Biotin, und Spurenelementelösung umfassend $Na_2EDTA$, $CaCl_2$, $H_3BO_3$, $ZnSO_4$, $FeSO_4$, $MnCl_2$, $Na_2MoO_4$, $CoCl_2$, $CuSO_4$, und KI.

### Stand der Technik

Anorganisches Polyphosphat (PolyP) ist das lineare Polymer von Orthophosphat ($P_i$). Sowohl PolyP als auch $P_i$ werden oft als Nahrungsmittelzusätze verwendet. Die Nahrungseigenschaften von PolyP gehen einher mit seinen chemischen Eigenschaften: es enthält energiereiche Phosphoanhydrid-Bindungen (z.B. Stabilisierung und Zartmachung von Fleischprodukten wie Wurst), fungiert als löslicher Kation-Austauscher (z.B. Komplexierung bivalenter Kationen, um Weichkäse seine einzigartige Viskosität zu verleihen), seine starke Hydrophilie (z.B. um Fleischprodukte saftig zu halten), seine bakteriostatische Wirkung (z.B. in Döner Kebab, bei dem das Fleisch für längere Zeit bei Umgebungstemperatur gehalten wird), puffert den pH, ist nicht toxisch für Menschen und ist biologisch abbaubar (Kulaev et al., 2nd Ed. John Wiley & Sons, Ltd., West Sussex, England (2005)).

Da jeweils nur die erste und letzte P-Untereinheit jeder PolyP-Kette eine Hydroxylgruppe mit puffernden Eigenschaften bei neutralem pH besitzt, wird $P_i$ zur pH-Stabilisierung in Nahrungsprodukten bevorzugt. $PolyP_2$ findet in Fleischprodukten eine einzigartige Anwendung. Myosin ist der primäre wasser-, protein-, und fettbindende Stoff in Muskelprodukten. In Fleisch ohne $PolyP_2$-Zusatz hat Myosin verringerte Funktionalität, da es innerhalb der dicken Filamente der Myofibrillen gebunden ist. Innerhalb der Muskelmatrix muss Myosin umstrukturiert und/oder freigesetzt werden, um geeignete Funktionalität zu erhalten (d.h. zartes Fleisch). Dies wird durch Zugabe oder Injektion von $PolyP_2$ erreicht (Shen et al., Meat Science (2010), 84: 364). $PolyP_2$ erleichtert dann die Extraktion von Myosin aus dem dicken Filament des A-Bandes. Das Ergebnis ist eine Schwellung der Myofibrillen und eine erhöhte Wasserspeicherkapazität des Fleisches. Zusammenfassend besitzt $P_i$ die höchste pH-Pufferfähigkeit, welche mit zunehmender PolyP-Kettenlänge abnimmt. Im Gegensatz dazu nehmen Eigenschaften wie Komplexierungsfähigkeit, Anzahl der Phosphoanhydrid-Bindungen, bakteriostatische Eigenschaften sowie Hydrophilie mit wachsender PolyP-Kettenlänge zu. Die Aktivierung von Myosin in Fleischprodukten ist mit $PolyP_2$ am ausgeprägtesten.

PolyP ist aus dem Stand der Technik bekannt (Liss und Langen, 1962, Archiv für Mikrobiologie, 41, 383-392; GB1161693; Christ und Blank, FEMS Yeast Research 2019). Derzeit wird PolyP für die Nahrungsmittelindustrie chemisch durch hitze-induzierte Kondensationsreaktion von $P_i$ synthetisiert. Allerdings erlaubt die chemische Synthese von PolyP nur Kettenlängen von bis zu 40 P-Untereinheiten (Christ & Blank, Anal Biochem (2018), 548: 82-90, *inter alia* Tabelle 4 darin). Um die Eigenschaften längerkettiger PolyP-Polymere in Nahrungsmitteln nutzen zu können, müssen daher mitunter hohe Konzentrationen chemisch synthetisierten PolyP eingesetzt werden, was eine höhere P-Aufnahme durch die Konsumenten mit sich bringt. Außerdem ist hochreines $P_i$ für die chemische Synthese von PolyP notwendig. Dieses hochreine $P_i$ wird von geologischen Phosphaterz Ablagerungen gewonnen, die vor allem in Marokko und der West-Sahara vorkommen. Der Abbau benötigt große Mengen Schwefelsäure und Wasser und produziert erhebliche Mengen Abfall. Wasserlösliches Natrium-PolyP mit einer durchschnittlichen Kettenlänge von 2 bis 40 P-Untereinheiten ist als Lebensmittelzusatzstoffe weit verbreitet und wird derzeit chemisch synthetisiert. Jedoch müssen Lebensmittel mit zugesetztem chemisch synthetisiertem PolyP gekennzeichnet werden.

Zusammenfassend lässt sich feststellen, dass die chemische Synthese von PolyP sehr energieaufwendig ist. Der Ausgangsstoff hierfür nicht unendlich verfügbar ist und zudem ist dessen Gewinnung wiederum energieaufwendig, wenig Ressourcen schonend und überdies zum Teil wenig nachhaltig, ja sogar teilweise umweltschädlich. Insbesondere die Lebensmittel hat einen großen Bedarf an PolyP, der gedeckt werden muss. Wünschenswert wäre es, wenn man PolyP, idealerweise in reiner Form, auf eine andere Art und Weise bereitstellen könnte, die eben nicht aus industrieller Herstellung stammt.

Von daher besteht ein Verlangen, PolyP auf eine alternative Art und Weise bereitzustellen. Es ist die Aufgabe der vorliegenden Erfindung, dieses Verlangen zu befriedigen, indem PolyP auf eine alternative Art und Weise bereitgestellt wird. Diese Aufgabe wird vom Gegenstand der vorliegenden Erfindung gelöst, wie er in den Ansprüchen und der nachfolgenden Beschreibung definiert wird. Die Beispiele der vorliegenden Erfindung dienen als Beleg für die Lösung der der Erfindung zugrundeliegenden Aufgabe.

Erstmals wird im Rahmen der vorliegenden Erfindung ein Verfahren zur biotechnologischen Herstellung von wasserlöslichem lebensmitteltauglichem PolyP in Pulverform (Bio-PolyP) beschrieben. Zwei Kettenlängenfraktionen (42 und 11 P-Untereinheiten durchschnittliche PolyP-Kettenlänge, Reinheit auf Augenhöhe mit chemisch hergestelltem PolyP) wurden erhalten. Die physikalisch-chemischen Eigenschaften des Bio-PolyP der vorliegenden Erfindung wurden unter anderem mit einem Enzym-Assay, $^{31}$P Kernspinresonanz und Polyacrylamid-Gelelektrophorese (PAGE) analysiert. Der erfindungsgemäße Prozess ist hochskalierbar, benötigt wenig Chemikalien (z.B. Ethanol, HCl, NaOH, NaCl) und produziert nur 0,75 g NaCl als Nebenprodukt pro g Bio-PolyP (das Ethanol ist destillierbar). Infolgedessen kann die vorliegende Erfindung u.a. für ein wirtschaftliches biotechnologisches Phosphatrecycling von Phosphatabfällen zu hochwertigem Bio-PolyP verwendet werden.

Anorganisches Polyphosphat (PolyP$_n$, wobei n die durchschnittliche PolyP-Kettenlänge ist) ist das lineare Polymer von Orthophosphat (P$_i$) mit einer Kettenlänge von zwei bis tausend P-Untereinheiten und findet sich in allen lebenden Organismen [Ref. 1: aF. M. Harold, Bacteriol. Rev. 1966, 30, 772-794; bI. S. Kulaev, V. M. Vagabov, T. V. Kulakovskaya, The biochemistry of inorganic polyphosphates, Second ed., John Wiley & Sons, Ltd, West Sussex (England), 2005**;** cN. N. Rao, M. R. Gomez-Garcia, A. Kornberg, Annu. Rev. Biochem. 2009, 78, 605-647]. Die PolyP-Struktur wird in lineares, zyklisches (auch Metaphosphate genannt) und vernetztes (sogenannte Ultraphosphate) PolyP unterteilt. PolyP tritt nur ohne Vernetzungen in wässriger Lösung auf, was durch die Antiverzweigungsregel ([Ref. 2: J. R. Van Wazer, K. A. Holst, J. Am. Chem. Soc. 1950, 72, 639-644], S. 437 in [Ref. 3: J. R. Van Wazer, Phosphorus and its compounds. Volume I: Chemistry, Interscience publishers, Inc., New York (USA), 1958]) erklärt wird.

Zyklisches PolyP mit einer Kettenlänge von drei bis ca. 12 P-Untereinheiten kann chemisch synthetisiert werden, entsteht spontan bei der Hydrolyse von langkettigem linearem PolyP und ist in wässriger Lösung stabil (S. 4 in Ref. [1b], S. 680 und 790 in Ref. [3]: J. R. Van Wazer, Phosphorus and its compounds. Volume I: Chemistry, Interscience publishers, Inc., New York (USA), 1958). Im Vergleich zu linearem PolyP weist zyklisches PolyP keine Hydroxylgruppe mit neutralem pK$_a$ an den Endgruppen auf und besitzt nicht die biotechnologisch wertvolle multivalente Kationenkomplexfähigkeit (S. 466 und 680 in Ref. [3]: J. R. Van Wazer, Phosphorus and its compounds. Volume I: Chemistry, Interscience publishers, Inc., New York (USA), 1958). Da lebende Organismen ausschließlich lineares PolyP produzieren, muss der Nachweis geringer Mengen an cyclischem PolyP in Extrakten aus lebenden Organismen auf die spontane Bildung aus linearem PolyP zurückgeführt werden ([1c, 4], S. 692 in Ref. [3]: J. R. Van Wazer, Phosphorus and its compounds. Volume I: Chemistry, Interscience publishers, Inc., New York (USA), 1958).

Die Molekularformel von linearem PolyP ist in Gleichung 1 (S. 433 in Ref. [3]: J. R. Van Wazer, Phosphorus and its compounds. Volume I: Chemistry, Interscience publishers, Inc., New York (USA), 1958) dargestellt. Das Molekulargewicht hängt von der Kettenlänge n und dem Gegenion M ab. Die Gegenionen können $H^+$, Metallionen oder kationische organische Moleküle [Ref. 5: aM. Dürr, K. Urech, T. Boller, A. Wiemken, J. Schwencke, M. Nagy, Arch. Microbiol. 1979, 121, 169-175; bL. Jacobson, M. Halmann, J. Yariv, Biochem. J. 1982, 201, 473-479; cG. M. Roomans, Physiol. Plant. 1980, 48, 47-50] sein. Jede P-Untereinheit in der PolyP-Kette besitzt einen starken Wasserstoff (pK$_a$ = ca. 0 bis 3). Außerdem befindet sich an beiden Enden der PolyP-Kette ein schwacher Wasserstoff (pK$_a$ = ca. 7 bis 9) (S. 460 in Ref. [3]: J. R. Van Wazer, Phosphorus and its compounds. Volume I: Chemistry, Interscience publishers, Inc., New York (USA), 1958). Nach der Henderson-Hasselbalch-Gleichung hängt der Grad der PolyP-Protonierung in wässriger Lösung vom pH-Wert der Lösung und dem pK$_a$ der Funktionsgruppe ab. Die PolyP-Kette ist bei neutralem pH-Wert vollständig deprotoniert, mit Ausnahme der Endgruppen, von denen die Hälfte protoniert ist. Daher kann die Molekularformel von linearem PolyP bei neutralem pH-Wert mit Gleichung 2 bestimmt werden. Während bei einem pH-Wert von ca. 4 beide Endgruppen protoniert werden, kann die Molekularformel von linearem PolyP mit Gl. 3 berechnet werden.

In Rahmen der vorliegenden Erfindung bezieht sich die Abkürzung "PolyP", sofern nicht anders angegeben, auf lineares PolyP. M in Gleichung 1, Gleichung 2 und Gleichung 3 sollte eine einzige positive Ladung tragen. Die Anzahl der höherwertigen Kationen nimmt proportional zu ihrer Ladung ab.

**Gl. 1:** *generische Molekularformel des linearen PolyP:* $M_{n+2}P_nO_{3n+1}$

**Gl. 2:** *Molekularformel des linearen PolyP mit neutralem pH-Wert:* $M_{n+1}HP_nO_{3n+1}$

**Gl. 3:** *Molekularformel des linearen PolyP mit einem pH-Wert von ca. 4:* $M_nH_2P_nO_{3n+1}$

PolyP wird häufig als Lebensmittelzusatzstoff verwendet, da es hochenergetische Phosphoanhydridbindungen enthält, höherwertige Kationen komplexieren kann, Wasser bindet, den pH-Wert puffert, das Wachstum von Mikroorganismen hemmt, für den menschlichen Verzehr sicher ist, als Dispergiermittel wirkt und biologisch abbaubar ist [Ref. 1b: S. Kulaev, V. M. Vagabov, T. V. Kulakovskaya, The biochemistry of inorganic polyphosphates, Second ed., John Wiley & Sons, Ltd, West Sussex (England), 2005, 6: T. V. Kulakovskaya, V. M. Vagabov, I. S. Kulaev, Process Biochemistry 2012, 47, 1-10]. Um nur einige Anwendungen zu nennen, stabilisiert und erweicht PolyP Fleischprodukte, gibt Weichkäse seine einzigartige Viskosität und hemmt das Bakterienwachstum im Dönerspieß beim langsamen Kochen. Die PolyP-Kettenlänge definiert, welche chemischen Eigenschaften ausgeprägter sind. Nur die schwachen Wasserstoffatome an beiden Enden der PolyP-Kette puffern den pH-Wert. So ist kurzkettiges PolyP nützlich bei der Stabilisierung des pH-Wertes. $PolyP_2$ und $PolyP_3$ haben die einzigartige Eigenschaft, das Actomyosin von Fleisch nach der Rigor Mortis zu reaktivieren und so die natürliche Weichheit und Wasserhaltefähigkeit von Rindfleisch wiederherzustellen [Ref. 7: Q. W. Shen, D. R. Swartz, Meat science 2010, 84, 364-364]. Die kationkomplexierende Fähigkeit, die Anzahl der Phosphoanhydridbindungen, die bakteriostatische Aktivität und die Hydrophilie von PolyP nehmen mit zunehmender Kettenlänge zu.

Die Methoden "Analytische PolyP-Extraktion" und "Präparative PolyP-Extraktion" stehen für maximale PolyP-Rückgewinnung für PolyP-Analytik bzw. PolyP-Präparation für weitere Anwendungen. Für die analytische Extraktion werden toxische Chemikalien verwendet, während die Extraktion nur im kleinen Maßstab funktioniert, arbeitsintensiv ist und das Ziel hat, so viel PolyP wie möglich aus einer Zelle zu extrahieren, ohne das PolyP zu hydrolysieren. Das PolyP wird in gelöster Form extrahiert. Für die präparative Extraktion werden nur lebensmitteltaugliche Chemikalien verwendet, während die Extraktion hochskalierbar und kostengünstig ist. Das PolyP wird als festes Pulver extrahiert. Ziel einer präparativen Extraktion ist u.a. die Herstellung eines Lebensmittelzusatzes mit Potenzial für die großtechnische biotechnologische Produktion.

PolyP kann im μg- bis mg-Bereich mit einem breiten Spektrum an Kettenlängen durch Polyacrylamid-Gelelektrophorese (PAGE) [Ref. 8: J. E. Clark, H. G. Wood, Anal. Biochem. 1987, 161, 280-290] oder fraktionierte Fällung [Ref. 9: S. A. Smith, C. J. Baker, J. M. Gajsiewicz, J. H. Morrissey, Blood 2017, 130, 88-91] hergestellt werden. Letzteres hängt von der höheren Löslichkeit des kurzkettigen PolyP in einem bestimmten Lösungsmittel sowie von der Tatsache ab, dass die Lösungsmittelleistung eines binären Flüssigkeitsgemisches (aus Lösungsmittel und Nicht-Lösungsmittel) vom Verhältnis der beiden Flüssigkeiten abhängt [Ref. 10: L. H. Cragg, H. Hammerschlag, Chem. Rev. 1946, 39, 79-135]. Wird einem Polymer-Lösungsmittelsystem ein Nicht-Lösungsmittel zugesetzt, erhält man zwei Phasen im Gleichgewicht, wenn die Anteile der Komponenten richtig eingestellt sind. Die obere Schicht enthält gelöstes kurzkettiges Polymer. Die untere Schicht enthält ausgefälltes langkettiges Polymer (s. z.B. SS. 603 und 670 in Ref. [3]: J. R. Van Wazer, Phosphorus and its compounds. Volume I: Chemistry, Interscience publishers, Inc., New York (USA), 1958**,** [10]: L. H. Cragg, H. Hammerschlag, Chem. Rev. 1946, 39, 79-135).

Im Vergleich zum Labor, in dem selbst langkettiges PolyP synthetisch zugänglich ist, erlaubt die großtechnische industrielle chemische Synthese nur die Herstellung kürzerer Kettenlängen bis zu 40 P-Untereinheiten [Ref. 11: aJ. J. Christ, L. M. Blank, Anal. Biochem. 2018, 548, 82-90; bT. Shiba, in Inorganic polyphosphates in eukaryotic cells (Eds.: T. Kulakovskaya, E. Pavlov, E. N. Dedkova), Springer International Publishing, Basel (Switzerland), 2016, pp. 139-158]. Die chemische Synthese (eine Kondensationsreaktion) erfolgt durch Erhitzen von reinem $P_i$ auf 400 bis 800°C. Um eine Phosphoanhydridbindung zu bilden, muss ein Wasser aus zwei Monomeren kondensieren. Mit $Na_2HPO_4$ als Substrat für eine chemische PolyP-Synthese bildet sich meist das kürzest mögliche PolyP mit einer Kettenlänge von 2 P-Untereinheiten ($2\ Na_2HPO_{4\ (s)} \rightarrow Na_4P_2O_{7\ (s)} + H_2O_{\ (g)}$). Mit $Na_{5/3}H_{4/3}PO_4$ als Substrat bilden sich meist PolyP mit einer Kettenlänge von 3 P-Untereinheiten ($3\ Na_{5/3}H_{4/3}PO_{4\ (s)} \rightarrow Na_5P_3O_{10\ (s)} + 2\ H_2O_{\ (g)}$). Schlussfolgernd bestimmt das Verhältnis von Wasserstoff zu Natrium im Substrat die maximal mögliche PolyP-Kettenlänge in der chemischen Synthese (je mehr Wasserstoff und desto weniger Natrium, desto länger die PolyP-Kettenlänge). Für die chemische PolyP-Synthese kann jedoch keine reine Phosphorsäure verwendet werden, da sie zu hygroskopisch ist. Im industriellen Umfeld wird Phosphorsäure mit NaOH teilweise neutralisiert. Je saurer die Substratlösung bleibt, desto länger ist die PolyP-Kettenlänge. Dies ist der Grund, warum chemisch hergestelltes langkettiges PolyP einen sauren pH-Wert aufweist. Zusätzlich zur Substratzusammensetzung ist die erhaltene Kettenlänge proportional zur Dauer und Temperatur der Wärmebehandlung.

## Kurzfassung der Erfindung

Die vorliegende Erfindung betrifft ein Verfahren zur Gewinnung von Polyphosphat aus polyphosphat-haltigen Hefezellen, umfassend:

(1) Aufschließen von polyphosphathaltigen Hefezellen nach deren Ernte durch Einfrieren und Auftauen;
(2) Inkubieren einer wässrigen Lösung, enthaltend die aufgeschlossenen polyphosphathaltigen Hefezellen bei einer Temperatur von ca. 70°C [z.B. bis 80°C] für ca. 10 Minuten;
(3) Entfernen von Zelldebris aus der wässrigen Lösung;
(4) Entfernen von Nukleinsäuren und Proteinen aus der wässrigen Lösung durch Zugabe einer Säure;
(5) Neutralisieren der verbleibenden wässrigen Lösung; und
(6) Gewinnen von Polyphosphat aus der wässrigen Lösung durch Fällung mittels eines Alkohols.

Des Weiteren betrifft die vorliegende Erfindung eine Zusammensetzung, enthaltend getrocknetes Polyphosphat, wobei das getrocknete Polyphosphat:

(a) einen Reinheitsgrad von zumindest 80% aufweist, vorzugsweise definiert als Gehalt von linearem Polyphosphat und Kristallwasser, in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Natriumionen und/oder Kaliumionen und Magnesiumionen als Gegenionen enthält,
(b) zumindest 20% (mol/mol) Natriumionen und/oder zumindest 20% (mol/mol) Kaliumionen und bis zu 50% (mol/mol) Magnesiumionen als Gegenionen enthält,
(c) eine durchschnittliche Kettenlänge von 11-44 P-Einheiten aufweist, wobei die durchschnittliche Kettenlänge enzymatisch bestimmt wird, und/oder
(d) einen Anteil an zyklischem Polyphosphat von nicht mehr als 2% bezogen auf die Trockensubstanz aufweist.

Zudem betrifft die vorliegende Erfindung eine Zusammensetzung, wobei das Polyphosphat durch erfindungsgemäßes Verfahren der vorliegenden Erfindung erhältlich ist.

Das biotechnologische Herstellungsverfahren des PolyP der vorliegenden Erfindung erscheint vielversprechend, da die PolyP-Kettenlänge in Mikroorganismen bis zu tausend P-Untereinheiten erreichen kann. Darüber hinaus können Mikroorganismen PolyP aus unreinem P herstellen, während die chemische PolyP-Synthese auf reinem $P_i$ basiert. Nach bestem Wissen und Gewissen der Erfinder gibt es im Stand der Technik keine Berichte über die Herstellung eines lebensmittelechten wasserlöslichen PolyP mit einem hochskalierbaren biotechnologischen Prozess. Ein Verfahren zur Gewinnung von *Saccharomyces cerevisiae* (Backhefe) mit 28 % PolyP (als $KPO_3$) im Zelltrockengewicht wurde kürzlich berichtet [Ref. 12: J. J. Christ, L. M. Blank, FEMS yeast research 2019, 19]. Das übergeordnete Ziel der vorliegenden Erfindung war u.a. die Reinigung von PolyP aus polyP-reichem *S. cerevisiae*. Die gewünschten Eigenschaften eines solchen biotechnologisch hergestellten PolyPs waren u.a.: Erscheinungsbild als trockenes weißes wasserlösliches Pulver, Lebensmittelqualität, lineare Molekularstruktur, Reinheit vergleichbar mit chemisch hergestelltem PolyP und ein PolyP mit überwiegend Natrium und eines mit überwiegend Kalium als Gegenion. Der Begriff "Bio-PolyP" wird für das Produkt des hier beschriebenen Prozesses verwendet. Alle Prozessschritte wurden so konzipiert, dass sie für die geplante Großserienproduktion hochskalierbar sind. Die physikalisch-chemischen Eigenschaften des Bio-PolyP wurden im Vergleich zu chemisch hergestelltem PolyP analysiert.

## Kurzbeschreibung der Abbildungen

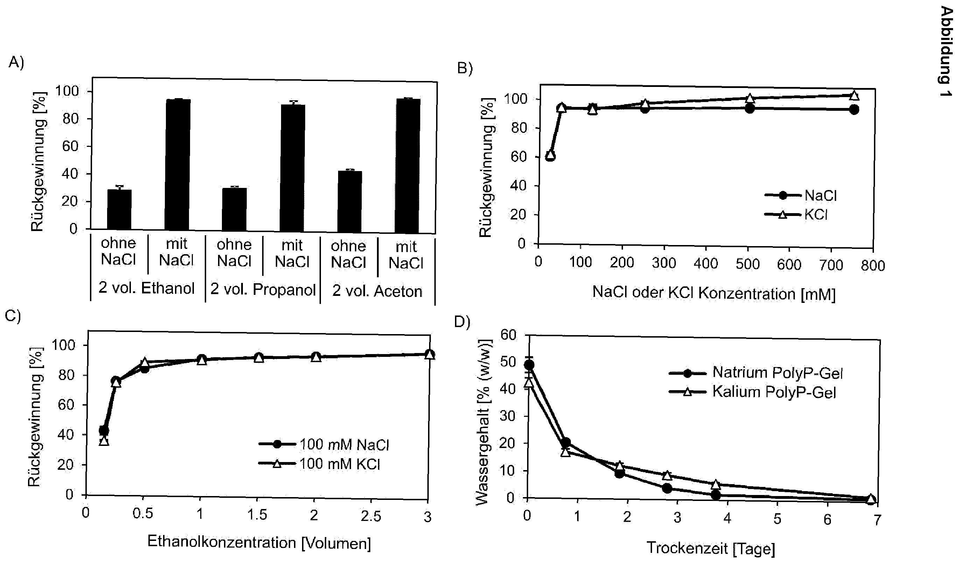

**Abbildung 1: Fällung und Trocknung von chemisch hergestelltem PolyP.**
**(A)** Budit 4 (30 g * $L^{-1}$, pH 7 mit NaOH) wurde mit oder ohne 454 mM NaCl versetzt und mit 2 Volumen Lösungsmittel ausgefällt. Nach 1 Stunde Inkubation wurde die Lösung zentrifugiert (5.000 g, 10 Minuten). Das Pellet wurde bei 120°C getrocknet und gewogen. Die Referenz für (A), (B) und (C) war Budit 4 (pH bis 7 mit NaOH oder KOH), das in Wasser gelöst und dann bei 120°C ohne Fällung getrocknet wurde. Die Rückgewinnungsraten in (A), (B) und (C) wurden berechnet, indem das Gewicht von PolyP, das nach der Ausfällung erhalten wurde, durch das Gewicht der Referenz dividiert wurde. **(B)** Der pH-Wert einer Budit 4-Lösung (30 g * $L^{-1}$) wurde mit NaOH auf 7 eingestellt, wenn NaCl während der Fällung verwendet wurde, und mit KOH, wenn KCl verwendet wurde. Die Lösung wurde mit unterschiedlichen Konzentrationen von NaCl oder KCl versetzt. Nach Ausfällen mit 2 Volumen Ethanol und 1 Stunde Inkubation bei Raumtemperatur wurde die Lösung zentrifugiert (5.000 g, 10 Minuten). Das Pellet wurde bei 120 °C getrocknet und gewogen. **(C)** Budit 4 (30 g * $L^{-1}$, pH auf 7 mit NaOH oder KOH) wurde mit 100 mM NaCl oder KCl

versetzt und mit verschiedenen Mengen Ethanol ausgefällt. Nach 1 Stunde Inkubation wurde die Lösung zentrifugiert (5.000 g, 10 Minuten). Das Pellet wurde bei 120°C getrocknet und gewogen. **(D)** Einer Budit 4-Salzlösung wurden zwei Volumen Ethanol zugesetzt (30 g Budit 4 * $L^{-1}$, pH bis 7 mit NaOH oder KOH, 100 mM NaCl oder KCl). Nach 1 Stunde Inkubation wurde die Lösung zentrifugiert (5.000 g, 10 Minuten). Etwa 730 mg PolyP-Gel aus dem Sediment wurden gleichmäßig auf einem Trocknungstablett (ca. 22 $cm^2$) verteilt und in einem Exsikkator inkubiert, der mit getrockneter Kieselsäure gefüllt wurde. Der Wasserverlust wurde durch das Wiegen der Trays ermittelt. Nach 7 Tagen wurde der endgültige Wassergehalt durch Trocknung bei 120°C und anschließende Verwiegung gemessen. Die Bezeichnungen "Natrium-PolyP-Gel" und "Kalium-PolyP-Gel" zeigen an, dass der pH-Wert mit NaOH oder KOH eingestellt wurde und NaCl oder KCl für die Fällung verwendet wurden. Es werden die Mittelwerte von zwei unabhängigen Experimenten mit jeweils einer (B, C, D) oder zwei (A) Replikatmessungen dargestellt. Die Fehlerbalken zeigen den Standardfehler des Mittelwertes zwischen den Experimenten an. Abkürzungen: "vol.", Volumen.

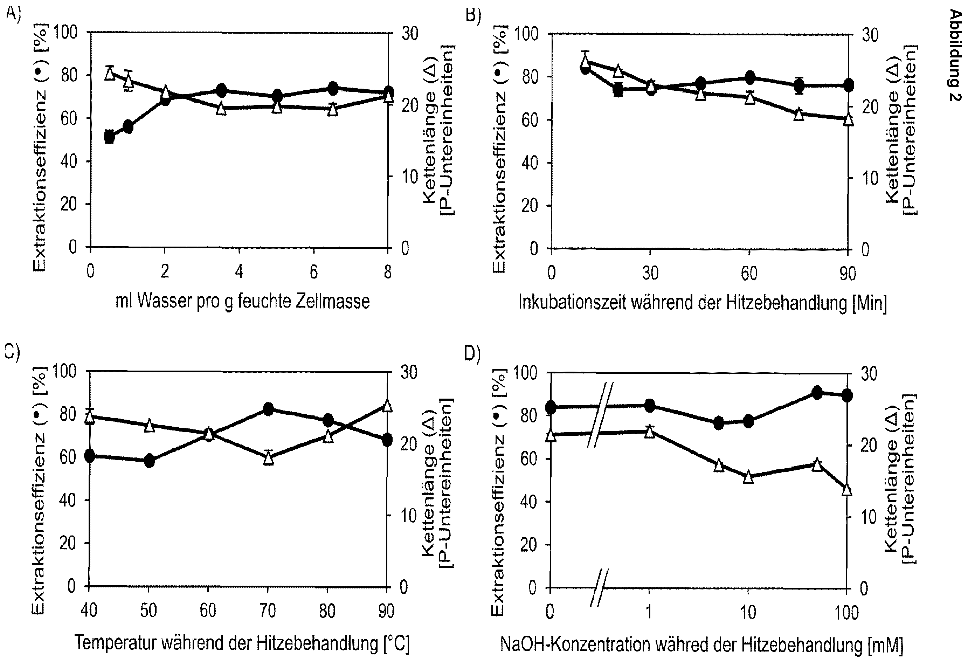

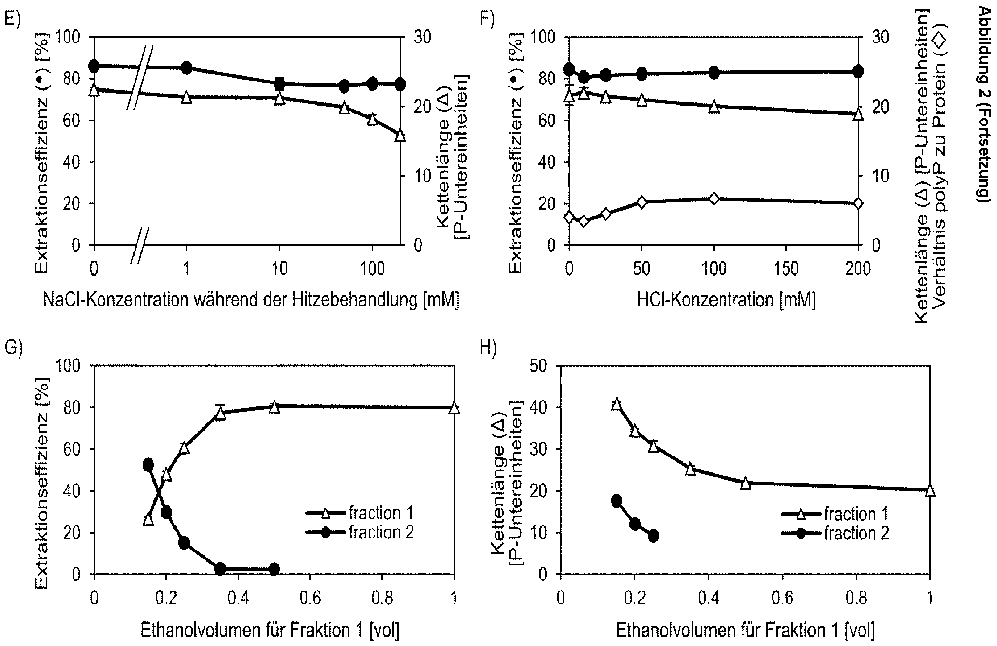

**Abbildung 2: Freisetzung von Bio-PolyP aus polyP-reichem *S. cerevisiae* und fraktionierte Fällung von Bio-PolyP.**
(A) bis (F) zeigen einzelne Experimente, während (G) und (H) zeigen unterschiedliche Ergebnisse von einem Experiment. Die Versuchsaufbauten von (A) bis (G/H) bauen aufeinander auf. Siehe Abschnitt Materialien und Methoden für das Startprotokoll. Das gesamte PolyP und die Kettenlänge wurden mit dem Enzymassay bestimmt. **(A)** Das Volumen des Milli-Q-Wassers wurde variiert und auf 5 ml pro g Nasszellenmasse eingestellt. **(B)** Die Inkubationszeit der Wärmebehandlung wurde getestet und auf 10 Minuten eingestellt. **(C)** Die Temperatur während der Wärmebehandlung wurde analysiert und auf 70°C eingestellt. **(D)** Verschiedene Konzentrationen von NaOH wurden als Extraktionsmittel getestet. Es wurde weiterhin reines Wasser verwendet. **(E)** Verschiedene Konzentrationen von NaCl wurden anstelle von reinem Wasser als Extraktionsmittel getestet. Für weitere Experimente wurde reines Wasser gewählt. **(F)** Nach der Übertragung in das neue Reaktionsgefäß wurde eine HCl-Präzipitation in das Protokoll eingefügt. Der pH-Wert wurde durch die Zugabe verschiedener HCl-Konzentrationen (Endkonzentrationen auf der X-Achse) gesenkt. Das Präzipitat wurde durch Zentrifugation entfernt (10.000 g, 5 Minuten) und der Überstand in ein neues Reaktionsgefäß gebracht, wo das ursprüngliche Protokoll durch Einstellen des pH-Wertes auf 7 fortgesetzt wurde. Die Summe aus Nukleinsäure und Protein im PolyP, die nach der Ethanolfällung erhalten wurde, wurde gemessen (Absorption bei 280 nm) und durch die PolyP-Konzentration dividiert (Molekulargewicht $_{polyP}$ : 118,07 g/mol). 50 mM HCl wurde gewählt. **(G, H)** Verschiedene Ethanolkonzentrationen wurden getestet, um PolyP auszufällen. Nach der Rückgewinnung der ersten PolyP-Fraktion durch Zentrifugation wurde das verbleibende PolyP (d. h. die Fraktion 2) durch Zugabe von finalem 1 Volumen Ethanol gewonnen. Die Fällung mit 0,15 Volumen Ethanol und anschließend 1 Volumen Ethanol wurde gewählt. Jeder Messpunkt in (A) bis (G/H) zeigt den Mittelwert aus doppelten präparativen Extraktionen. Die Fehlerbalken zeigen den Standardfehler des Mittelwertes zwischen den Extraktionen an.

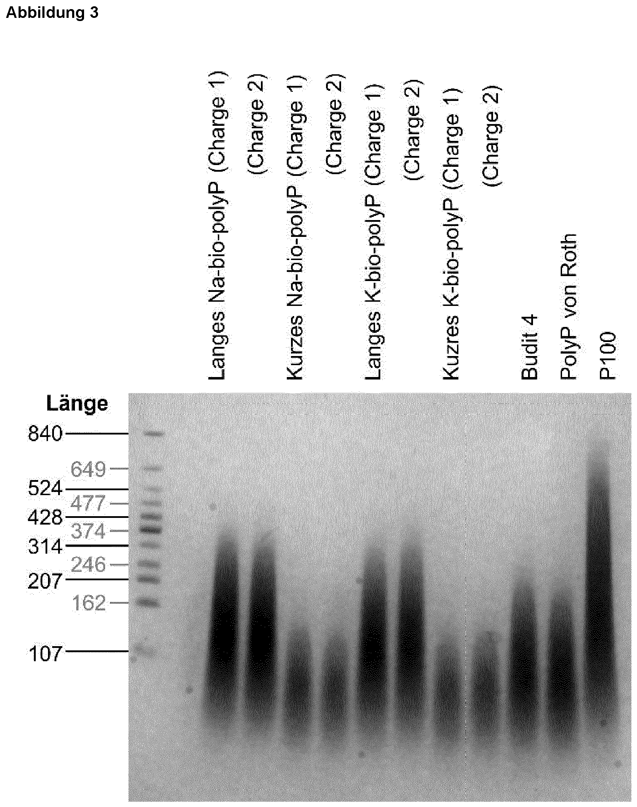

**Abbildung 3: PAGE-Analyse von Bio-PolyP und chemisch hergestelltem PolyP.**
Abkürzungen: K, Kalium; Na, Natrium.

## Ausführliche Beschreibung der Erfindung

Derzeit gibt es nur den chemischen Weg, um lebensmitteltaugliches PolyP im industriellen Maßstab herzustellen. In der chemischen Synthese (einer Kondensationsreaktion) wird festes reines $P_i$ für einige Stunden auf mehrere hundert Grad erhitzt. Der Synthese-Teil selbst hat u.a. einen großen Nachteil: Im großen Maßstab können nur Kettenlängen bis zu ca. 40 P-Untereinheiten hergestellt werden. Die drei längsten PolyPs, die im Handel in großen Mengen erhältlich waren, wurden in Rahmen der vorliegenden Erfindung getestet. Von den drei PolyPs bemaß die längste (P100) 42 P-Untereinheiten. Dieser Befund bestätigt, dass die chemische Synthese tatsächlich auf maximal ca. 40 P-Untereinheiten Kettenlänge beschränkt ist. Im Labormaßstab wird die fraktionierte Fällung verwendet, um aus dem chemisch hergestellten PolyP [Ref. 11b: bT. Shiba, in Inorganic polyphosphates in eukaryotic cells (Eds.: T. Kulakovskaya, E. Pavlov, E. N. Dedkova), Springer International Publishing, Basel (Switzerland), 2016, pp. 139-158] langkettiges PolyP herzustellen. PolyP mit einer längeren Kettenlänge wäre ein industriell attraktives Produkt, da einige Eigenschaften des PolyP mit zunehmender Kettenlänge zunehmen (z. B. Hydrophilie, Kationenkomplexfähigkeit etc.).

Bis heute gibt es nur einen Prozess, der sich mit der biotechnologischen PolyP-Produktion beschäftigt (so genannter "Heatphos-Prozess" [Ref. 16: A. Kuroda, N. Takiguchi, T. Gotanda, K. Nomura, J. Kato, T. Ikeda, H. Ohtake, Biotechnol. Bioeng. 2002, 78, 333-338, 18: aN. Takiguchi, A. Kuroda, H. Ohtake, S. Tsuneda, in Phosphorus Recovery and Recycling, First ed. (Eds.: H. Ohtake, S. Tsuneda), Springer Singapore, Singapore, 2019, pp. 515-526; bR. Hirota, A. Kuroda, J. Kato, H. Ohtake, J. Biosci. Bioeng. 2010, 109, 423-432]). Im "Heatphos"-Verfahren wird Klärschlamm für 1 Stunde bei 70°C wärmebehandelt. Das freigesetzte PolyP wird mit $Ca^{2+}$ ausgefällt. Die Anwendung des Produkts erfolgt im Düngemittel. Das "Heatphos" PolyP kann nicht in Lebensmitteln verwendet werden, da das Produkt weder lebens-

mitteltauglich ($P_i$-Quelle: Klärschlamm) noch wasserlöslich (Calcium-PolyP) ist. Die Wasserlöslichkeit von PolyP ist von Bedeutung für Lebensmittelanwendungen, bei denen PolyP nur in gelöstem Zustand seine gewünschten physikalisch-chemischen Eigenschaften zeigen kann. Der hier beschriebene erfindungsgemäße Prozess umgeht die Nachteile des "Heatphos"-Prozesses, indem das PolyP aus lebensmitteltauglichem *S. cerevisiae* ("GRAS" - generell als sicher anerkannt wird) und PolyP-Fällung mit NaCl (oder KCl) und Ethanol extrahiert wird.

"Polyphosphat-haltige Hefen" sind Hefen, die Polyphosphat (PolyP) enthalten. Wie hierin beschrieben, sind Hefen in der Lage, PolyP herzustellen. Wie aus dem Stand der Technik bekannt ist und auch hierin beschrieben, kann man Hefen forcieren, PolyP herzustellen.

Im Rahmen der vorliegenden Erfindung beträgt der Polyphosphatanteil einer Hefemasse vorzugsweise mind. etwa 5, mind. etwa 6, mind. etwa 7, mind. etwa 8, mind. etwa 9, mind. etwa 10, mind. etwa 11, mind. etwa 12, mind. etwa 13, mind. etwa 14, mind. etwa 15, mind. etwa 16, mind. etwa 17, mind. etwa 18, mind. etwa 19, mind. etwa 20, mind. etwa 21, mind. etwa 22, mind. etwa 23, mind. etwa 24, oder mind. etwa 25 Gew%, bezogen auf die Trockenzellmasse.

Als "Polyphosphat" oder "PolyP" wie hier beschrieben wird das lineare Polymer von Orthophosphat ($P_i$; Salz der Orthophosphorsäure) verstanden, wobei von "Polyphosphat" oder "PolyP" wie hier verwendet mindestens 2 P-Untereinheiten umfasst sind. Die Kettenlänge von PolyP kann bestimmt werden mit Verfahren wie dem Fachmann allgemein bekannt (bspw. Christ & Blank, Anal Biochem (2018), 548: 82-90; Christ JJ, Willbold S, Blank LM (2019) Analytical Chemistry, 91 (12), 7654-7661) und wie hier auch exemplarisch beschrieben.

Christ & Blank (2018), Anal Biochem 563, 71-78, zeigen auf Seite 74, Abbildung 1 Ergebnisse verschiedener Extraktionsverfahren von PolyP aus *S. cerevisiae.* Dazu wurde *S. cerevisiae,* so wie in Liss und Langen (1962), Arch. Mikrobiol. 41, 383-392 beschrieben, gezüchtet. Anschließend wurde PolyP aus *S. cerevisiae* extrahiert. Der PolyP-Gehalt war im besten Fall ca. 11% bezogen auf die Trockenzellmasse, wenn die Extraktion gemäß dem Protokoll von Bru et al. (2016), Microbial Cell 4, 6-15 erfolgte (siehe Abbildung 1 von Christ und Blank (loc. cit.). Unter Verwendung des ursprünglichen Extraktionsprotokolls von Liss und Langen (loc. cit.) erhielten Christ und Blank (loc. cit.) nur ca. 3 Gew% PolyP-Gehalt aus *S. cerevisiae* bezogen auf die Trockenzellmasse (siehe Abbildung 1 von Christ und Blank (loc. cit.), obgleich Liss und Langen (loc. cit.) behaupten, ca. 23 Gew% PolyP-Gehalt in *S. cerevisiae* bezogen auf die Trockenzellmasse erhalten zu haben.

Die "Trockenzellmasse" Trockenzellmasse (hier auch "Zelltrockengewicht" genannt), auf die sich der PolyP-Anteil der Hefemasse (in Gew%) bezieht, weist bevorzugt einen Wassergehalt von mind. etwa 5, 10, 12, 15, 20, 25, 30, 35, 40, 45, 50, 55, 60, 65, oder 70 Gew% (bevorzugt mind. etwa 65 oder 70 Gew%), und/oder bevorzugt nicht mehr als etwa 70, 75, 80, oder 85 Gew% Wasser auf, bevorzugt nicht mehr als etwa 75 Gew% Wasser. Der Trockenanteil bzw. die Trockenzellmasse kann dabei nach üblichen Methoden ermittelt werden wie dem Fachmann allgemein bekannt und wie hier auch exemplarisch beschrieben.

Verfahren zur analytischen PolyP-Extraktion sind bekannt und bspw. auch beschrieben in Christ & Blank, Anal Biochem (2018), 563: 71-78. Der Polyphosphat (PolyP) Anteil wird als $KPO_3$ gemessen mit Verfahren wie dem Fachmann allgemein bekannt (bspw. Christ & Blank, Anal Biochem (2018), 548: 82-90). Zum Beispiel und bevorzugt kann im Rahmen der vorliegenden Erfindung folgende Formel 1 verwendet werden:

$$\text{PolyP (als } KPO_3\text{)-Gehalt in Trockenzellgewicht [Gew\%]} = \left( \frac{\text{echte polyP-Konzentration [µM Pi]} * \text{Molekulargewicht polyP } [g{*}mol^{-1}] * \text{Eluatvolumen [µl]} * \text{Verdünnung [Verdünnungsgrad]}}{\text{Zellfeuchtmasse [mg]} * \text{Trockenanteil [Gew\%]} * 10^5} \right) \quad \text{(Formel 1)}$$

wobei polyP als $KPO_3$ definiert wird mit einem Molekulargewicht von 118,07 $g{*}mol^{-1}$.

Verfahren zur PolyP-Extraktion sind dem Fachmann allgemein bekannt (bspw. Bru et al., Microbial Cell (2016), 4: 6-15; insb. auch das dort beschriebene Verfahren ohne Ethanol-Präzipitierung oder Christ & Blank, Anal Biochem (2018), 563: 71-78 zur analytischen PolyP-Extraktion).

**Grundlegende Überlegungen zur Reinigung von PolyP**

Eine häufig verwendete Reinigungsstrategie ist die Kristallisation. Homogene PolyP-Zubereitungen mit einer einzigen Kettenlänge können kristallisieren und bis zu einer Kettenlänge von ca. 5 P-Untereinheiten (S. 6 in Ref. [1b]: bl. S. Kulaev, V. M. Vagabov, T. V. Kulakovskaya, The biochemistry of inorganic polyphosphates, Second ed., John Wiley & Sons, Ltd, West Sussex (England), 2005;, S. 602-604 in Ref. [3]: J. R. Van Wazer, Phosphorus and its compounds. Volume I: Chemistry, Interscience publishers, Inc., New York (USA), 1958) erhalten werden. Außergewöhnlich langes (> 1000 P-

Untereinheiten), wasserunlösliches $P_i$-Glas kann sich auch kristallisieren, da einzelne Ketten durch viele Einheitszellen im Kristall laufen, und somit ist die genaue Länge der Kette kein wichtiger Faktor mehr für die Bestimmung der Gitterparameter des Kristalls (S. 602 und 675 - 676 in Ref. [3]: J. R. Van Wazer, Phosphorus and its compounds. Volume I: Chemistry, Interscience publishers, Inc., New York (USA), 1958). Wasserlösliches PolyP mit einer heterogenen Kettenlänge im Bereich von ca. 6 bis 1000 P-Untereinheiten kann nicht kristallisieren. Tatsächlich hemmt PolyP die Kristallisation anderer Substanzen in seiner Umgebung [Ref. 19: S. Omelon, W. Habraken, in Inorganic polyphosphates in eukaryotic cells (Eds.: T. Kulakovskaya, E. Pavlov, E. N. Dedkova), Springer International Publishing, Basel (Switzerland), 2016, pp. 177-205]. Die Unfähigkeit zur Kristallisation kann sowohl auf die Schwierigkeit der Kristallisation aus einem heterogenen Polymergemisch als auch auf die Wirkung polarer Gruppen auf die Kristallisation zurückgeführt werden (S. 6 in Ref. [1b]: bl. S. Kulaev, V. M. Vagabov, T. V. Kulakovskaya, The biochemistry of inorganic polyphosphates, Second ed., John Wiley & Sons, Ltd, West Sussex (England), 2005;, S. 602-604 in Ref. [3]: J. R. Van Wazer, Phosphorus and its compounds. Volume I: Chemistry, Interscience publishers, Inc., New York (USA), 1958). Abschließend war die Kristallisation als PolyP-Reinigungsstrategie in Rahmen der vorliegenden Erfindung keine bevorzugte Option.

In der Literatur wurde die fraktionierte Fällung von PolyP mit Aceton [Ref. 9: S. A. Smith, C. J. Baker, J. M. Gajsiewicz, J. H. Morrissey, Blood 2017, 130, 88-91, 20: aE. C. De Oliveira Lima, G. B. Alcantara, F. C. Damasceno, J. M. M. Neto, F. Galambeck, Quim. Nova. 2010, 33, 1991-1995; bJ. R. Van Wazer, J. Am. Chem. Soc. 1950, 72, 647-655], Isopropanol [Ref. 9: S. A. Smith, C. J. Baker, J. M. Gajsiewicz, J. H. Morrissey, Blood 2017, 130, 88-91] und Ethanol [Ref. 11b: bT. Shiba, in Inorganic polyphosphates in eukaryotic cells (Eds.: T. Kulakovskaya, E. Pavlov, E. N. Dedkova), Springer International Publishing, Basel (Switzerland), 2016, pp. 139-158, 21: aL. K. Seidlmayer, M. R. Gomez-Garcia, T. Shiba, G. A. Porter, Jr., E. V. Pavlov, D. M. Bers, E. N. Dedkova, Arch. Biochem. Biophys. 2019, 662, 177-189; bT. Shiba, Y. Takahashi, T. Uematsu, Y. Kawazoe, K. Ooi, K. Nasu, H. Itoh, H. Tanaka, M. Yamaoka, M. Shindoh, T. Kohgo, Key Engineering Materials 2004, 254-256, 1119-1122] durchgeführt. Die wichtigste Anwendung des hier produzierten PolyP liegt u.a. in Lebensmitteln. Im Falle von Lösungsmittelspuren, die im fertigen Bio-PolyP-Produkt verbleiben, wurde in Rahmen der vorliegenden Erfindung ein Lösungsmittel mit geringeren Gesundheitsrisiken (Ethanol) gewählt. Billiges denaturiertes Ethanol war bei der Herstellung eines Lebensmittelzusatzes keine Option, da es toxische und/oder bitter schmeckende Verbindungen (z. B. Methylethylketon, Isopropylalkohol, Dinatoniumbenzoat) enthält. Es gibt zwei Reinheitsstufen von Ethanol. 96 % Ethanol ist die höchste Konzentration an Ethanol, die durch Destillation erhältlich ist, da es sich um ein Azeotrop handelt. 100 % Ethanol wird durch Zugabe von beispielsweise Benzol erhalten, um die Azeotropzusammensetzung zu unterbrechen und eine weitere Destillation zu ermöglichen. Aus folgenden Gründen wurde 96 % Ethanol für die Experimente ausgewählt: Erstens waren Verunreinigungen mit toxischen Chemikalien (z. B. Benzol) unerwünscht. Zweitens war 96 % Ethanol billiger als 99-100 % Ethanol. Drittens ist 100 % Ethanol hygroskopisch und verliert schnell seine Konzentration. Viertens reichten 96 % Ethanol aus, um die gewünschten Ethanolkonzentrationen zu erreichen.

## Ein neues Verfahren und ein neues Produkt

In Rahmen der vorliegenden Erfindung wurde ein hochskalierbarer Prozess zur Reinigung eines wasserlöslichen Bio-PolyP in Pulverform aus polyP-reichem *S. cerevisiae* in Lebensmittelqualität entwickelt. In Kombination mit der Herstellung von polyP-reichen *S. cerevisiae* Zellen ermöglicht das hier entwickelte Verfahren erstmals die biotechnologische Herstellung des neuartigen Produkts - Bio-PolyP (d.h. erfindungsgemäßes-PolyP). Der lebensmittelechte Status des Bio-PolyP wurde durch die Verwendung eines allgemein als sicher anerkannten ("GRAS") Mikroorganismus (Wildtyp *S. cerevisiae* - Backhefe) und nur lebensmittelechte Chemikalien erreicht. Die Bio-PolyP-Gegenionen Natrium, Kalium und Magnesium sind für den menschlichen Verzehr sicher. Im Bio-PolyP wurden keine toxischen Metalle nachgewiesen. Die lineare Struktur des Bio-PolyP wurde durch die Art der Gesamt-PolyP-Messung sichergestellt. PolyP wurde enzymatisch zu $P_i$ durch *S. cerevisiae* Exopolyphosphatase 1 und *S. cerevisiae* anorganische Pyrophosphatase 1 hydrolysiert. Anschließend wurde das $P_i$ kolorimetrisch gemessen. Da die Enzyme zyklisches PolyP nicht hydrolysieren können, kann der Assay zyklisches PolyP [Ref. 11a: aJ. J. Christ, L. M. Blank, Anal. Biochem. 2018, 548, 82-90] nicht nachweisen. Daher waren alle gemessenen PolyPs der vorliegenden Erfindung - lineare PolyPs.

Während Erdalkalisalze von PolyP wasserunlöslich sind, sind Alkalisalze von PolyP wasserlöslich (S. 10 in Ref. [1b]: bl. S. Kulaev, V. M. Vagabov, T. V. Kulakovskaya, The biochemistry of inorganic polyphosphates, Second ed., John Wiley & Sons, Ltd, West Sussex (England), 2005, S. 671 in Ref. [3]: J. R. Van Wazer, Phosphorus and its compounds. Volume I: Chemistry, Interscience publishers, Inc., New York (USA), 1958). Von den Alkalimetallen können nur Natrium und Kalium in Lebensmitteln verwendet werden. Aus diesem Grund wurden sie in Rahmen der vorliegenden Erfindung ausgewählt. Darüber hinaus bietet Kalium eine Alternative für natriumarme menschliche Ernährung. Das Natrium-Bio-PolyP (Na-Bio-PolyP) enthielt zu gleichen Teilen molare Anteile an Natrium, Kalium und Magnesium als Gegenionen. Streng genommen war das Natrium-Bio-PolyP eher ein Natrium-Kalium-Magnesium-Bio-PolyP. Das Kalium Bio-PolyP war ein Kalium-Magnesium-Bio-PolyP. Obwohl das Magnesium die Auflösung des Bio-PolyP in Wasser verlangsamte, erlaubte es die Rückgewinnung des Bio-PolyP als Feststoff (anstelle eines viskosen Gels mit nur einwertigen Kationen) während der Ethanolfällung.

Das Ethanol, das für die präparative Extraktion benötigt wurde, kann durch Destillation wirtschaftlich zurückgewonnen werden. Darüber hinaus wird Ethanol als Nebenprodukt bei der Fermentation der polyP-reichen Zellen hergestellt [Ref. 12: J. J. Christ, L. M. Blank, FEMS yeast research 2019, 19]. Die präparative Extraktion erfordert nur wenige kostengünstige Chemikalien (HCl, NaOH oder KOH, NaCl oder KCl). Das primäre Abfallprodukt war NaCl oder KCl, die umweltverträglich sind und kostengünstig entsorgt werden können. Die Zelltrümmer enthielten noch etwas PolyP. Eine mögliche Anwendung wäre als $P_i$- und Mineraldünger.

Der erfindungsgemäße Prozess wurde auf "einfache" Weise gestaltet, um ein "einfaches" Hochskalieren zu ermöglichen. Optional können die Zentrifugationsschritte durch Filtrationsschritte ersetzt werden. Es sollte geprüft werden, ob Zentrifugation oder Filtration bei einem Hochskalieren wirtschaftlicher ist. Die Trocknungsmethode kann geändert werden, solange die Prozessbedingungen nicht zu hart (zu heiß für zu lange) sind. Mögliche Alternativen sind Tunneltrocknung, Rotationstrocknung oder Schalentrocknung. Dementsprechend kann das Fräsverfahren etwas frei gewählt werden. Mögliche Optionen sind Kugelmahlen oder Strahlmahlen.

Das größte Problem bei der chemischen PolyP-Synthese liegt in der Abhängigkeit vom reinen Substrat ($P_i$). $P_i$ wird hauptsächlich in Marokko und der Westsahara abgebaut (eine politisch instabile Region, in der die meisten der globalen fossilen $P_i$-Reserven zu finden sind), gereinigt und beispielsweise in europäische Länder importiert. Die fossilen $P_i$-Reserven werden in einigen hundert Jahren erschöpft sein [Ref. 22: N. Gilbert, Nature 2009, 461, 716-718]. Die Qualität des $P_i$-Gesteins in Bezug auf den Gehalt an schweren und radioaktiven Metallen nimmt bereits ab [Ref. 22: N. Gilbert, Nature 2009, 461, 716-718]. Es müssen Strategien für ein $P_i$-Recycling aus ungenutzten Abfallströmen (z. B. aus Pflanzenmaterial [Ref. 23: aL. Carraresi, S. Berg, S. Bröring, Journal of Cleaner Production 2018, 183, 87-101; bK. R. Herrmann, A. J. Ruff, B. Infanzon, U. Schwaneberg, Appl. Microbiol. Biotechnol. 2019, 103, 6435-6448]) entwickelt werden. Diese Abfallströme enthalten in der Regel nur wenig $P_i$ (< 5 %). Die chemische PolyP-Synthese kann nicht direkt aus solch unreinem $P_i$ durchgeführt werden. Das hier entwickelte erfindungsgemäße Verfahren ist von Wert, da die polyP-reiche Zellmassenproduktion mit $P_i$ erfolgen kann, das aus $P_i$-Abfallströmen gewonnen wurde. Die potenziellen Auswirkungen eines solchen $P_i$-Recyclings aus $P_i$-Abfallströmen in eine wertvolle Verbindung (reines, langkettiges, lebensmitteltaugliches, wasserlösliches PolyP) sind enorm. $P_i$ sollte recycelt werden, um eine kreisförmige $P_i$-Wirtschaft aufrechtzuerhalten. Aus wirtschaftlicher Sicht würde das Recycling von $P_i$ die Abhängigkeit von $P_i$-Importen in europäische Länder (und andere) bis zu einem gewissen Grad verringern und die Produktion eines wirklich "grünen" PolyP ermöglichen, das für die Herstellung von Bioprodukten notwendig ist.

Die PolyPs Budit 4 und Budit 7 werden in Lebensmitteln verwendet (durchschnittliche PolyP-Kettenlänge: 11 bzw. 20 P-Untereinheiten). Die Kettenlänge des kurzkettigen Bio-PolyP entsprach der von Budit 7. Die Kettenlänge des langkettigen Natrium-Bio-PolyP war 2-fach und die Kettenlänge des langkettigen Kalium-Bio-PolyP 1,5-fach höher als die Kettenlänge von Budit 4. Eine ähnliche Länge der PolyP-Kette sollte ein Indikator für ähnliche Anwendungsbereiche sein. Die Anwendung von Bio-PolyP ist u.a. in allen Lebensmittelanwendungen vorgesehen, in denen derzeit regulär chemisch hergestelltes PolyP verwendet wird (z. B. in Fleischprodukten wie Wurst und Dönerkebab oder in Weichkäse). Darüber hinaus kann es für technische Anwendungen (z. B. in Farben, in Flammschutzmitteln, in Industriereinigern) eingesetzt werden.

## Schlussfolgerung

in Rahmen der vorliegenden Erfindung wurde ein hochskalierbarer erfindungsgemäßer Prozess zur Reinigung von erfindungsgemäßem PolyP aus polyP-reichem *S. cerevisiae* entwickelt. In Kombination mit der Herstellung von polyP-reichem *S. cerevisiae* ermöglicht das erfindungsgemäße Verfahren die biotechnologische Herstellung des neuartigen Produkts "wasserlösliches lebensmitteltaugliches Bio-PolyP". Das hier entwickelte Verfahren öffnet die Tür für das biotechnologische $P_i$-Recycling aus ungenutzten $P_i$-Abfallströmen zu einem hochwertigen organischen ("Bio") Produkt.

## Ausführungsformen der vorliegenden Erfindung

In einer Ausführungsform betrifft die vorliegende Erfindung ein Verfahren zur Gewinnung von Polyphosphat aus polyphosphat-haltigen Hefezellen [beispielsweise sind die polyphosphat-haltigen Hefezellen dem Fachmann allgemein bekannt und hierin auch exemplarisch beschrieben, s. z.B. auch Christ und Blank (2019) FEMS Yeast Res. Vol. 19, (3), oder auch Schritt 1 im hierin beschriebenen experimentellen Abschnitt "Herstellung von Bio-PolyP" oder an anderer Stelle hierin, bevorzugte Beispiele von Hefezellen enthaltend: *S. cerevisiae, Candida utilis, S. pombe*], umfassend folgende Schritte:

(1) Aufschließen von polyphosphathaltigen Hefezellen nach deren Ernte durch Einfrieren und Auftauen;

i) vorzugsweise Einfrieren bei vorzugsweise -20°C bis voll durchgefroren;

ii) vorzugsweise Auftauen bei vorzugsweiser Raumtemperatur bis vollständig aufgetaut, d.h. vorzugsweise keine sichtbaren Kristalle oder Klumpen;

iii) optional kann der Grad des Aufschlusses mikroskopiert werden, bei teilweisem Aufschluss kann vorzugsweise Einfrieren und Auftauen ggfs. Wiederholt werden;

(2) Inkubieren einer wässrigen Lösung, enthaltend die aufgeschlossenen polyphosphat-haltigen Hefezellen bei einer Temperatur von ca. 70°C [vorzugsweise bis ca. 71°C, 72°C, 73°C, 74°C, 75°C, 76°C, 77°C, 78°C, 79°C oder 80°C] für ca. 10 Minuten [überraschenderweise und im Gegensatz zum Stand der Technik (Ref. 16: A. Kuroda, N. Takiguchi, T. Gotanda, K. Nomura, J. Kato, T. Ikeda, H. Ohtake, Biotechnol. Bioeng. 2002, 78, 333-338) oder WO2013/011996, liefern ca. 10 Min bei ca. 70°C die höchste Ausbeute und damit "Reinheit" von PolyP, s. z.B. Abbildung 2B im experimentellen Abschnitt hierin dazu];

(3) Entfernen von Zelldebris aus der wässrigen Lösung [vorzugsweise durch Sedimentation und/oder Zentrifugation];

(4) Entfernen von Nukleinsäuren und Proteinen aus der wässrigen Lösung durch Zugabe einer Säure; [vorzugsweise durch eine starke anorganische Säure, z.B. $H_2SO_4$, HCl, oder $HNO_3$, wobei Schwefelsäue und/oder Salpetersäure sind weniger bevorzugt, beispielsweise weil sie für den Menschlichen Verzehr unter toxikologischen Hinsicht bedenklich sind; HCl ist besonders bevorzugt, wobei HCl-Konzentration kann beispielsweise empirisch ermittelt werden, siehe z.B. Abbildung 2F im hierin beschriebenen experimentellen Abschnitt dazu];

(5) Neutralisieren der verbleibenden wässrigen Lösung [vorzugsweise kann der Grad der Neutralisation durch pH-Wert Messung bestimmt werden; bevorzugte Beispiele für Basen enthaltend: NaOH, KOH, $Na_2CO_3$, $K_2CO_3$; das Anion der Base könnte vorzugsweise zu Wasser reagieren oder ausgasen (z.B. Carbonat)]; und

(6) Gewinnen von Polyphosphat aus der wässrigen Lösung durch Fällung mittels eines Alkohols [z.B. mittels eines EtOH, Isopropanol oder Aceton; hierfür wird aber jeder Alkohol geeignet, der mit Wasser vollständig mischbar ist; Aceton und Isopropanol sind weniger bevorzugt, weil sie beispielsweise für den Menschlichen Verzehr unter toxikologischen Hinsicht bedenklich sind];

Um polyphosphat-haltigen Hefezellen zu erhalten, kann im Rahmen des erfindungsgemäßen Verfahrens u.a. folgende Verfahren zur Herstellung von polyP-reichen *S. cerevisiae*-Zellen verwendet werden:

**Herstellung von polyP-reichen *S. cerevisiae*-Zellen**

Dieses Protokoll kann zur Herstellung von *S. cerevisiae* Zellen mit 28 Gew% polyP (als $KPO_3$) in Trockenzellgewicht verwendet werden. Zwei Medien wurden verwendet für dieses Verfahren, wobei "Nährmedium Komponente A" und "Nährmedium Komponente B" als ein Medium angesehen wurden, da sie beide Bestandteil eines orthophosphathaltigen Mediums waren, jedoch nicht unverzüglich gemeinsam angesetzt sondern erst kurzfristig zusammengebracht wurden, um Verklumpungen zu vermeiden.

- Orthophosphat-Mangelmedium:
    - 133 mM Glucose, 10 mM $(NH_4)SO_4$, 13,3 mM KCl, 12,5 mM $Na_2$Succinat, 4 mM $CaCl_2$, 1x Vitamin- und 1x Spurenelementelösung (1.000x Vitaminlösung: 138,77 mM Myo-inositol, 8,12 mM Nicotinsäure, 4,91 mM Pyridoxalhydrochlorid, 2,96 mM Thiaminhydrochlorid, 2,10 mM Ca-D-pantothenat, 1,46 mM p-Aminobenzoesäure, und 0,20 mM D-Biotin; 100x Spurenelementelösung: 4,45 mM $Na_2$EDTA, 3,06 mM $CaCl_2$, 1,61 mM $H_3BO_3$, 1,56 mM $ZnSO_4$, 1,07 mM $FeSO_4$, 0,61 mM $MnCl_2$, 0,16 mM $Na_2MoO_4$, 0,12 mM $CoCl_2$, 0,12 mM $CuSO_4$, und 0,06 mM KI)
    - pH 5 mit HCl/ NaOH, gefiltert (0,2 µm Poren), Lagerung bei 4 °C
- Orthophosphat-haltiges Medium:
    - Nährmedium Komponente A
        - 277,5 mM Glucose, 66,6 mM $KH_2PO_4$

        - pH 6,4 mit HCl/ KOH, gefiltert (0,2 μm Poren), Lagerung bei 4 °C

    - Nährmedium Komponente B

        - 200 mM $MgC_2$

        - gefiltert (0,2 μm Poren), Lagerung bei RT

Das Orthophosphat-Mangelmedium wurde versetzt mit 1 ml 1.000x Vitamin-Lösung pro l und 10 ml 100x Spurenelementelösung pro l, um jeweils eine 1x Lösung im Medium zu erhalten.

Zur Herstellung der Vitaminlösung wurde das D-Biotin in 10 ml 100 mM NaOH gelöst, alle anderen Bestandteile wurden nach Verdünnung auf 750 ml unter pH-Kontrolle (pH 6,5 mit HCl/ NaOH) gelöst. Das Volumen wurde auf 1 l aufgefüllt und die Lösung gefiltert (0,2 μm Poren), die Lagerung erfolgte bei 4 °C. Zur Herstellung der Spurenelementelösung wurde $Na_2EDTA$ und $ZnSO_4$ in 750 ml Aqua bidest. gelöst. Nach Einstellung auf pH 6,0 mit 1 M NaOH wurden die übrigen Chemikalien hinzugegeben. Nach Einstellung auf pH 4,0 mit 1 M HCl wurde die Lösung gefiltert (0,2 μm Poren) und bei -20 °C gelagert. Die Nährmedien-Komponenten A und B wurden 1,11-fach (A) bzw. 10-fach konzentriert und im Volumenverhältnis A:B=9:1 gemischt, und zwar unmittelbar vor Inokulierung, um die Bildung (Verklumpung) von Magnesium-Phosphat-Präzipitaten zu vermeiden. Alle Medien wurden vor Inokulierung auf 30 °C erwärmt. Die Fermentation selbst erfolgte aerob oder anaerob, wobei die anaerobe Fermentation kaum nennenswert höhere PolyP-Mengen erzielte. Zellmasse wurde durch SD (synthetic defined; 0,68 Gew% Hefe-Stickstoffbasis ohne Aminosäuren, 2 Gew% Glucose, pH 5,4, gefiltert (0,2 μm Poren)) Medium erzeugt. Als Hefe wurden *S. cerevisiae* Zellen VH2.200 oder kommerziell erhältliche Hefe (Bäckerhefe, aus dem Supermarkt) eingesetzt. Nach Ernte durch Zentrifugation (5.000 g, 10 min) wurde die Zellmasse mit Aqua bidest. gewaschen und getrocknet (verstrichen auf $P_i$-freiem Filterpapier, Lufttrocknung 5 min bei RT). Das führte zu einer Zellpaste mit ca. 25 Gew% Trockenanteil, die bei 4 °C bis zu 2 Wochen gelagert wurde.

Das Animpfen (Inokulieren) des Orthophosphat-Mangelmediums erfolgte mit 2,5 g CWW (Zellfeuchtmasse oder Zellfeuchtgewicht, Cell Wet Weight) pro l (0,62 g CDW, Zelltrockenmasse oder Zelltrockengewicht, Cell Dry Weight). Die Fermentation erfolgte für 6 h bei 30 °C, gefolgt von Zentrifugation (5.000 g, 10 min), Waschen mit Aqua bidest. und Trocknung (auf $P_i$-freiem Filterpapier, Lufttrocknung 5 min bei RT). Anschließend wurden die Zellen für 17 h bei 4 °C gelagert. Die 17 h Lagerung begann bzw. endete, als die Schüttel- oder Rührplatte, die bei der Fermentation genutzt worden waren, ab- bzw. angestellt wurde. Die Zelldichte bei Animpfen des Orthophosphat-haltigen Mediums war 30 g CWW pro l (7,5 g CDW), die Fermentation erfolgte für 2,5 h bei 30 °C, gefolgt von Zentrifugation (5.000 g, 10 min), Waschen mit Aqua bidest. und Trocknung (auf $P_i$-freiem Filterpapier, Lufttrocknung 5 min bei RT). Zur PolyP-Analyse wurden Aliquots von 24 bis 26 mg in 2 ml Gefäßen bei -20 °C gelagert.

In einer weiteren Ausführungsform betrifft die vorliegende Erfindung ein erfindungsgemäßes Verfahren, weiterhin umfassend Schritt (7): Waschen des gewonnenen Polyphosphats mit einem Alkohol [vorzugsweise 50% Alkohol].

In einer weiteren Ausführungsform betrifft die vorliegende Erfindung ein erfindungsgemäßes Verfahren, weiterhin umfassend Schritt (8): Trocknen des gewonnenen Polyphosphats [vorzugsweise im Exsikkator mit Trocknungsmittel, z.B. Silikagel; weiter vorzugsweise durch Wärmebehandlung, Tunneltrocknung, Rotationstrocknung oder Tray-Trocknung].

In einer weiteren Ausführungsform betrifft die vorliegende Erfindung ein erfindungsgemäßes Verfahren, weiterhin umfassend Schritt (9): Mahlen des getrockneten Polyphosphats.

In einer weiteren Ausführungsform betrifft die vorliegende Erfindung ein erfindungsgemäßes Verfahren, wobei im Schritt (6) die Fällung mittels Alkoholes eine fraktionierte Fällung ist.

In einer weiteren Ausführungsform betrifft die vorliegende Erfindung ein erfindungsgemäßes Verfahren, wobei die fraktionierte Fällung durch aufeinander folgende Fällungen mit ansteigendem Volumen des Alkohols im Bezug auf das Gesamtvolumen der wässrigen Lösung erfolgt.

In einer weiteren Ausführungsform betrifft die vorliegende Erfindung ein erfindungsgemäßes Verfahren, wobei nach Zugabe eines ersten Volumens des Alkohols langkettiges Polyphosphat [vorzugsweise mit einer durchschnittlichen Kettenlänge von 32-44 P-Einheiten] durch Fällung gewonnen wird, wobei das erste Volumen des Alkohols vorzugsweise 15 Vol% beträgt.

In einer weiteren Ausführungsform betrifft die vorliegende Erfindung ein erfindungsgemäßes Verfahren, wobei nach dem Gewinnen von langkettigem Polyphosphat ein zweites Volumen des Alkohols zugegeben wird, wobei das zweite Volumen des Alkohols größer ist als das erste Volumen des Alkohols.

In einer weiteren Ausführungsform betrifft die vorliegende Erfindung ein erfindungsgemäßes Verfahren, wobei nach Zugabe des zweiten, größeren Volumens des Alkohols kurzkettiges Polyphosphat [vorzugsweise mit einer durchschnittlichen Kettenlänge von 11-12 P-Einheiten] durch Fällung gewonnen wird, wobei das zweite Volumen des Alkohols vorzugsweise zumindest 85 Vol% beträgt.

In einer weiteren Ausführungsform betrifft die vorliegende Erfindung ein erfindungsgemäßes Verfahren, wobei im Schritt (5) eine Base verwendet wird, die ein einwertiges Kation als Gegenion enthält.

In einer weiteren Ausführungsform betrifft die vorliegende Erfindung ein erfindungsgemäßes Verfahren, wobei im Schritt (5) eine Base verwendet wird, die (a) Natrium, oder (b) Kalium als Gegenion enthält.

In einer weiteren Ausführungsform betrifft die vorliegende Erfindung ein erfindungsgemäßes Verfahren, umfassend: (4') gegebenenfalls Entfernen von zwei-oder höherwertigen Kationen aus der wässrigen Lösung durch Zugabe eines Carbonatsalzes eines einwertigen Kations [vorzugsweise Natrium- oder Kaliumcarbonat].

In einer weiteren Ausführungsform betrifft die vorliegende Erfindung eine erfindungsgemäße Zusammensetzung, enthaltend getrocknetes Polyphosphat, wobei das getrocknete Polyphosphat [vorzugsweise kurzes und langes Na/K-PolyP zusammen, z.B. die eine oder mehrere in der Tabelle 1 definierten Eigenschaften aufweisen]:

(a) einen Reinheitsgrad von zumindest 80% aufweist, definiert als Gehalt von linearem Polyphosphat und Kristallwasser [beispielsweise wird das Kristallwasser als Gewichtsverlust während des Trocknens der Exsikkator-Trockensubstanz bei 120°C bis konstantes Gewicht erreicht ist definiert] in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Natriumionen und/oder Kaliumionen und Magnesiumionen als Gegenionen enthält [wobei vorzugsweise Mg Gegenion einen Vorteil hat, nämlich bei der Ethanolfällung kommt das PolyP nicht als visköses Gel, sondern als Feststoff runter. Das macht den Prozess günstiger, da Fest-Flüssig-Trennung billiger und einfacher ist als Flüssig-Flüssig-Trennung]; und/oder

(b) zumindest 20% (mol/mol) Natriumionen [vorzugsweise 30%] und/oder zumindest 20% (mol/mol) Kaliumionen [vorzugsweise 30%] und bis zu 50% [vorzugsweise 40, 35, 30] (mol/mol) Magnesiumionen als Gegenionen enthält [wobei vorzugsweise Mg Gegenion einen Vorteil hat, nämlich bei der Ethanolfällung kommt das PolyP nicht als visköses Gel, sondern als Feststoff runter. Das macht den Prozess günstiger, da Fest-Flüssig-Trennung billiger und einfacher ist als Flüssig-Flüssig-Trennung]; und/oder

(c) eine durchschnittliche Kettenlänge von 11-44 P-Einheiten aufweist, wobei die durchschnittliche Kettenlänge enzymatisch bestimmt wird, und/oder

(d) einen Anteil an zyklischem Polyphosphat von nicht mehr als 2% bezogen auf die Trockensubstanz aufweist [beispielsweise enthält industrielles PolyP >5% zyklisches PolyP, wobei zyklisches PolyP vorzugsweise ausgenommen ist, weil es beispielsweise keine höhervalenten Kationen komplexieren kann. Der Vorteil der erfindungsgemäßen Zusammensetzung liegt z.B. daran, dass die erfindungsgemäßen Zusammensetzung eine bakteriostatische Wirkung durch PolyP hat, weil die Mg komplexiert, was aber durch einen zu hohen Anteil an zyklischem PolyP gemindert wird; durch Komplexieren von Ca im z.B. Schmelzkäse wird der Schmelzkäse cremiger, weil das "harte" Ca komplexiert ist. Wie aus Tabelle 1 ersichtlich ist, weist insbesondere industrielles PolyP einen höheren Gehalt an zyklischem PolyP auf, was ungünstig ist. Es ist überraschend, dass das PolyP der vorliegenden Erfindung einen so geringen Gehalt an zyklischem PolyP aufweist, was es gegenüber anderen PolyP-Präparationen, insbesondere industriell hergestelltem PolyP vorteilhaft macht]; und/oder

(e) eine oder mehrere der in der Tabelle 1 definierten Eigenschaften von Natrium-Bio-PolyP (z.B. kurzes und/oder langes PolyP) und/oder Kalium-Bio-PolyP (z.B. kurzes und/oder langes PolyP) aufweist.

Im Stand der Technik konnte bei Extraktion von PolyP aus Hefe bestenfalls ein Reinheitsgrad von ca. 70% erreicht werden; z.B. in WO2013/011996. Jedoch enthält WO2013/011996 keinen Hinweis, geschweige denn eine Lehre, wie der Reinheitsgrad erhöht werden könnte. Tatsächlich kann der Reinheitsgrad aber nur dann erhöht werden, wenn die Hefen genügend PolyP hergestellt und dieses auch ausreichend, idealerweise vollumfänglich extrahiert werden kann. In WO2013/011996 ist jedoch kein solches Protokoll beschrieben, mit dem man PolyP in einem solchen Maß extrahieren kann wie mit den Protokollen bzw. Verfahren der vorliegenden Erfindung. Beispielsweise lehrt WO2013/011996 geradezu von der idealen Temperatur (ca. 70°C) und Zeit (ca. 10 Min.) für den Aufschluss von Hefezellen weg. Gleiches gilt für Ref. 16: A. Kuroda, N. Takiguchi, T. Gotanda, K. Nomura, J. Kato, T. Ikeda, H. Ohtake, Biotechnol. Bioeng. 2002, 78, 333-338.

In einer weiteren Ausführungsform betrifft die vorliegende Erfindung eine erfindungsgemäße Zusammensetzung, wobei das Polyphosphat erhältlich ist durch ein erfindungsgemäßes Verfahren der vorliegenden Erfindung [vorzugsweise kurzes und langes Na/K-PolyP zusammen, z.B. die eine oder mehrere in der Tabelle 1 definierten Eigenschaften aufweisen].

In einer weiteren Ausführungsform betrifft die vorliegende Erfindung eine erfindungsgemäße Zusammensetzung, wobei das getrocknete Polyphosphat [vorzugsweise langes und kurzes Na-PolyP, z.B. das eine oder mehrere in der Tabelle 1 definierten Eigenschaften aufweist]:

(a) einen Reinheitsgrad von zumindest 85% aufweist, definiert als Gehalt von linearem Polyphosphat und Kristallwasser in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Natriumionen, Kaliumionen und Magnesiumionen als Gegenionen enthält; und/oder

(b) zumindest 20% [vorzugsweise 30%] (mol/mol) Natriumionen, zumindest 20% (mol/mol) Kaliumionen [vorzugsweise 30%] und bis zu 50% [vorzugsweise 40, 30, 20%] (mol/mol) Magnesiumionen als Gegenionen enthält; und/oder

(c) eine durchschnittliche Kettenlänge von 11-44 P-Einheiten aufweist, wobei die durchschnittliche Kettenlänge enzymatisch bestimmt wird, und/oder

(d) einen Anteil an zyklischem Polyphosphat von nicht mehr als 2% bezogen auf die Trockensubstanz aufweist.

In einer weiteren Ausführungsform betrifft die vorliegende Erfindung eine erfindungsgemäße Zusammensetzung, wobei das getrocknete Polyphosphat [langes Na-PolyP, z.B. das eine oder mehrere in der Tabelle 1 definierten Eigenschaften aufweist]:

(a) einen Reinheitsgrad von zumindest 90% aufweist, definiert als Gehalt von linearem Polyphosphat und Kristallwasser in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Natriumionen, Kaliumionen und Magnesiumionen als Gegenionen enthält; und/oder

(b) zumindest 30% (mol/mol) Natriumionen, zumindest 30% [vorzugsweise 38%] (mol/mol) Kaliumionen und bis zu 35% [vorzugsweise 32%] (mol/mol) Magnesiumionen als Gegenionen enthält; und/oder

(c) eine durchschnittliche Kettenlänge von 40-44 P-Einheiten aufweist, wobei die durchschnittliche Kettenlänge enzymatisch bestimmt wird, und/oder

(d) einen Anteil an zyklischem Polyphosphat von nicht mehr als 2% bezogen auf die Trockensubstanz aufweist.

In einer weiteren Ausführungsform betrifft die vorliegende Erfindung eine erfindungsgemäße Zusammensetzung, wobei das getrocknete Polyphosphat [vorzugsweise kurzes Na-PolyP, z.B. das eine oder mehrere in der Tabelle 1 definierten Eigenschaften aufweist]:

(a) einen Reinheitsgrad von zumindest 85% aufweist, definiert als Gehalt von linearem Polyphosphat und Kristallwasser in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Natriumionen, Kaliumionen und Magnesiumionen als Gegenionen enthält; und/oder

(b) zumindest 30% [vorzugsweise 38%] (mol/mol) Natriumionen, zumindest 30% [vorzugsweise 36%] (mol/mol) Kaliumionen und bis zu 30% [vorzugsweise 26%] (mol/mol) Magnesiumionen als Gegenionen enthält; und/oder

(c) eine durchschnittliche Kettenlänge von 11 P-Einheiten aufweist, wobei die durchschnittliche Kettenlänge enzymatisch bestimmt wird, und/oder

(d) einen Anteil an zyklischem Polyphosphat von nicht mehr als 2% bezogen auf die Trockensubstanz aufweist.

In einer weiteren Ausführungsform betrifft die vorliegende Erfindung eine erfindungsgemäße Zusammensetzung, wobei das getrocknete Polyphosphat [vorzugsweise langes und kurzes K-PolyP, z.B. das eine oder mehrere in der Tabelle 1 definierten Eigenschaften aufweist]:

(a) einen Reinheitsgrad von zumindest 83% aufweist, definiert als Gehalt von linearem Polyphosphat und Kristallwasser in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Kaliumionen und Magnesiumionen als Gegenionen enthält, und/oder

(b) zumindest 50% [vorzugsweise 60%] (mol/mol) Kaliumionen und bis zu 50% [vorzugsweise 40%] (mol/mol) Magnesiumionen als Gegenionen enthält, und/oder

(c) eine durchschnittliche Kettenlänge von 12-33 P-Einheiten aufweist, wobei die durchschnittliche Kettenlänge enzymatisch bestimmt wird, und/oder

(d) einen Anteil an zyklischem Polyphosphat von nicht mehr als 2% bezogen auf die Trockensubstanz aufweist.

In einer weiteren Ausführungsform betrifft die vorliegende Erfindung eine erfindungsgemäße Zusammensetzung, wobei das getrocknete Polyphosphat [vorzugsweise langes K-PolyP, z.B. das eine oder mehrere in der Tabelle 1 definierten Eigenschaften aufweist]:

(a) einen Reinheitsgrad von zumindest 88% aufweist, definiert als Gehalt von linearem Polyphosphat und Kristallwasser in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Kaliumionen und Magnesiumionen als Gegenionen enthält,

(b) zumindest 60% [vorzugsweise 63%] (mol/mol) Kaliumionen und bis zu 40% [vorzugsweise 37%] (mol/mol) Magnesiumionen als Gegenionen enthält,

(c) eine durchschnittliche Kettenlänge von 32-33 P-Einheiten aufweist, wobei die durchschnittliche Kettenlänge enzymatisch bestimmt wird, und/oder

(d) einen Anteil an zyklischem Polyphosphat von nicht mehr als 2% bezogen auf die Trockensubstanz aufweist.

In einer weiteren Ausführungsform betrifft die vorliegende Erfindung eine erfindungsgemäße Zusammensetzung, wobei das getrocknete Polyphosphat [vorzugsweise kurzes K-PolyP, z.B. das eine oder mehrere in der Tabelle 1 definierten Eigenschaften aufweist]

(a) einen Reinheitsgrad von zumindest 83% aufweist, definiert als Gehalt von linearem Polyphosphat und Kristallwasser in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Kaliumionen und Magnesiumionen als Gegenionen enthält,

(b) zumindest 70% [vorzugsweise 74%] (mol/mol) Kaliumionen und bis zu 30% [vorzugsweise 26%] (mol/mol) Magnesiumionen als Gegenionen enthält,

(c) eine durchschnittliche Kettenlänge von 12 P-Einheiten aufweist, wobei die durchschnittliche Kettenlänge enzymatisch bestimmt wird, und/oder

(d) einen Anteil an zyklischem Polyphosphat von nicht mehr als 2% bezogen auf die Trockensubstanz aufweist.

In einer weiteren Ausführungsform betrifft die vorliegende Erfindung eine erfindungsgemäße Zusammensetzung, wobei die enzymatische Bestimmung der durchschnittlichen Kettenlänge von P-Einheiten des Polyphosphats colorimetrisch quantitativ unter Verwendung von Exopolyphosphatase, anorganischer Pyrophosphatase und einem Detektionsmittel durchgeführt wird. [wie dem Fachmann allgemein bekannt s. z.B. 7655, Abb. 1 in Christ et al. (2019), Anal Chem 91, 7654-7661].

In einer weiteren Ausführungsform betrifft die vorliegende Erfindung eine erfindungsgemäße Zusammensetzung, wobei die enzymatische Bestimmung der durchschnittlichen Kettenlänge von P-Einheiten des Polyphosphats fluorometrisch quantitativ unter Verwendung von Exopolyphosphatase, ATP Sulfurlyase, Hexokinase und Glucose-6-phosphat-Dehydrogenase durchgeführt wird. [wie dem Fachmann allgemein bekannt s. z.B. 7655, Abb. 1 in Christ et al. (2019), Anal Chem 91, 7654-7661].

In einer weiteren Ausführungsform betrifft die vorliegende Erfindung eine erfindungsgemäße Zusammensetzung, wobei eine wässrige 1% (w/v) Lösung davon einen pH-Wert zwischen 6 und 8 aufweist.

In einer weiteren Ausführungsform betrifft die vorliegende Erfindung eine erfindungsgemäße Zusammensetzung, wobei das Polyphosphat erhältlich (z.B. hergestellt) nach einem der Verfahren der vorliegenden Erfindung.

In einer weiteren Ausführungsform betrifft die vorliegende Erfindung eine erfindungsgemäße Zusammensetzung, wobei die Zusammensetzung (z.B. umfassend erfindungsgemäßes Polyphosphat) erhältlich (z.B. hergestellt) nach einem der Verfahren der vorliegenden Erfindung.

In einer weiteren Ausführungsform betrifft die vorliegende Erfindung eine erfindungsgemäße Zusammensetzung, erhältlich nach einem der erfindungsgemäßen Verfahren der vorliegenden Erfindung. In einer weiteren Ausführungsform ist das erfindungsgemäße Verfahren der vorliegenden Erfindung ein Verfahren zur Herstellung der erfindungsgemäßen Zusammensetzung (z.B. umfassend erfindungsgemäßes Polyphosphat).

In einer weiteren Ausführungsform betrifft die vorliegende Erfindung eine erfindungsgemäße Zusammensetzung, zur Verwendung als Medikament, z.B. als bakteriostatisches Mittel.

In einer weiteren Ausführungsform betrifft die vorliegende Erfindung eine erfindungsgemäße Zusammensetzung, zur Verwendung bei der Vorbeugung und/oder Behandlung einer Krankheit, eines Symptomenkomplexes, eines Syndroms oder körperlichen Beschwerden ausgewählt aus der Gruppe bestehend aus: Hypophosphatemie, Rachitis, Osteomalazie, Osteoporose, Vitamin D-Mangel, Durchfall, Darmentzündung, Nährstoffmangel, Mineralstoffmangel, Protein-Energie-Untererernährung (PEU) und Phosphatdiabetes.

In einer weiteren Ausführungsform betrifft die vorliegende Erfindung ein erfindungsgemäßes Lebensmittel, Lebensmittelzusatzstoff, Nahrungsergänzungsmittel, Futtermittel, Düngemittel, Lebensmittel-, Lebensmittelzusatzstoff-, Nahrungsergänzungsmittel-, Futtermittel-, Düngemittel-Zwischenprodukt oder pharmazeutische Zusammensetzung, umfassend eine erfindungsgemäße Zusammensetzung der vorliegenden Erfindung.

In einer weiteren Ausführungsform betrifft die vorliegende Erfindung erfindungsgemäße Verwendung der Zusammensetzung der vorliegenden Erfindung bei der Herstellung eines Lebensmittels, eines Lebensmittelzusatzstoffs, eines Nahrungsergänzungsmittels, eines Futtermittels, eines Düngemittels (z.B. als Mineraldünger), eines Lebensmittel-, Lebensmittelzusatzstoff-, Nahrungsergänzungsmittel-, Futtermittel- oder Düngemittel-Zwischenproduktes, einer pharmazeutischen Zusammensetzung, eines Farbstoffs, eines Flammschutzmittels, eines Reinigungsmittels oder einer Mischung davon, vorzugsweise:

i) zur Stabilisierung und/oder Zartmachung von Produkten und/oder Zusammensetzungen, insbesondere Lebensmittelprodukten, z.B. Fleischprodukten, z.B. Wurst; und/oder

ii) als löslicher Kation-Austauscher; und/oder

iii) zur Komplexierung bivalenter Kationen, z.B. um Viskosität zu erhöhen; und/oder

iv) zur Erhöhung der hydrophilen Eigenschaften, insbesondere zur Erhöhung der hydrophilen Eigenschaften von Lebensmittelprodukten, z.B. um Fleischprodukte saftig zu halten; und/oder

v) als bakteriostatisches Mittel, insbesondere in/mit Lebensmittelprodukten, z.B. in/mit Fleischprodukten, z.B. "Kebab", wo das Fleisch für längere Zeit bei Umgebungstemperatur gehalten wird;

vi) als $P_i$- und/oder Mineraldünger.

Wie bereits beschrieben, nehmen Eigenschaften wie Komplexierungsfähigkeit (bspw. bei Weichkäse wünschenswert), Anzahl der Phosphoanhydrid-Bindungen, bakteriostatische Eigenschaften (bspw. Dönerfleisch) sowie Hydrophilie mit wachsender PolyP-Kettenlänge zu. Durch die höheren PolyP-Ketten kann auch eine geringere PolyP-Konzentration eingesetzt werden. Weiterhin kann $P_i$ durch Mikroorganismen (Hefe) bspw. aus Abwasserströmen aufgenommen und recycelt werden. Zudem kann erfindungsgemäß biologisch hergestelltes PolyP unter Umständen weniger restriktiven Kennzeichnungspflichten unterliegen, so dass die damit versetzten Nahrungsmittel ggf. "label-free" bleiben könnten. Schließlich bietet der Zusatz von PolyP zu Nahrungsmitteln wie im Zusammenhang mit der vorliegenden Erfindung möglich und beschrieben gegenüber dem Zusatz von reinem, chemisch synthetisierten PolyP den Vorteil, dass verbesserter Geschmack, erhöhte Bioverfügbarkeit sowie reduzierte Toxizität zutage tritt.

Der Mg Gegenion hat einen wichtigen Vorteil gegenüber dem chemisch-synthetisierten PolyP, nämlich bei der Ethanolfällung kommt das PolyP nicht als visköses Gel, sondern als Feststoff runter. Das macht den Prozess günstiger, da Fest-Flüssig-Trennung billiger und einfacher ist als Flüssig-Flüssig-Trennung.

Im Rahmen der hier vorgelegten und beschriebenen Erfindung kann prinzipiell jede Hefe eingesetzt werden, bspw. *Saccharomyces cerevisiae, Kluyveromyces marxianus* oder *Candida utilis,* bevorzugt *Saccharomyces cerevisiae* (Bäcker- oder Backhefe (bspw. Stamm VH2.200 (von der Versuchsanstalt der Hefeindustrie e. V., Berlin)), ober- oder untergärige Bierhefe (Weißbierhefe, Pilshefe)). In einer Ausführungsform der vorliegenden Erfindung wird Bäckerhefe eingesetzt, bevorzugt nicht gentechnisch veränderte Bäckerhefe ("non-GMO") oder "GRAS" (Generally Regarded As Safe) Hefe, um Kennzeichnungs- oder Zulassungspflichten zu vermeiden.

Die Ausführungsformen, welche den Gegenstand der vorliegenden Erfindung charakterisieren, sind hier beschrieben, in den Abbildungen gezeigt, in den Bespielen dargestellt, und/oder in den Ansprüchen definiert.

Im Rahmen der hier vorliegenden Erfindung umfassen die Begriffe "der/die/das" oder "ein/eine" im Singular und im Plural, auch jeweils den Plural bzw. den Singular, soweit nichts anderes bestimmt ist oder sich aus dem Kontext nichts anderes eindeutig herleiten lässt.

Die Begriffe "Trockenmasse" oder "Trockensubstanz", wie im Rahmen der hier vorliegenden Erfindung verwendet, können sich auf die Exsikkator-trockene Trockenmasse beziehen, z.B. wie in Tabelle 1 hierin dargestellt ist.

Der Begriff "mindestens", "mind.", oder "mehr als" vor einer Serie von Elementen ist im Rahmen der hier vorliegenden Erfindung so aufzufassen, dass er jeweils auch jedes einzelne Element dieser Serie bestimmt. Gleiches gilt analog für Begriffe wie "max(imal)", "bis zu" oder "höchstens".

Der Begriff "und/oder" wie im Rahmen der hier vorliegenden Erfindung verwendet umfasst sowohl den Begriff "und" allein, "oder" allein, als auch jede mögliche Kombination aus den Elementen, die von dem Begriff "und/oder" verbunden sind.

Der Begriff "etwa", "ungefähr", "ca." oder ähnlich wie im Rahmen der hier vorliegenden Erfindung verwendet ist synonym zu verstehen und bedeutet, sofern nicht explizit anders angegeben, innerhalb 20%, bevorzugt innerhalb 15%, weiter bevorzugt innerhalb 10%, weiter bevorzugt innerhalb 5%, weiter bevorzugt innerhalb 3%, weiter bevorzugt innerhalb 2% oder - besonders bevorzugt - 1% des jeweils folgenden Wertes oder Bereichs. Es ist jeweils auch exakt der folgende Wert oder Bereich umfasst.

Die Begriffe "umfassen", "einschließen", "beinhalten", "inkludieren" oder ähnlich sowie Beugungen davon wie im Rahmen der hier vorliegenden Erfindung verwendet implizieren die Einschließung der folgenden Elemente oder Gruppe(n) von Elementen, aber nicht die Ausschließung der jeweiligen Elemente oder Gruppen davon. Diese Begriffe umfassen auch den einschränkenden Begriff "bestehen aus" oder ähnlich sowie Beugungen davon, welcher wiederum, wenn expressis verbis aufgeführt, alles andere außer den darauf folgenden Elementen oder Schritten ausschließt.

Der Begriff "bestehend aus" oder ähnlich sowie Beugungen davon schließt, wenn expressis verbis aufgeführt, alles andere außer den darauf folgenden Elementen oder Schritten aus. Der Begriff "im Wesentlichen bestehend aus" oder ähnlich sowie Beugungen davon hingegen schließt keine Elemente oder Schritte aus, welche den grundlegenden Charakter des folgenden Elements oder Schrittes nicht in relevanter Weise beeinflussen.

Der Begriff "erhältlich durch" (oder "herstellbar durch" oder ähnliche Begrifflichkeiten) wie hier verwendet bedeutet, dass das nach dem entsprechenden beschriebenen Verfahren erhältliche Produkt auch durch andere Verfahren erhalten werden kann, soweit sich das Produkt strukturell nicht von einem Produkt unterscheidet, welches durch genau das beschriebene Verfahren erhalten wurde. Der Begriff "erhältlich durch" umfasst insoweit auch den einschränkenden Begriff "erhalten durch" (oder "hergestellt durch" oder ähnliche Begrifflichkeiten), wobei bei letzterem das Produkt tatsächlich durch genau das beschriebene Verfahren erhalten wurde.

Die Definitionen und Beschreibungen verschiedener Termini wie hier beschrieben dient lediglich dem Zweck der Beschreibung bestimmter Ausführungsformen und darf nicht als limitierend hinsichtlich des Gegenstands der Erfindung verstanden werden, welcher ausschließlich in den Ansprüchen definiert wird.

**Die folgenden Paragraphen beziehen sich ferner auf die folgenden Gegenstände der Beschreibung, wobei die Erfindung ausschließlich in den Ansprüchen definiert wird:**

1. Verfahren zur Gewinnung von Polyphosphat aus polyphosphat-haltigen Hefezellen [polyphosphat-haltigen Hefezellen sind dem Fachmann allgemein bekannt, s. z.B. Christ und Blank (2019) FEMS Yeast Res. Vol. 19, (3), siehe auch Schritt 1 im hierin beschriebenen experimentellen Abschnitt "Herstellung von Bio-PolyP" oder an anderer Stelle hierin; Im Rahmen der hier vorgelegten und beschriebenen Erfindung kann prinzipiell jede Hefe eingesetzt werden, bspw. *Saccharomyces cerevisiae, Kluyveromyces marxianus* oder *Candida utilis,* bevorzugt *Saccharomyces cerevisiae* (Bäcker- oder Backhefe (bspw. Stamm VH2.200 (von der Versuchsanstalt der Hefeindustrie e. V., Berlin)), ober- oder untergärige Bierhefe (Weißbierhefe, Pilshefe)) bevorzugte Beispiele von Hefen sind: *S. cerevisiae, Candida utilis,* oder *S. pombe*] umfassend:

(1) Aufschließen von polyphosphathaltigen Hefezellen nach deren Ernte durch Einfrieren und Auftauen;

i) vorzugsweise Einfrieren bei vorzugsweise -20°C bis voll durchgefroren;

ii) vorzugsweise Auftauen bei vorzugsweiser Raumtemperatur bis vollständig aufgetaut, d.h. keine sichtbaren Kristalle oder Klumpen.

iii) vorzugsweise kann der Grad des Aufschlusses bei Bedarf mikroskopiert werden, bei teilweisem Aufschluss kann Einfrieren und Auftauen ggfs. Wiederholt werden]

(2) Inkubieren einer wässrigen Lösung, enthaltend die aufgeschlossenen polyphosphat-haltigen Hefezellen bei einer Temperatur von ca. 70°C [vorzugsweise bis 80°C] für ca. 10 Minuten;

(3) Entfernen von Zelldebris aus der wässrigen Lösung [vorzugsweise durch Sedimentation und/oder durch Zentrifugation];

(4) Entfernen von Nukleinsäuren und Proteinen aus der wässrigen Lösung durch Zugabe einer Säure; [vorzugsweise eine starke anorganische Säure, z.B. $H_2SO_4$, HCl, oder $HNO_3$, wobei Schwefelsäue und/oder Salpetersäure sind aber weniger bevorzugt, z.B. weil sie für den Menschlichen Verzehr unter toxikologischen Hinsicht bedenklich sind; HCl ist besonders bevorzugt; HCl-Konz kann empirisch ermittelt werden, siehe Abb. 2F im hierin beschriebenen experimentellen Abschnitt];

(5) Neutralisieren der verbleibenden wässrigen Lösung [vorzugsweise kann der Grad der Neutralisation durch pH-Wert Messung bestimmt werden; Bevorzugte Beispiele für Basen sind: NaOH, KOH, $Na_2CO_3$, $K_2CO_3$; Das Anion der Base könnte vorzugsweise zu Wasser reagieren oder ausgasen (z.B. Carbonat)]; und

(6) Gewinnen von Polyphosphat aus der wässrigen Lösung durch Fällung mittels eines Alkohols [z.B. EtOH, Isopropanol oder Aceton; hierfür wird jeder Alkohol geeignet, der mit Wasser vollständig mischbar ist; Aceton und Isopropanol sind weniger bevorzugt z.B. weil sie für den Menschlichen Verzehr unter toxikologischen Hinsicht bedenklich sind]

2 Verfahren nach einem vorhergehenden Gegenstände, weiterhin umfassend
(7) Waschen des gewonnenen Polyphosphats mit einem Alkohol. [vorzugsweise 50%]

3. Verfahren nach einem vorhergehenden Gegenstände, weiterhin umfassend
(8) Trocknen des gewonnenen Polyphosphats [vorzugsweise im Exsikkator mit Trocknungsmittel, z.B. Silikagel; weiter vorzugsweise durch Wärmebehandlung, Tunneltrocknung, Rotationstrocknung oder Tray-Trocknung]

4. Verfahren nach einem der vorhergehenden Gegenstände, weiterhin umfassend (9) Mahlen des getrockneten Polyphosphats.

5. Verfahren nach einem der vorhergehenden Gegenstände, wobei im Schritt (6) die Fällung mittels Alkohol eine fraktionierte Fällung ist.

6. Verfahren nach Gegenstand 5, wobei die fraktionierte Fällung durch aufeinander folgende Fällungen mit ansteigendem Volumen des Alkohols im Bezug auf das Gesamtvolumen der wässrigen Lösung erfolgt.

7. Verfahren nach Gegenstand 6, wobei nach Zugabe eines ersten Volumens des Alkohols langkettiges Polyphosphat [vorzugsweise mit einer durchschnittlichen Kettenlänge von 32-44 P-Einheiten] durch Fällung gewonnen wird, wobei das erste Volumen des Alkohols vorzugsweise 15 Vol% beträgt.

8. Verfahren nach Gegenstand 6 oder 7, wobei nach dem Gewinnen von langkettigem Polyphosphat ein zweites Volumen des Alkohols zugegeben wird, wobei das zweite Volumen des Alkohols größer ist als das erste Volumen des Alkohols.

9. Verfahren nach einem der Gegenstand 6 bis 8, wobei nach Zugabe des zweiten, größeren Volumens des Alkohols kurzkettiges Polyphosphat [vorzugsweise mit einer durchschnittlichen Kettenlänge von 11-12 P-Einheiten] durch Fällung gewonnen wird, wobei das zweite Volumen des Alkohols vorzugsweise zumindest 85 Vol% beträgt.

10. Verfahren nach einem der Gegenstände 1 bis 9, wobei im Schritt (5) eine Base verwendet wird, die ein einwertiges Kation als Gegenion enthält.

11. Verfahren nach einem der Gegenstände 1 bis 10, wobei im Schritt (5) eine Base verwendet wird, die

(a) Natrium, oder

(b) Kalium

als Gegenion enthält.

12. Verfahren nach einem der vorhergehenden Gegenstände, umfassend
(4') gegebenenfalls Entfernen von zwei-oder höherwertigen Kationen aus der wässrigen Lösung durch Zugabe eines Carbonatsalzes eines einwertigen Kations [vorzugsweise Natrium- oder Kaliumcarbonat].

13. Zusammensetzung, enthaltend getrocknetes Polyphosphat, wobei das getrocknete Polyphosphat [vorzugsweise kurzes und langes Na/K-PolyP zusammen, z.B. die eine oder mehrere in der Tabelle 1 definierten Eigenschaften aufweisen]

(a) einen Reinheitsgrad von zumindest 80% aufweist, definiert als Gehalt von linearem Polyphosphat und Kristallwasser [vorzugsweise wird das Kristallwasser als Gewichtsverlust während des Trocknens der Exsikkator-Trockensubstanz bei 120°C bis konstantes Gewicht erreicht ist definiert] in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Natriumionen und/oder Kaliumionen und Magnesiumionen als Gegenionen enthält, und/oder
(b) einen Reinheitsgrad von linearem Polyphosphat von zumindest 80% aufweist, vorzugsweise definiert als Gehalt von linearem Polyphosphat in der Trockensubstanz (z.B. lineares PolyP inkl. Gegenionen in Exsikkator-Trockenmasse z.B. wie in Tabelle 1 hierin dargestellt ist), weiter vorzugsweise ohne Kristallwasser [weiter vorzugsweise wird das Kristallwasser als Gewichtsverlust während des Trocknens der Exsikkator-Trockensubstanz bei 120°C bis konstantes Gewicht erreicht ist definiert], wobei das Polyphosphat Natriumionen und/oder Kaliumionen und Magnesiumionen als Gegenionen enthält,
(c) zumindest 20% (mol/mol) Natriumionen [vorzugsweise 30%] und/oder zumindest 20% (mol/mol) Kaliumionen [vorzugsweise 30%] und bis zu 50% [vorzugsweise 40, 35, 30] (mol/mol) Magnesiumionen als Gegenionen enthält,
(d) eine durchschnittliche Kettenlänge von 10-125 P-Einheiten [vorzugsweise 11-44 P-Einheiten] aufweist, wobei die durchschnittliche Kettenlänge enzymatisch bestimmt wird, und/oder
(e) einen Anteil an zyklischem Polyphosphat von nicht mehr als 5%, vorzugsweise nicht mehr als 2%, bezogen auf die Trockensubstanz aufweist;
(f) eine oder mehrere der in der Tabelle 1 definierten Eigenschaften von Natrium-Bio-PolyP (z.B. kurzes und/oder langes PolyP) und/oder Kalium-Bio-PolyP (z.B. kurzes und/oder langes PolyP) aufweist.

14. Zusammensetzung nach Gegenstand 13, wobei das Polyphosphat erhältlich ist durch ein Verfahren nach einem der Gegenstände 1-12 [vorzugsweise kurzes und langes Na/K-PolyP zusammen, z.B. die eine oder mehrere in der Tabelle 1 definierten Eigenschaften aufweisen], weiter vorzugsweise ein Verfahren nach einem der Gegenstände 1-12 ist ein Verfahren zur Herstellung der Zusammensetzung nach Gegenstand 13.

15. Zusammensetzung nach Gegenstand 13, wobei das getrocknete Polyphosphat [vorzugsweise langes und kurzes Na-PolyP, z.B. die eine oder mehrere in der Tabelle 1 definierten Eigenschaften aufweisen]

(a) einen Reinheitsgrad von zumindest 85% aufweist, definiert als Gehalt von linearem Polyphosphat und Kristallwasser in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Natriumionen, Kaliumionen und Magnesiumionen als Gegenionen enthält, und/oder
(b) einen Reinheitsgrad von linearem Polyphosphat von zumindest 85% aufweist, vorzugsweise definiert als Gehalt von linearem Polyphosphat in der Trockensubstanz (weiter vorzugsweise ohne Kristallwasser), wobei das Polyphosphat Natriumionen und/oder Kaliumionen und Magnesiumionen als Gegenionen enthält,
(c) zumindest 20% [vorzugsweise 30%] (mol/mol) Natriumionen, zumindest 20% (mol/mol) Kaliumionen [vorzugsweise 30%] und bis zu 50% [vorzugsweise 40, 30, 20%] (mol/mol) Magnesiumionen als Gegenionen enthält,
(d) eine durchschnittliche Kettenlänge von 10-125 P-Einheiten [vorzugsweise 11-44 P-Einheiten] aufweist, wobei die durchschnittliche Kettenlänge enzymatisch bestimmt wird, und/oder
(e) einen Anteil an zyklischem Polyphosphat von nicht mehr als 5%, vorzugsweise nicht mehr als 2%, bezogen auf die Trockensubstanz aufweist.

16. Zusammensetzung nach Gegenstand 15, wobei das Polyphosphat erhältlich ist durch ein Verfahren nach einem der Gegenstände 1-11(a) [vorzugsweise langes und kurzes Na-PolyP, z.B. die eine oder mehrere in der Tabelle 1 definierten Eigenschaften aufweisen], weiter vorzugsweise ein Verfahren nach einem der Gegenstände 1-11(a) ist ein Verfahren zur Herstellung der Zusammensetzung nach Gegenstand 15.

17. Zusammensetzung nach Gegenstand 13 oder 15, wobei das getrocknete Polyphosphat [langes Na-PolyP, z.B. das eine oder mehrere in der Tabelle 1 definierten Eigenschaften aufweist]

(a) einen Reinheitsgrad von zumindest 90% aufweist, definiert als Gehalt von linearem Polyphosphat und Kristallwasser in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Natriumionen, Kaliumionen und Magnesiumionen als Gegenionen enthält, und/oder
(b) einen Reinheitsgrad von linearem Polyphosphat von zumindest 90% aufweist, vorzugsweise definiert als Gehalt von linearem Polyphosphat in der Trockensubstanz (weiter vorzugsweise ohne Kristallwasser), wobei das Polyphosphat Natriumionen und/oder Kaliumionen und Magnesiumionen als Gegenionen enthält,
(c) zumindest 30% (mol/mol) Natriumionen, zumindest 30% [vorzugsweise 38%] (mol/mol) Kaliumionen und bis zu 35% [vorzugsweise 32%] (mol/mol) Magnesiumionen als Gegenionen enthält,
(d) eine durchschnittliche Kettenlänge von 40-44 P-Einheiten aufweist, wobei die durchschnittliche Kettenlänge enzymatisch bestimmt wird, und/oder
(e) einen Anteil an zyklischem Polyphosphat von nicht mehr als 5%, vorzugsweise nicht mehr als 2%, bezogen auf die Trockensubstanz aufweist.

18. Zusammensetzung nach Gegenstand 17 wobei das Polyphosphat erhältlich ist durch ein Verfahren nach einem der Gegenstände 1-7, 10 und 11(a) [vorzugsweise langes Na-PolyP, z.B. das eine oder mehrere in der Tabelle 1 definierten Eigenschaften aufweist], weiter vorzugsweise ein Verfahren nach einem der Gegenstände 1-7, 10 und 11(a) ist ein Verfahren zur Herstellung der Zusammensetzung nach Gegenstand 17.

19. Zusammensetzung nach Gegenstand 13 oder 15, wobei das getrocknete Polyphosphat [vorzugsweise kurzes Na-PolyP, z.B. das eine oder mehrere in der Tabelle 1 definierten Eigenschaften aufweist]

(a) einen Reinheitsgrad von zumindest 85% aufweist, definiert als Gehalt von linearem Polyphosphat und Kristallwasser in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Natriumionen, Kaliumionen und Magnesiumionen als Gegenionen enthält, und/oder
(b) einen Reinheitsgrad von linearem Polyphosphat von zumindest 85% aufweist, vorzugsweise definiert als Gehalt von linearem Polyphosphat in der Trockensubstanz (weiter vorzugsweise ohne Kristallwasser), wobei das Polyphosphat Natriumionen und/oder Kaliumionen und Magnesiumionen als Gegenionen enthält,
(c) zumindest 30% [vorzugsweise 38%] (mol/mol) Natriumionen, zumindest 30% [vorzugsweise 36%] (mol/mol) Kaliumionen und bis zu 30% [vorzugsweise 26%] (mol/mol) Magnesiumionen als Gegenionen enthält,
(d) eine durchschnittliche Kettenlänge von 11 P-Einheiten aufweist, wobei die durchschnittliche Kettenlänge enzymatisch bestimmt wird, und/oder
(e) einen Anteil an zyklischem Polyphosphat von nicht mehr als 5%, vorzugsweise nicht mehr als 2%, bezogen auf die Trockensubstanz aufweist.

20. Zusammensetzung nach Gegenstand 19, wobei das Polyphosphat erhältlich ist durch ein Verfahren nach einem der Gegenstände 1-11(a) [vorzugsweise kurzes Na-PolyP, z.B. das eine oder mehrere in der Tabelle 1 definierten Eigenschaften aufweist], weiter vorzugsweise ein Verfahren nach einem der Gegenstände 1-11(a) ist ein Verfahren zur Herstellung der Zusammensetzung nach Gegenstand 19.

21. Zusammensetzung nach Gegenstand 13, wobei das getrocknete Polyphosphat [vorzugsweise langes und kurzes K-PolyP, z.B. das eine oder mehrere in der Tabelle 1 definierten Eigenschaften aufweist]

(a) einen Reinheitsgrad von zumindest 83% aufweist, definiert als Gehalt von linearem Polyphosphat und Kristallwasser in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Kaliumionen und Magnesiumionen als Gegenionen enthält, und/oder
(b) einen Reinheitsgrad von linearem Polyphosphat von zumindest 83% aufweist, vorzugsweise definiert als Gehalt von linearem Polyphosphat in der Trockensubstanz (weiter vorzugsweise ohne Kristallwasser), wobei das Polyphosphat Natriumionen und/oder Kaliumionen und Magnesiumionen als Gegenionen enthält,
(c) zumindest 50% [vorzugsweise 60%] (mol/mol) Kaliumionen und bis zu 50% [vorzugsweise 40%] (mol/mol) Magnesiumionen als Gegenionen enthält,
(d) eine durchschnittliche Kettenlänge von 12-33 P-Einheiten aufweist, wobei die durchschnittliche Kettenlänge enzymatisch bestimmt wird, und/oder
(e) einen Anteil an zyklischem Polyphosphat von nicht mehr als 5%, vorzugsweise nicht mehr als 2%, bezogen auf die Trockensubstanz aufweist.

22. Zusammensetzung nach Gegenstand 21, wobei das Polyphosphat erhältlich ist durch ein Verfahren nach einem der Gegenstände 1-10 und 11(b) [vorzugsweise langes und kurzes K-PolyP, z.B. die eine oder mehrere in der Tabelle 1 definierten Eigenschaften aufweisen], weiter vorzugsweise ein Verfahren nach einem der Gegenstände 1-10 und 11(b) ist ein Verfahren zur Herstellung der Zusammensetzung nach Gegenstand 21.

23. Zusammensetzung nach Gegenstand 13 oder 21, wobei das getrocknete Polyphosphat [vorzugsweise langes K-PolyP, z.B. das eine oder mehrere in der Tabelle 1 definierten Eigenschaften aufweist]

(a) einen Reinheitsgrad von zumindest 88% aufweist, definiert als Gehalt von linearem Polyphosphat und Kristallwasser in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Kaliumionen und Magnesiumionen als Gegenionen enthält, und/oder
(b) einen Reinheitsgrad von linearem Polyphosphat von zumindest 88% aufweist, vorzugsweise definiert als Gehalt von linearem Polyphosphat in der Trockensubstanz (weiter vorzugsweise ohne Kristallwasser), wobei das Polyphosphat Natriumionen und/oder Kaliumionen und Magnesiumionen als Gegenionen enthält,
(c) zumindest 60% [vorzugsweise 63%] (mol/mol) Kaliumionen und bis zu 40% [vorzugsweise 37%] (mol/mol) Magnesiumionen als Gegenionen enthält,
(d) eine durchschnittliche Kettenlänge von 32-33 P-Einheiten aufweist, wobei die durchschnittliche Kettenlänge enzymatisch bestimmt wird, und/oder
(e) einen Anteil an zyklischem Polyphosphat von nicht mehr als 5%, vorzugsweise nicht mehr als 2%, bezogen auf die Trockensubstanz aufweist.

24. Zusammensetzung nach Gegenstand 23 wobei das Polyphosphat erhältlich ist durch ein Verfahren nach einem der Gegenstände 1-7, 10 und 11(b) [vorzugsweise langes K-PolyP, z.B. das eine oder mehrere in der Tabelle 1 definierten Eigenschaften aufweist], weiter vorzugsweise ein Verfahren nach einem der Gegenstände 1-7, 10 und 11(b) ist ein Verfahren zur Herstellung der Zusammensetzung nach Gegenstand 23.

25. Zusammensetzung nach Gegenstand 13 oder 21, wobei das getrocknete Polyphosphat [vorzugsweise kurzes K-PolyP, z.B. das eine oder mehrere in der Tabelle 1 definierten Eigenschaften aufweist]

(a) einen Reinheitsgrad von zumindest 83% aufweist, definiert als Gehalt von linearem Polyphosphat und Kristallwasser in der Trockensubstanz (z.B. lineares PolyP inkl. Gegenionen und Kristallwasser in Exsikkator-Trockenmasse z.B. wie in Tabelle 1 hierin dargestellt ist), wobei das Polyphosphat Kaliumionen und Magnesiumionen als Gegenionen enthält, und/oder
(b) einen Reinheitsgrad von linearem Polyphosphat von zumindest 83% aufweist, vorzugsweise definiert als Gehalt von linearem Polyphosphat in der Trockensubstanz (weiter vorzugsweise ohne Kristallwasser), wobei das Polyphosphat Natriumionen und/oder Kaliumionen und Magnesiumionen als Gegenionen enthält,
(c) zumindest 70% [vorzugsweise 74%] (mol/mol) Kaliumionen und bis zu 30% [vorzugsweise 26%] (mol/mol) Magnesiumionen als Gegenionen enthält,
(d) eine durchschnittliche Kettenlänge von 12 P-Einheiten aufweist, wobei die durchschnittliche Kettenlänge enzymatisch bestimmt wird, und/oder
(e) einen Anteil an zyklischem Polyphosphat von nicht mehr als 5%, vorzugsweise nicht mehr als 2%, bezogen auf die Trockensubstanz aufweist.

26. Zusammensetzung nach Gegenstand 25, wobei das Polyphosphat erhältlich ist durch ein Verfahren nach einem der Gegenstände 1-10 und 11(b) [vorzugsweise kurzes K-PolyP, z.B. das eine oder mehrere in der Tabelle 1 definierten Eigenschaften aufweist], weiter vorzugsweise ein Verfahren nach einem der Gegenstände 1-10 und 11(b) ist ein Verfahren zur Herstellung der Zusammensetzung nach Gegenstand 25.

27. Zusammensetzung nach einem der Gegenstände 13 bis 26, wobei die enzymatische Bestimmung der durchschnittlichen Kettenlänge von P-Einheiten des Polyphosphats colorimetrisch quantitativ unter Verwendung von Exopolyphosphatase, anorganischer Pyrophosphatase und einem Detektionsmittel durchgeführt wird. [wie dem Fachmann allgemein bekannt s. z.B. 7655, Abb. 1 in Christ et al. (2019), Anal Chem 91, 7654-7661].

28. Zusammensetzung nach einem der Gegenstände 13 bis 26, wobei die enzymatische Bestimmung der durchschnittlichen Kettenlänge von P-Einheiten des Polyphosphats fluorometrisch quantitativ unter Verwendung von Exopolyphosphatase, ATP Sulfurlyase, Hexokinase und Glucose-6-phosphat-Dehydrogenase durchgeführt wird. [wie dem Fachmann allgemein bekannt s. z.B. 7655, Abb. 1 in Christ et al. (2019), Anal Chem 91, 7654-7661]

29. Zusammensetzung nach einem der Gegenstände 13 bis 28, wobei eine wässrige 1% (w/v) Lösung davon einen pH-Wert zwischen 6 und 8 aufweist.

30. Zusammensetzung nach einem der Gegenstände 13 bis 29, zur Verwendung als Medikament, z.B. als bakteriostatisches Mittel.

31. Zusammensetzung nach einem der Gegenstände 13 bis 30, zur Verwendung bei der Vorbeugung und/oder Behandlung einer Krankheit, eines Symptomenkomplexes, eines Syndroms oder körperlichen Beschwerden ausgewählt aus der Gruppe bestehend aus: Hypophosphatemie, Rachitis, Osteomalazie, Osteoporose, Vitamin D-Mangel, Durchfall, Darmentzündung, Nährstoffmangel, Mineralstoffmangel, Protein-Energie-Unterernährung (PEU) und Phosphatdiabetes.

32. Lebensmittel, Lebensmittelzusatzstoff, Nahrungsergänzungsmittel, Futtermittel, Düngemittel, Lebensmittel-, Lebensmittelzusatzstoff-, Nahrungsergänzungsmittel-, Futtermittel-, Düngemittel-Zwischenprodukt oder pharmazeutische Zusammensetzung umfassend eine Zusammensetzung nach einem der Gegenstände 13 bis 31.

33. Verwendung der Zusammensetzung nach einem der Gegenstände 13 bis 31 bei der Herstellung eines Lebensmittels, eines Lebensmittelzusatzstoffs, eines Nahrungsergänzungsmittels, eines Futtermittels, eines Düngemittels (z.B. als Mineraldünger), eines Lebensmittel-, Lebensmittelzusatzstoff-, Nahrungsergänzungsmittel-, Futtermittel- oder Düngemittel-Zwischenproduktes, einer pharmazeutischen Zusammensetzung, eines Farbstoffs, eines Flammschutzmittels, eines Reinigungsmittels oder einer Mischung davon, vorzugsweise:

i) zur Stabilisierung und/oder Zartmachung von Produkten und/oder Zusammensetzungen, insbesondere Lebensmittelprodukten, z.B. Fleischprodukten, z.B. Wurst; und/oder

ii) als löslicher Kation-Austauscher; und/oder

iii) zur Komplexierung bivalenter Kationen, z.B. um Viskosität zu erhöhen; und/oder

iv) zur Erhöhung der hydrophilen Eigenschaften, insbesondere zur Erhöhung der hydrophilen Eigenschaften von Lebensmittelprodukten, z.B. um Fleischprodukte saftig zu halten; und/oder

v) als bakteriostatisches Mittel, insbesondere in/mit Lebensmittelprodukten, z.B. in/mit Fleischprodukten, z.B. "Kebab", wo das Fleisch für längere Zeit bei Umgebungstemperatur gehalten wird;

vi) als $P_i$- und/oder Mineraldünger.

Die folgenden Beispiele dienen lediglich der Illustration der vorliegenden Erfindung, wobei die Erfindung ausschließlich in den Ansprüchen definiert wird.

## BEISPIELE

## MATERIALEN UND METHODEN

### Chemikalien, Materialien und Hefestämme

Das PolyP "Budit 4" (Los MV 5392) war ein Geschenk der Chemischen Fabrik Budenheim (Budenheim, Deutschland). Ethanol (96 %, nicht denaturiert), das "PolyP von Roth" (Art.-Nr. 4356, Lot 177257814), $P_i$-freies Filterpapier (Art.-Nr. HK16.1), die Aluminiumflaschen (Art.-Nr. P794.1) und die Dichtungen aus Polytetrafluorethylen (PTFE) (Art.-Nr. NT18.1, manuell zugeschnitten auf die Aluminium-Flaschendeckel) wurden von Carl Roth (Karlsruhe, Deutschland) bezogen. Das polyP "p100" (Lot 6T1202) war ein freundliches Geschenk von Dr. Toshikazu Shiba (Regenetiss Incorporation, Tokyo, Japan). Die Edelstahlröhrchen (Art.-Nr. 2007), die Stahlkugeln (Durchmesser 3,2 mm, Art.-Nr. 11079132ss), die Silikon-Gummistopfenkappen (Art.-Nr. 2008) und der Kugelmühle wurden von BioSpec Products (Bartlesville, OK, USA) bezogen. Reines Wasser (bezeichnet als "MilliQ-Wasser") wurde mit einem MilliQ Integral Water Purification System (Merck Millipore, Burlington, MA, USA) hergestellt. Der DNA-Leiter mit niedrigem Molekulargewicht (Art.-Nr. N3233S) wurde von NEB (Ipswich, MA, USA) gekauft. Die vorgefertigten Polyacrylamidgele (Artikel-Nr. EC62255) wurden von Thermo Fisher Scientific (Waltham, MA, USA) bezogen. Der Bäckerhefestämme S. *cerevisiae* VH2.200, ein Geschenk der Versuchsanstalt der Hefeindustrie e. V. (Berlin, Deutschland), wurde für die biotechnologische Herstellung von PolyP verwendet.

**Startprotokoll für die präparative Extraktion von PolyP aus *S. cerevisiae***

Dies ist das Startprotokoll für Abbildung 2. 100 mg Zellmasse wurden mit autoklaviertem Milli-Q-Wasser in einem 2 ml Reaktionsgefäß suspendiert. Die Suspension wurde für 1 Stunde bei 70°C und 750 U/min unter Rühren in einem Thermomixer "CTM" (HTA-Biotec, Bovenden, Deutschland) inkubiert. Das Rühren der Suspension wurde durch eine 3,2 mm Edelstahlperle pro 2 ml Reaktionsgefäß verstärkt. Nachdem die unlösliche Substanz durch Zentrifugation entfernt wurde (10.000 g, 5 Minuten), wurde der Überstand in ein neues Reaktionsgefäß überführt. Anschließend wurde der pH-Wert auf 7 mit NaOH (100 mM) und Phenolrot (0,02 Vol., 1,4 mM) als pH-Indikator eingestellt. Zur Fällung des PolyP wurden dann 0,02 Volumen NaCl (5 M) und 1 Volumen Ethanol zugegeben. Nach 1 Stunde Inkubation bei Raumtemperatur wurde das PolyP durch Zentrifugation (10.000 g, 5 Minuten) gewonnen.

**Herstellung von Bio-PolyP (erfindungsgemäßes PolyP)**

Das vollständig optimierte Protokoll für die Bio-PolyP-Produktion mit *S. cerevisiae* wird hier beschrieben. Die Schritte sind nummeriert, um eine bequeme Zuordnung zu ermöglichen. Die Flüssigkeiten wurden durch manuelles Verwirbeln des Behälters gemischt. NaCl und NaOH wurden in den Schritten 10 bis 12 verwendet, um Natrium-Bio-PolyP herzustellen. Dementsprechend wurden KCl und KOH zur Herstellung von Kalium-Bio-PolyP verwendet. Es wurde nicht denaturiertes Ethanol verwendet. Alle Schritte wurden bei Raumtemperatur durchgeführt, außer Schritt 6.

**1.** Die polyP-reichen *S. cerevisiae* Zellen wurden nach dem Fachmann bekanntem Verfahren vorbereitet [s. z.B. Ref. 12 - J. J. Christ, L. M. Blank, FEMS yeast research 2019, 19. oder an anderer Stelle hierin]. Dieses Protokoll beinhaltete zwei letzte Waschschritte der polyP-reichen Zellmasse mit autoklaviertem Milli-Q-Wasser und 5 Minuten Trocknung auf $P_i$-freiem Filterpapier, um eine Nasszellmasse zu erhalten, die ca. 25 % Trockenmasse enthielt.

**2.** Die Zellmasse wurde auf einen Aluminiumflasche übertragen, was für Schritt 5 erforderlich war. Zu beachten ist, dass die Aluminiumdichtung in der Kappe gegen eine PTFE-Dichtung ausgetauscht wurde. Ein relativ großer Aluminiumflasche (600 ml) wurde verwendet für ca. 23 g Zellen-Nassgewicht, um eine große Kontaktfläche zwischen dem Flascheninhalt und den Flaschenwänden zu gewährleisten, was die Wärmeübertragung in Schritt 5 optimiert. Edelstahl war ein geeignetes alternatives Flaschenmaterial. Das Gewicht der Nasszellenmasse wurde notiert (w2). Die Flasche wurde zur Lagerung in einem -20°C Gefrierschrank gelagert. Es war vorteilhaft, diesen Gefrierschritt nicht auszulassen, denn das Einfrieren und Auftauen half, die Zellen zu lysieren.

**3.** Die Zellmasse wurde aufgetaut, indem die Flasche für ca. 15 Minuten auf dem Schreibtisch stehen gelassen wurde. Größere Mengen an Zellmasse wurden in einem Wasserbad bei Raumtemperatur aufgetaut.

**4.** Der Zellmasse wurden 5 ml autoklaviertes Milli-Q-Wasser pro g Nasszellmasse (bezogen auf w2) zugesetzt. Die Suspension wurde kurzzeitig gemischt.

**5.** Die Flasche wurde 10 Minuten lang bei 70°C in einem stark schaukelnden Wasserbad inkubiert. Es war wichtig, eine Aluminiumflasche im Vergleich zu einer Glasflasche für eine optimale Wärmeübertragung zu verwenden. Der Flaschendeckel sollte bei Undichtigkeiten an der Dichtung nicht eingetaucht werden.

**6.** Die Flasche wurde 5 Minuten lang auf Eis gelegt, um den Inhalt auf Raumtemperatur abzukühlen. Der Inhalt wurde dann in einen Zentrifugationsbehälter überführt.

**7.** Das unlösliche Material wurde durch Zentrifugation entfernt (10.000 g, 5 Minuten). Das Pellet wurde entsorgt, das Volumen des Überstandes gemessen (v7) und der Überstand in einen neuen Zentrifugationsbehälter überführt. Es wurde mit ca. 5,2 ml Filtrat pro g Nasszellenmasse gerechnet.

**8.** Um das Protein und die Nukleinsäure auszufällen, wurden 0,02 Vol. v7 HCl (2,5 M) zugegeben und der Inhalt kurz gemischt. Da PolyP bei niedrigen pH-Werten hydrolysiert, wurden die Schritte 9 bis 11 schnell durchgeführt. Es war vorteilhaft, die Reihenfolge der Schritte 8 bis 10 nicht zu ändern, da die HCl-Ausfällung durch Alkaliaddition reversibel war.

**9.** Unmittelbar nach der HCl-Zugabe wurden das unlösliche Protein und die Nukleinsäure durch Zentrifugation entfernt (10.000 g, 15 Minuten). Die verlängerte Zentrifugationszeit war aufgrund der Feinartigkeit der suspendierten Feststoffe (Partikelgröße < 0,2 μm) vorteilhaft. Das Gewicht eines neuen Zentrifugationsbehälters wurde notiert, um später das Gewicht des gewonnenen PolyP zu bestimmen. Der Überstand wurde in diesen neuen Zentrifugationsbehälter überführt, während das Pellet entsorgt wurde.

**10.** Um die HCl aus Schritt 8 zu neutralisieren, wurden 0,02 Vol. v7 NaOH oder KOH (beide 2,5 M) unmittelbar nach der Zentrifugation zugegeben und die Lösung kurz gemischt.

**11.** Der pH-Wert wurde mit Hilfe einer pH-Elektrode auf 7 eingestellt. Es wurde NaOH oder KOH verwendet. Das verwendete Alkalivolumen wurde notiert. Es wurde ein Verbrauch zwischen 50 und 100 µl $OH^-$ (1 M) pro g Nasszellenmasse (bezogen auf w2) erwartet. Das Gesamtvolumen (v11) wurde durch Addition der Volumina der Schritte 8, 10 und 11 zu v7 berechnet.

**12.** Entweder $(0{,}020 - 0{,}010 * v7 * (v11)^{-1})$ vol v11 NaCl (5 M) oder $(0{,}028 - 0{,}014 * v7 * (v11)^{-1})$ vol v11 KCl (3,5 M) wurde zugegeben und die Lösung gemischt. Einschließlich der Schritte 8 und 10 betrug die endgültige NaCl- oder KCl-Konzentration nun 100 mM. v12 wurde berechnet, indem das Salzvolumen zu v11 addiert wurde.

**13.** Um das langkettige PolyP auszufällen, wurden 0,156 Vol. v12-Ethanol (96 % (v/v)) zugegeben und die Flüssigkeit kurz gemischt.

**14.** Die Suspension wurde 1 Stunde lang bei Raumtemperatur ohne Rühren inkubiert. Es war vorteilhaft, nicht zu rühren, um die Agglomeration des ausfällenden PolyP zu ermöglichen.

**15.** Die Suspension wurde zentrifugiert (10.000 g, 5 Minuten). Das Pellet und der Überstand wurden getrennt. Das Pellet enthielt das langkettige PolyP (ca. 30 - 40 P-Untereinheiten) und wurde gemäß den Schritten 19 - 21 behandelt. Der Überstand, der das kurzkettige PolyP (ca. 11 P-Untereinheiten) enthielt, wurde in einen neuen vorverwogenen Zentrifugationsbehälter überführt und gemäß den Schritten 16 - 21 behandelt.

**16.** Um das kurzkettige PolyP auszufällen, wurden 0,885 Vol. v12-Ethanol zugegeben und die Suspension kurz gemischt.

**17.** Die Suspension wurde 1 Stunde lang bei Raumtemperatur ohne Rühren inkubiert.

**18.** Die Suspension wurde zentrifugiert (9.000 g, 10 Minuten). Das kurzkettige PolyP befand sich im Pellet. Der Überstand wurde verworfen.

**19.** Zu den langkettigen und kurzkettigen PolyP-Pellets wurden jeweils 0,5 Vol. w2-Ethanol (50 % (v/v)) zugegeben und sanft über das Pellet geschwenkt, um Salzreste von den Pellets und Behälterwänden zu entfernen.

**20.** Das PolyP-Pellet verblieb im Zentrifugationsbehälter. Um das PolyP zu trocknen, wurde der offene Behälter für 1 Woche in einen Exsikkator gestellt, der mit trockenem Siliziumdioxid gefüllt wurde. Der Behälter wurde nach dem Trocknen gewogen, um das Gewicht des zurückgewonnenen Exsikkator-trockenen PolyP zu bestimmen.

**21.** Das Exsikkator-trockene PolyP wurde gemahlen, indem ca. 0,5 g PolyP (es ist vorteilhaft nicht mehr zu verwenden) und 3 Stahlperlen (3,2 mm Durchmesser) in ein 2 ml Edelstahlgefäß gegeben wurden. Das Edelstahlgefäß wurde mit einer Silikon-Gummi-Steckkappe verschlossen, bevor es für 2 Minuten in einer Kugelmühle gemahlen wurde. Es ist zu beachten, dass die Verwendung des Stahlgefäßes und der Silikonkappe vorteilhaft war, da andere Behältermaterialien beim Mahlen brechen können. Das PolyP wurde in einem Exsikkator gelagert, der bei Raumtemperatur mit trockenem Silica gefüllt wurde. Wenn dem trockenen PolyP Wasser zugegeben wurde, wurde das Gefäß sofort verwirbelt, um ein Verklumpen des PolyP am Boden des Behälters zu vermeiden. Zu beachten ist, dass die Auflösung des Bio-PolyP unter starkem Rühren 1 bis 2 Stunden dauerte.

**PolyP-Analytik**

Um den zellulären PolyP-Gehalt und die durchschnittliche PolyP-Kettenlänge in der Hefe zu bestimmen, wurde das PolyP aus den Zellen mit einer analytischen PolyP-Extraktion extrahiert [Ref. 13: J. J. Christ, L. M. Blank, Anal. Biochem. 2018, 563, 71-78.]. ATP-freies Wasser wurde durch Filtersterilisation (0,2 µm Poren) und anschließende Autoklavierung von MilliQ-Wasser hergestellt. Sofern nicht anders angegeben, wurde PolyP in Pulverform in ME-Puffer (25 mM MOPS, 2,5 mM EDTA, pH 7,0 mit NaOH, hergestellt mit ATP-freiem Wasser, sterilisiert, gelagert bei 4°C) gelöst und in Verdünnungspuffer (1 mM MOPS, 0,1 mM EDTA, pH 7 mit NaOH, hergestellt mit ATP-freiem Wasser, gelagert bei Raumtemperatur) für analytische Zwecke verdünnt. Das gesamte PolyP (nur lineares PolyP, kein zyklisches PolyP) und $P_i$

wurden enzymatisch bestimmt [Ref. 11a: aJ. J. Christ, L. M. Blank, Anal. Biochem. 2018, 548, 82-90, 14: J. J. Christ, S. Willbold, L. M. Blank, Anal. Chem. 2019, 91, 7654-7661.]. Für die Untersuchung des Fällungsverhaltens von chemisch hergestelltem PolyP (Abbildung 1) wurde das gesamte PolyP nach dem Trocknen des gelösten PolyP bei 120°C gravimetrisch gemessen. Die durchschnittliche Länge der PolyP-Kette wurde mit einem Enzymassay [Ref. 14: J. J. Christ, S. Willbold, L. M. Blank, Anal. Chem. 2019, 91, 7654-7661.] bestimmt. Um die Wasserlöslichkeit und den pH-Wert des PolyP zu bestimmen, wurde PolyP in einer Konzentration von 10 g pro Liter in ATP-freiem Wasser suspendiert. Um eine maximale Auflösung zu erreichen, wurde die Flüssigkeit 5 Stunden lang kräftig geschüttelt. Wenn sich ein Teil des PolyP nicht auflöste, wurde die Suspension zentrifugiert (5 Minuten, 10.000 g), das Pellet in einem Exsikkator für 7 Tage getrocknet und die unlösliche Substanz gewogen. Die PolyP-Kettenlängenverteilung wurde mit PAGE [Ref. 15: S. A. Smith, Y. Wang, J. H. Morrissey, Electrophoresis 2018, 39, 2454-2459] analysiert. Kurz gesagt, 48 nmol polyP * $lane^{-1}$ wurde für 35 Minuten bei 150 V bei Raumtemperatur auf einem 4 bis 20 %igen Polyacrylamid-Gel (Gradient) (8 cm * 8 cm * 8 cm * 1 mm) im TRIS-Borat-EDTA-Puffer getrennt. Der DNA-Leiter mit niedrigem Molekulargewicht von NEB wurde als Kettenlängenstandard verwendet. Die DNA-Fragmente gemessen 766, 500, 350, 250, 200, 150, 100, 75, 50 und 25 Basenpaaren. Die in Abbildung 3 dargestellten PolyP-Kettenlängen wurden aus den DNA-Kettenlängen [Ref. 15: S. A. Smith, Y. Wang, J. H. Morrissey, Electrophoresis 2018, 39, 2454-2459] berechnet. Das Gel wurde nach der Elektrophorese mit Toluidinblau O gefärbt. Der zyklische PolyP-Gehalt wurde mit $^{31}P$ Kernspinresonanz gemessen [Ref. 14: J. J. Christ, S. Willbold, L. M. Blank, Anal. Chem. 2019, 91, 7654-7661]. Der Wassergehalt im PolyP wurde durch gravimetrische Messung des Wasserverlustes bei 120°C quantifiziert. Chlorid wurde im PolyP mittels Ionenchromatographie untersucht. Die Nukleinsäuren im Bio-PolyP wurden spektrophotometrisch mit einem NanoDrop (Thermo Fisher Scientific) gemessen.

**Bestimmung des durchschnittlichen Molekulargewichts von PolyP, der PolyP-Reinheit und der durchschnittlichen Molekular-PolyP-Formel**

Die komplexe Berechnung der PolyP-Reinheit wird durch ein Beispiel veranschaulicht, das in Klammern dargestellt ist. Um die PolyP-Kationenzusammensetzung zu bestimmen, wurde PolyP in verdünntem $HNO_3$ gelöst. Die molaren Kationenkonzentrationen in dieser Lösung (z. B. 3,47 mM $Na^+$, 0,41 mM $Mg^{2+}$) wurden durch Atomabsorptionsspektroskopie oder optische Emissionsspektroskopie bestimmt. Das durchschnittliche Kationenmolekulargewicht (z. B. $(3,47 * 22,99 + 0,41 * 24,305) * (3,47 + 0,41)^{-1} = 23,1$ g * $mol^{-1}$) wurde als gewichteter Durchschnitt der molaren Kationenkonzentration und des Molekulargewichts ("*Mw*") berechnet. Die durchschnittliche Kationenladung (z. B. $(3,47 * 1 + 0,41 * 2) * (3,47 + 0,41)^{-1} = 1,106$ Ladung * $Kation^{-1}$) wurde als gewichteter Durchschnitt der molaren Kationenkonzentration und der Kationenladung berechnet. Das durchschnittliche Kationengewicht wurde durch die durchschnittliche Kationenladung dividiert, um das korrigierte durchschnittliche Kationenmolekulargewicht zu erhalten - $Molekulargewicht_{kation}$ (z. B. $23.1 * 1.106^{-1} = 20.9$ g * $mol^{-1}$). Der Grund für diese Korrektur ist, dass M in Gleichung 2 eine Ladung von 1 haben sollte. Das Molekulargewicht des pH-neutralen Polymers wurde nach Gleichung 4 berechnet, die von Gleichung 2 abgeleitet wurde. Gleichung 4 (Gl. 4) wurde nur verwendet, wenn das PolyP einen neutralen pH-Wert hatte (alle Bio-PolyPs). Für die sauren PolyPs (Budit 4, PolyP von Roth, P100) wurde Gleichung 5 verwendet, die von Gleichung 3 (Gl. 3) abgeleitet wurde. Das Molekulargewicht des Monomers wurde mit Gleichung 6 (Gl. 6) berechnet. Die durchschnittliche PolyP-Kettenlänge n wurde wie oben beschrieben bestimmt. Die Molekulargewichte von Wasserstoff, Phosphor und Sauerstoff betragen 1,0079, 30,974 und 15,999 g * $mol^{-1}$. Im Beispiel und unter der Annahme einer Kettenlänge von 20 P-Untereinheiten und eines pH-neutralen PolyP beträgt das Molekulargewicht von Polymer und Monomer 2035,7 bzw. 101,8 g * $mol^{-1}$.

$$Molekulargewicht_{Polymer,\ pH\ 7}\ [g * mol^{-1}] = (n\ [\text{P-Untereinheiten}] + 1) * Molekulargewicht_{Kation}\ [g * mol^{-1}] + Molekulargewicht_{Wasserstoff}\ [g * mol^{-1}] + n * Molekulargewicht_{Phosphor}\ [g * mol^{-1}] + (3 * n + 1) * Molekulargewicht_{Sauerstoff}\ [g * mol^{-1}] \quad \text{Gl. 4}$$

$$Molekulargewicht_{Polymer,\ pH\ 4}\ [g * mol^{-1}] = (n\ [\text{P-Untereinheiten}] * Molekulargewicht_{Kation}\ [g * mol^{-1}] + 2 * Molekulargewicht_{Wasserstoff}\ [g * mol^{-1}] + n * Molekulargewicht_{Phosphor}\ [g * mol^{-1}] + (3 * n + 1) * Molekulargewicht_{Sauerstoff}\ [g * mol^{-1}] \quad \text{Gl. 5}$$

$$Molekulargewicht_{Monomer}\ [g * mol^{-1}] = Molekulargewicht_{Polymer}\ [g * mol^{-1}] * (n\ [\text{P- Untereinheiten}])^{-1} \quad \text{Gl. 6}$$

Das PolyP wurde mit dem Konzentrationsgewicht $Konz_{PolyP}$ (z. B. 2 g PolyP * $L^{-1}$) gelöst. Die molare Gesamt-PolyP-Konzentration $mol\ Konz_{PolyP\ Monomer}$ (z. B. 0,018 mol PolyP (als Monomer) * $L^{-1}$) dieser Lösung wurde wie oben beschrieben gemessen. Die PolyP-Reinheit wurde nach Gleichung 7 (Gl. 7) berechnet. Im Beispiel wird eine Reinheit von 91,6 % (0,018 * 101,8 * 100 * $2^{-1}$) erreicht.

PolyP-Reinheit [% (w/w)] = (*mol Konz*$_{PolyP\ Monomer}$ [mol * $L^{-1}$] * *Molekulargewicht*$_{Monomer}$ [g * $mol^{-1}$] * 100) * (*Gewicht Konz*$_{PolyP}$ [g * $L^{-1}$])$^{-1}$ Gl. 7

Die relative molare Häufigkeit jedes Kations im Vergleich zu allen Kationen (z. B. 3,47 * 100 * (3,47 + 0,41) = 89,4 % (mol/mol) für Na; 0,41 * 100 * (3,47 + 0,41) = 10,6 % (mol/mol) für Mg) wurde durch Division der molaren Konzentration des Kations durch die Summe aller molaren Kationenkonzentrationen bestimmt. Die Anzahl der Kationenatome pro PolyP-Polymermolekül wurde gemäß Gleichung 8 (Gl. 8) bzw. Gleichung 9 (Gl. 9) für PolyP mit neutralem und saurem pH-Wert berechnet (z. B. 89,4 * (20 + 1) * (1.106 * 100)$^{-1}$ = 17,0 für Na; 10,6 * (20 + 1) * (1.106 * 100)$^{-1}$ = 2,0 für Mg).

Absolute Anzahl der Kationenatome pro pH-neutrales PolyP-Polymermolekül = relative molare Häufigkeit jedes Kations [% (mol/mol)] * (n [P-Untereinheiten] + 1) * (durchschnittliche Kationenladung [Ladung * $Kation^{-1}$] * 100)$^{-1}$ Gl. 8

Absolute Anzahl der Kationenatome pro saurem (pH 4) PolyP-Polymermolekül = relative molare Häufigkeit jedes Kations [% (mol/mol)] * (n [P-Untereinheiten]) * (durchschnittliche Kationenladung [Ladung * $Kation^{-1}$] * 100)$^{-1}$ Gl. 9

Die Molekularpolymerformel wurde dann mit 1 Wasserstoffatom für pH-neutrales PolyP und 2 Wasserstoffatomen für saures PolyP, n Phosphoratomen und 3 * n + 1 Sauerstoffatomen ergänzt. Die Molekularpolymerformel im Beispiel ist $Na_{17}Mg_2HP_{20}O_{61}$. Diese Summenformel sollte als durchschnittlich angesehen werden, da alle getesteten PolyPs eine beträchtliche Polydispersität hatten.

## ERGEBNISSE

Der vorgesehene Produktionsprozess für die Produktion von Bio-PolyP beinhaltete drei Prozessschritte. Im ersten Prozessschritt wurde *Saccharomyces cerevisiae* mit PolyP durch PolyP Hyperakkumulation beladen. Dieser Schritt wurde bereits in einer früheren Studie entwickelt [Ref. 12: J. J. Christ, L. M. Blank, FEMS yeast research 2019, 19]. Im zweiten Prozessschritt wurde das PolyP aus der Hefezelle gelöst und in eine wässrige Lösung gebracht. Im dritten Prozessschritt wurde das gelöste PolyP durch Fällung gewonnen. Die letzten beiden Prozessschritte wurden in Rahmen der vorliegenden Erfindung entwickelt.

### Optimale Bedingungen für die Fällung, Trocknung und Vermahlung von PolyP

Das erste Ziel war es, zu verstehen, welche Prozessbedingungen für die quantitative Fällung, die schonende und industriell einsetzbare Trocknung und Vermahlung von PolyP erforderlich sind. Um diese Ziele zu erreichen, wurden ca. 100 g reines chemisch synthetisiertes PolyP benötigt. Die Prozessbedingungen, die mit diesem chemisch hergestellten PolyP entwickelt wurden, wurden später für die Herstellung von Bio-PolyP verwendet. Aus diesem Grund wurden die Länge und Konzentration der PolyP-Kette auf der Grundlage der Werte ausgewählt, die von der Bio-PolyP-Produktion erwartet wurden. Das Natrium-PolyP "Budit 4" war das längste PolyP (durchschnittliche Kettenlänge von 20 P-Untereinheiten), das in großen Mengen verfügbar war und daher für die Experimente ausgewählt wurde. Bei einer Extraktionsausbeute von 86 % (mol/mol) während der Freisetzung von Bio-PolyP aus S. *cerevisiae* würden ca. 30 g Natrium-PolyP * $L^{-1}$ vor der PolyP-Präzipitation erhalten. Diese Budit 4 Konzentration wurde in den folgenden Experimenten verwendet.

Wenn nicht anders angegeben, wurden alle Schritte bei Raumtemperatur durchgeführt. Die Menge des Lösungsmittels wird im Einheitsvolumen angegeben (vol; 1 vol entspricht dem Mischen in einem volumetrischen Verhältnis von 1:1). Um zu überprüfen, ob PolyP mit einem organischen Lösungsmittel ausgefällt werden kann, wurde Budit 4 mit 2 Vol. Ethanol, Propanol oder Aceton mit oder ohne NaCl ausgefällt (Abbildung 1A). Das PolyP wurde nach der Fällung als viskoses, klebriges Gel gesammelt. Die Rückgewinnung ohne NaCl war bei allen organischen Lösungsmitteln unbefriedigend (≤ 44 %). Mit der Kombination aus NaCl und Ethanol oder NaCl und Aceton wurde fast das gesamte PolyP gewonnen (95 bzw. 97 %). Die Rückgewinnung mit Isopropanol und NaCl war etwas geringer, aber immer noch akzeptabel (92 %). Da Bio-PolyP unter anderem als Lebensmittelzusatzstoff verwendet wird, war eine geringe Toxizität der Fällungsmittel unerlässlich. Aus diesem Grund wurde Ethanol für das Standard-Fällungsprotokoll gewählt.

Es wurde der Schluss gezogen, dass Budit 4 durch die kombinierte Verwendung von Salz und Ethanol vollständig ausgefällt werden kann. Die Zielprodukte wurden als Natrium- und Kalium-PolyP definiert. Die verschiedenen PolyP-Kationenzusammensetzungen wurden durch Verdrängung der Gegenionen erreicht. Zu diesem Zweck wurden NaCl und KCl in den folgenden Experimenten getestet. Der saure pH-Wert der Budit 4-Lösungen wurde vor der Fällung auf pH 7 titriert, da PolyP im sauren und alkalischen Milieu spontan hydrolysiert. NaOH und KOH wurden verwendet, um den pH-Wert bei der Herstellung von Natrium- bzw. Kalium-PolyP einzustellen.

Budit 4 wurde mit einer Kombination aus verschiedenen Konzentrationen von NaCl oder KCl und 2 Vol. Ethanol ausgefällt (Abbildung 1B). In einer Konzentration von 50 bis 750 mM beider Salze wurde Budit 4 umfangreich gewonnen (≥ 95 %). Die Rückgewinnung mit 50 bis 750 mM NaCl war konstant bei 95 %. Da das gewonnene PolyP gravimetrisch gemessen wurde, wurde der Schluss gezogen, dass NaCl selbst nicht ausfällt. Die Fällung mit 500 und 750 mM KCl führte zur Bildung eines kristallinen Präzipitats (wahrscheinlich festes KCl) auf dem PolyP-Sediment. Die erhöhte Rückgewinnung mit 250 bis 750 mM KCl wurde durch die Fällung von KCl selbst und/oder die Verdrängung des Natrium-PolyP-Kations in das schwerere Kalium erklärt. Es sei auch darauf hingewiesen, dass Budit 4 mit 750 mM NaCl, aber nicht mit 750 mM KCl, ohne die Verwendung von Ethanol ausfiel. Abschließend kann entweder 50 bis 500 mM NaCl oder 50 bis 250 mM KCl in Kombination mit 2 Vol. Ethanol empfohlen werden. Aus wirtschaftlichen Gründen wurde für das Standardfällungsprotokoll (100 mM NaCl oder KCl) eine niedrige Salzkonzentration gewählt.

Der Einfluss der Ethanolkonzentration auf die Fällung von PolyP wurde als nächstes getestet (Abbildung 1C). Die Art des Salzes (NaCl oder KCl) hatte fast keinen Einfluss auf die Rückgewinnung. 0,15, 0,25 und 0,5 Vol. Ethanol waren geeignete Konzentrationen für die fraktionierte Fällung, da nur ca. 40, 76 bzw. 87 % des PolyPs gewonnen wurden. Weitere Experimente zeigten, dass Ethanolvolumina von 0,13 und 0,14 die minimal erforderlichen Konzentrationen waren, um PolyP-Präzipitation in Kombination mit 100 mM NaCl bzw. KCl zu erzeugen. Die maximale Rückgewinnung wurde mit 3 Vol. Ethanol (97 % mit NaCl oder KCl) gemessen.

Zwei Effekte wurden visuell beobachtet, wenn verschiedene Ethanolkonzentrationen getestet wurden (Abbildung 1C). Zunächst beeinflusste die Ethanolkonzentration die Viskosität des gewonnenen PolyP-Gels. Nach der Fällung mit 3 Vol. Ethanol war das PolyP-Gel so zähflüssig, dass es nicht floss, wenn das Reaktionsgefäß zur Seite gekippt wurde. Nach der Fällung mit 0,15 Vol. Ethanol war das PolyP-Gel fast so dünnflüssig wie Wasser. Zwischen 0,15 und 3 Vol. Ethanol wurde ein dosisabhängiger Anstieg der Viskosität des PolyP-Gels beobachtet. Dieser Effekt wurde unabhängig von der Salzart beobachtet. Darüber hinaus lösen sich die dünnflüssigen PolyP-Gele schneller in Wasser als die dickflüssigeren. Es wurde spekuliert, dass die höheren Ethanolkonzentrationen den Wassergehalt reduzieren und damit die Viskosität des PolyP-Gels erhöhen. Zweitens waren die mit NaCl ausgefällten PolyP-Gele viskoser als die mit den gleichen KCl-Konzentrationen erhaltenen PolyP-Gele. Die Hypothese, dass Kalium den Wassergehalt erhöht und damit die Viskosität des PolyP-Gels reduziert, wurde abgelehnt (siehe unten und Abbildung 1D). Vielleicht beeinflussen die unterschiedlichen Hydrationshüllen von Natrium und Kalium die Wechselwirkung zwischen PolyP-Ketten und damit die Viskosität des PolyP-Gels.

1 Vol. Ethanol wurde für das Standard-Fällungsprotokoll gewählt. Diese niedrige Ethanolkonzentration war u.a. aus wirtschaftlichen Gründen erwünscht. Mit 1 Vol. Ethanol wurden ausreichende Rückgewinnungen von 92 % mit NaCl und 91 % mit KCl gemessen.

Darüber hinaus wurde spekuliert, dass höhere Ethanolkonzentrationen die Fällung von Verunreinigungen bei der Herstellung von Bio-PolyP erleichtert hätten. Viskose PolyP-Gele, die mit höheren Ethanolkonzentrationen gewonnen wurden, zeigten die Konsistenz eines klebrigen Feststoffes, was die Handhabung schwerfällig machte. Das mit 1 Vol. Ethanol hergestellte PolyP-Gel verhielt sich wie eine viskose Flüssigkeit (ähnlich Glycerin).

Ein geeigneter visueller Marker für geeignete Fällungsbedingungen war die Trübung der Budit-4-Lösung. Als Budit 4 in Wasser gelöst wurde, erschien es als transparente Lösung. Unter Zusatz eines organischen Lösungsmittels wurde die Budit 4-Lösung trüb / milchigweiß, unabhängig davon, ob Salz verwendet wurde oder nicht. Anscheinend wurde das PolyP zunächst als feine Emulsion oder Suspension ausgefällt. Nach der Zentrifugation der Emulsion wurden zwei Ergebnisse beobachtet. Bei geeigneten Reaktionsbedingungen wurde der Überstand transparent und das PolyP als klares viskoses Gel ausgefällt. Bei ungeeigneten Fällungsbedingungen blieb der Überstand trüb, und das Pellet bestand aus einem klaren viskosen Gel und einem weißen pulverförmigen Feststoff. Die ungeeigneten Fällungsbedingungen korrelierten mit geringen Fällungserträgen. Zu den getesteten geeigneten Bedingungen gehörten 2 Vol. organisches Lösungsmittel (Ethanol, Propanol oder Aceton) mit 454 mM NaCl, 2 Vol. Ethanol mit 50 bis 750 mM NaCl oder KCl, 0,15 bis 3 Vol. Ethanol mit 100 mM NaCl oder KCl. Ungeeignete Bedingungen waren: 2 Vol. Ethanol mit 25 mM NaCl oder KCl und 2 Vol. organisches Lösungsmittel (Ethanol, Propanol oder Aceton) ohne Salz. Es wurde festgestellt, dass das PolyP unter geeigneten Fällungsbedingungen aus einer feinen Emulsion zu einem klaren viskosen Gel aggregiert wurde.

Im Standard-Fällungsprotokoll wurde die Zentrifugation zur Trennung des PolyP-Gels nach der Zugabe von NaCl und Ethanol verwendet. Bei größeren Mengen war es auch möglich, das Budit 4-Salz-Ethanol-Gemisch über Nacht in einem Scheidetrichter zu inkubieren. Während dieser Inkubation wurde der Überstand transparent, und das PolyP-Gel wurde vom Boden des Scheidetrichters abgelassen.

Die Entfernung von Wasser aus dem PolyP-Gel war der nächste Schritt. Die Trocknung erfolgte in einem Exsikkator, der mit getrocknetem Siliziumdioxid (ohne Vakuum) gefüllt wurde. Es war wichtig, das PolyP-Gel gleichmäßig auf den Trocknungsschalen zu verteilen, um die Bildung dicker Gelschichten zu vermeiden, die langsamer trockneten.

Tägliches Rühren und Zerkleinern der PolyP-Kruste mit einem Spatel half ebenfalls beim Trocknen.

Die Trocknungskinetik von Natrium- und Kalium-PolyP-Gel wurde analysiert (Abbildung 1D). Der anfängliche Wassergehalt der Natrium- und Kalium-PolyP-Gele betrug 49,0 bzw. 42,6 %. So erhöhte Kalium den anfänglichen Wassergehalt des Gels nicht, wie oben spekuliert wurde. Das Kalium-PolyP trocknete langsamer als das Natrium-PolyP-Gel. Es wurde festgestellt, dass - obwohl der anfängliche Wassergehalt geringer war - das Wasser stärker an das Kalium-PolyP-Gel gebunden war als an das Natrium-PolyP-Gel.

Nach 7 Tagen Trocknung wurde in den Natrium- bzw. Kalium-PolyP-Gelen ein Wassergehalt von 0,9 bzw. 1,6 % gemessen. Der Wassergehalt des unverarbeiteten Budit 4-Pulvers, der durch Trocknung bei 120°C bestimmt wurde, betrug 0,2 ± 0,0 % (Mittelwert ± Standardfehler des Mittelwertes, 5 Replikatmessungen). Obwohl dieser Referenzwert nicht erreicht wurde, wurde ein Wassergehalt von < 2 % für die Lagerung und Vermahlung als ausreichend angesehen. Die 7-tägige Trocknung in einem Exsikkator, der mit Siliziumdioxid ausgestattet war, wurde als Standard-Trocknungsprotokoll für weitere Experimente gewählt. Der einzige Nachteil dieser Methode ist ihre Langwierigkeit. Die Vorteile sind u.a. eine gute Wirtschaftlichkeit, hohe Skalierbarkeit und schonende Trocknung des Produkts.

Budit 4 wurde nach dem Trocknen als grobe weiße Kruste gewonnen. Um ein homogenes, feinkörniges Pulver zu erhalten, wurde PolyP mit einer Kugelmühle gemahlen. Drei Edelstahlperlen (Durchmesser: 3,2 mm) und ca. 1 g grobes Pulver wurden in ein 2 ml Edelstahlgefäß überführt. Es wurden verschiedene Vermahlungszeiten getestet (1, 2, 3, 4 und 5 Minuten). Feines weißes Pulver wurde nach 2 Minuten erhalten. Später wurde festgestellt, dass für Bio-PolyP maximal 0,5 g Pulver pro Probengefäß verwendet werden sollten. Die Vermahlung von ≤ 0,5 g PolyP mit 3 Perlen pro Probengefäß für 2 Minuten wurde als Standardmahlprotokoll definiert.

Die feinkörnigen Natrium- und Kalium-PolyP-Pulver lösen sich leicht in Wasser. Wie bei allen PolyPs war bei der Auflösung ein längeres starkes Rühren erforderlich, um die Bildung von Klumpen zu vermeiden. Beide Pulver wurden in einer Konzentration von 1 % (w/v) gelöst. Der pH-Wert der Natrium-PolyP-Lösung betrug 7,41 ± 0,04, und der pH-Wert der Kalium-PolyP-Lösung wurde bei 7,15 ± 0,03 (Mittelwert zwischen zwei unabhängigen Chargen ± Standardfehler des Mittelwerts) gemessen. Mit einem pH-Wert von 7,0 vor der Fällung blieb der pH-Wert während der Fällungs- und Trocknungsschritte nahezu unverändert.

Zusammenfassend lässt sich sagen, dass die optimalen Bedingungen für die Fällung (pH-Wert der PolyP-Lösung zu 7 mit NaOH oder KOH, Zugabe von 100 mM NaCl oder KCl, Zugabe von 1 Vol. Ethanol, Inkubation 1 Stunde, Rückgewinnung durch Zentrifugation), die Trocknung (Verteilung des PolyP-Gels auf der Schale, Trocknung 7 d im Exsikkator über Siliziumdioxid) und die Vermahlung (≤ 0,5 g PolyP und 3 Perlen pro 2 ml Röhrchen, Mühle für 2 min) von Budit 4 festgelegt wurden. Diese Prozessbedingungen wurden bei der Herstellung von Bio-PolyP verwendet, die im Folgenden beschrieben wird.

Zusammenfassend lässt sich sagen, dass die optimalen Bedingungen für die Fällung (pH-Wert der PolyP-Lösung zu 7 mit NaOH oder KOH, Zugabe von 100 mM NaCl oder KCl, Zugabe von 1 Vol. Ethanol, Inkubation 1 h, Rückgewinnung durch Zentrifugation), die Trocknung (Verteilung des PolyP-Gels auf der Schale, Trocknung 7 d im Exsikkator über Siliziumdioxid) und die Vermahlung (≤ 0,5 g PolyP und 3 Perlen pro 2 ml Röhrchen, Mühle für 2 min) von Budit 4 festgelegt wurden. Diese Prozessbedingungen wurden bei der Herstellung von Bio-PolyP verwendet, die im Folgenden beschrieben wird.

**Freisetzung von PolyP aus polyP-reichen *S. cerevisiae* Zellen**

Das Ziel der folgenden beiden Kapitel war die Entwicklung der präparativen Extraktion von PolyP aus polyP-reichem *S. cerevisiae.* Die polyP-reiche Zellmasse wurde durch $P_i$-Verhungern (Orthophosphat-Verhungern) und anschließende $P_i$-Fütterung von *S. cerevisiae* wie kürzlich beschrieben hergestellt [Ref. 12: J. J. Christ, L. M. Blank, FEMS yeast research 2019, 19]. Der PolyP-Gehalt in dieser Zellmasse betrug 26,5 ± 0,8 % PolyP (als $KPO_3$) in Zelltrockengewicht mit einer durchschnittlichen PolyP-Kettenlänge von 24 ± 1 P-Untereinheiten (Mittelwert ± Standardfehler des Mittelwertes aus drei analytischen Extraktionen). Dieser PolyP-Gehalt wurde als Referenz für die Berechnung der Extraktionseffizienz für die nachfolgend vorgestellten Experimente verwendet.

Das Startprotokoll für die präparative Extraktion beinhaltete eine Wärmebehandlung zur Freisetzung des PolyP aus den Zellen, pH-Neutralisation und NaCl-Ethanolfällung (weitere experimentelle Details in der Beschreibung von und in der Abbildung 2). Die Parameter der Wärmebehandlung (1 Stunde, 70°C) wurden von Kuroda et al. übernommen, die diese Parameter zur Freisetzung von PolyP aus Klärschlamm verwendeten [Ref. 16: A. Kuroda, N. Takiguchi, T. Gotanda, K. Nomura, J. Kato, T. Ikeda, H. Ohtake, Biotechnol. Bioeng. 2002, 78, 333-338].

Zwei abhängige Variablen wurden während der Optimierungsexperimente analysiert. Zunächst wurde die Menge des gewonnenen PolyP im Vergleich zur analytischen Extraktion (Extraktionseffizienz) gemessen. Eine Extraktionseffizienz von 100 % hätte gezeigt, dass das gesamte PolyP aus der Zelle gewonnen wurde. Zweitens wurde die durchschnittliche PolyP-Kettenlänge des gewonnenen PolyPs bestimmt. Ein guter Kompromiss zwischen einer hohen Extraktionseffizienz und einer langen PolyP-Kettenlänge war erwünscht.

Der erste Schritt der Optimierung bestand darin, das optimale Wasservolumen für die Suspension der Zellen zu bestimmen (Abbildung 2A). Interessanterweise fiel das Bio-PolyP nicht als Gel, sondern als Feststoff aus. Die Menge an extrahiertem PolyP und die Kettenlänge waren konstant bei 3,5 bis 8 ml Wasser pro g Nasszellmasse. Für weitere Experimente wurden fünf ml Wasser pro g Nasszellmasse gewählt, um den schwankenden Wassergehalt verschiedener Zellchargen zu berücksichtigen.

Anschließend wurde die Inkubationszeit während der Wärmebehandlung analysiert (Abbildung 2B). Eine Inkubationszeit von 10 Minuten führte zur höchsten Extraktionseffizienz und wurde daher für das optimierte Protokoll gewählt. Die Kettenlänge verringerte sich aufgrund der hitzekatalysierten Hydrolyse des Polymers deutlich um 1 P-Untereinheit pro 10 Minuten (multipler Korrelationskoeffizient $r = 0,983$, $p < 0,001$). Es wurde keine kürzere Inkubationszeit getestet, da ein schnelles Erwärmen und Abkühlen der Zellsuspension in einem Prozesshochskalieren eine Herausforderung darstellen würde.

Die Temperatur während der Wärmebehandlung wurde zwischen 40 und 90°C variiert (Abbildung 2C). Es wurde ein umgekehrter Zusammenhang zwischen dem Extraktionseffizienz und der Kettenlänge festgestellt. Offensichtlich, wenn suboptimale Mengen an PolyP extrahiert wurden, wurden in erster Linie längere Ketten aus der Zelle befreit oder kürzere Ketten selektiv abgebaut. Da die höchste Extraktionseffizienz bei einer Inkubationstemperatur von 70°C festgestellt wurde, wurde diese Temperatur für das optimierte Protokoll gewählt. Interessanterweise wurden 60 % des PolyP bereits bei 40°C extrahiert. Es wurde spekuliert, dass das für die Zelllagerung notwendige Einfrieren und Auftauen der Zellen einen Teil der Zellen lysiert hat. Optimal sollte der Lagerungsschritt bei - 20°C daher nicht ausgelassen werden.

Der pH-Wert nach der Wärmebehandlung war sauer. Verdünntes NaOH wurde als Extraktionsmittel anstelle von reinem Wasser getestet, um den pH-Wert während der Wärmebehandlung sofort zu neutralisieren. Die Extrakte von 1, 5 und 10 mM NaOH waren nach der Wärmebehandlung noch sauer. Im Gegensatz dazu waren die Extrakte von 50 und 100 mM NaOH alkalisch. Die Zugabe von HCl zur Neutralisierung der letzten beiden Extrakte führte zu einer unerwünschten Bildung von etwas Präzipitat. Nach der Ethanolfällung waren die PolyP-Pellets der 50 und 100 mM NaOH-Extrakte kaum wasserlöslich. Dies war wahrscheinlich auf agglomeriertes Protein zurückzuführen, das ausgefällt wurde, als HCl hinzugefügt wurde, um den pH-Wert vor der Ethanolfällung zu neutralisieren. Abschließend wurden 50 und 100 mM NaOH aufgrund dieser negativen Ergebnisse bereits für weitere Experimente ausgeschlossen. Dennoch wurden der Extraktionseffizienz und die Kettenlänge analysiert (Abbildung 2D). Ein mM NaOH zeigte die gleiche Leistung wie reines Wasser. 5 mM und 10 mM NaOH verringerten die Extraktionseffizienz und die Länge der PolyP-Kette. 50 mM und 100 mM NaOH erhöhten die Extraktionseffizienz, verringerten aber die Kettenlänge erheblich. Als Extraktionsmittel während der Wärmebehandlung wurde weiterhin reines Wasser verwendet.

In Kombination mit Hitze wird verdünntes NaCl häufig verwendet, um RNA aus Hefezellen freizusetzen [Ref. 17: A. Kuninaka, M. Fujimoto, K. Uchida, H. Yoshino, Agric. Biol. Chem. 1980, 44, 1821-1827]. Da sich PolyP und RNA chemisch etwas ähnlich verhalten, wurde auch hier dieses Extraktionsmittel getestet (Abbildung 2E). Alle getesteten Konzentrationen (1 bis 200 mM NaCl) reduzierten sowohl den Extraktionseffizienz als auch die Kettenlänge. So wurde für alle weiteren Experimente reines Wasser verwendet. Ein Vorteil von reinem Wasser war, dass die Wärmebehandlung für die Herstellung von Natrium- und Kalium-Bio-PolyP gleich war.

Nach der Wärmebehandlung wurde das PolyP durch Fällung mit Ethanol und NaCl gewonnen. Leider fallen unter diesen Bedingungen auch Proteine und Nukleinsäure aus. Um Protein und Nukleinsäure zu entfernen, wurde vor der Ethanolfällung eine HCl-Präzipitation in das Protokoll eingefügt. Durch den niedrigen $pK_a$-Wert der Hydroxylgruppen verliert PolyP auch bei sehr niedrigem pH-Wert seine negative Ladung nicht. Im Gegensatz dazu präzipitieren Protein und Nukleinsäure bei einem niedrigen pH-Wert. Verschiedene Konzentrationen von HCl wurden getestet (Abbildung 2F). Das Verhältnis von Protein und Nukleinsäure zu PolyP im PolyP-Pellet nach der Ethanolfällung wurde bestimmt (Diamanten in Abbildung 2F). Dieses Verhältnis könnte von 4,1 auf 6,2 (w/w) erhöht werden, wenn 50 mM HCl (Endkonzentration) verwendet würden. Da Protein und Nukleinsäuren über einen Summenparameter (Absorption bei 280 nm) gemessen wurden, wurde keine Differenzierung zwischen beiden gemacht. Der Extraktionseffizienz und die Kettenlänge nahmen nur geringfügig um 2,2 Prozentpunkte bzw. 0,6 P-Untereinheiten ab. Eine HCl-Präzipitation mit einer Endkonzentration von 50 mM HCl wurde für alle weiteren Experimente verwendet.

Verschiedene Konzentrationen von Ethanol wurden für die fraktionierte PolyP-Präzipitation getestet (Abbildung 2G und H). Nachdem die erste PolyP-Fraktion gewonnen wurde, wurde das restliche PolyP durch Zugabe von finalem 1 Vol. Ethanol gewonnen. Geeignete Ethanolkonzentrationen für die Trennung von langkettigem PolyP waren 0,15 bis 0,25 Vol. Eine erste Fällung mit 0,15 Vol. und dann mit 1 Vol. Ethanol wurde für das Standardprotokoll gewählt. Die Ethanolmengen wurden um die Konzentration des verfügbaren Stoffes (96 % Ethanol) korrigiert. Um beispielsweise 0,150 Vol. Ethanol zu erhalten, wurden 0,156 Vol. zugegeben. Mit der fraktionierten Fällung wurden 26,9 % des gesamten PolyP in der ersten Fraktion gewonnen. Dieses PolyP maß 41 P-Untereinheiten. Die Fraktion 2 enthielt weitere 52,5 % des gesamten PolyP und hatte eine Kettenlänge von 18 P-Untereinheiten. Insgesamt wurden 80 % des PolyP zurückgewonnen, was der Rückgewinnung entspricht, die mit nur einem Fällungsschritt mit 1 Vol. Ethanol erreicht wurde. Die Entwicklung der präparativen PolyP-Extraktion war nun abgeschlossen.

**Physikalisch-chemische Charakterisierung der Bio-PolyPs**

Die Produktion von Bio-PolyP wurde um den Faktor 200 (von 5 mg auf 1 g PolyP) erhöht, um eine gründliche physikalisch-chemische Charakterisierung des Bio-PolyP zu ermöglichen. Im letzten Kapitel wurde das Bio-PolyP aus den Zellen freigesetzt und mit Ethanol ausgefällt. Die Analyse wurde mit dem Pellet durchgeführt, das nach der Ethanolfällung erhalten wurde. In diesem Kapitel wurde das Bio-PolyP nach der Ethanolfällung in einem Exsikkator getrocknet und anschließend mit einer Kugelmühle gemahlen. Darüber hinaus wurde ein Kalium-Bio-PolyP sowie ein Natrium-Bio-PolyP hergestellt. Die Ergebnisse der physikalisch-chemischen Charakterisierung der Bio-PolyPs im Vergleich zu drei kommerziellen PolyPs sind in Tabelle 1 dargestellt. Es wurden die handelsüblichen PolyPs mit der längsten PolyP-Kettenlänge gewählt, die in größeren Mengen (ca. 50 g) erhältlich waren.

**Tabelle 1. Vergleich die physikalisch-chemischen Eigenschaften von Bio-PolyP aus der vorliegenden Erfindung mit den physikalisch-chemischen Eigenschaften von chemisch hergestelltem PolyP.** Die PolyPs vorliegender Erfindung stammen aus einer Charge polyP-reicher Zellen. Für diese PolyPs werden die Mittelwerte ± der Standardfehler des Mittelwertes aus zwei präparativen Extraktionen, die unabhängig voneinander an zwei verschiedenen Tagen durchgeführt wurden, angezeigt. Abkürzungen: GdQ, Grenze der Quantifizierung; n. a., nicht anwendbar; n. n., nicht nachweisbar. [a] Bezieht sich auf PolyP, das in einem Exsikkator getrocknet wurde. [b] Der Wert wurde definiert als (mol polyP gewonnen nach der präparativen Extraktion) * (mol polyP in der polyP-reichen Zellmasse, die für die präparative Extraktion verwendet wurde)$^{-1}$.

| Herkunft PolyP | In Rahmen der vorliegenden Erfindung | | | | Handelsüblich | | |
|---|---|---|---|---|---|---|---|
| PolyP-Name | Na-PolyP | | K-PolyP | | Budit 4 | Roth | P100 |
| | lange Kette | kurze Kette | lange Kette | kurze Kette | | | |
| Erscheinungsbild | feinkörniges weißes Pulver | | | | | | große weiße Flocken |
| Durchschnittliche Molekularformel des Polymers | $Na_{9,9}$<br>$K_{12,4}$<br>$Mg_{10,5}$<br>$HP_{42,3}$<br>$O_{127,8}$ | $Na_{3,7}$<br>$K_{3,6}$<br>$Mg_{2,5}$<br>$HP_{11,3}$<br>$O_{35,0}$ | $K_{15,3}$<br>$Mg_{9,1}$<br>$HP_{32,6}$<br>$O_{93,7}$ | $K_{7,8}$<br>$Mg_{2,7}$<br>$HP_{12,3}$<br>$O_{37,9}$ | $Na_{19,2}$<br>$H_2P_{19,2}$<br>$O_{58,5}$ | $Na_{20,1}$<br>$H_2P_{20,1}$<br>$O_{61,3}$ | $Na_{42,0}$<br>$H_2P_{42,0}$<br>$O_{127}$ |
| Durchschnittliches Molekulargewicht des | 4320 | 1197 | 3410 | 1362 | 1972 | 2066 | 4299 |
| Polymers [g * mol$^{-1}$] | ± 211 | ± 21 | ± 52 | ± 19 | | | |
| Molverhältnis Na + K zu Mg im Polymer | 2,1 ± 0,1 | 2,9 ± 0,1 | 1,7 ± 0,0 | 2,9 ± 0,1 | n. a. | n. a. | n. a. |
| Kettenlängenverteilung [P-Untereinheiten] | <15 - 314 | <15 - 130 | <15 - 314 | <15 - 130 | <15 - 207 | <15 - 207 | <15 - 700 |
| pH-Wert einer 1 %igen (w/v) PolyP-Lösung | 6,6 ± 0,0 | 6,8 ± 0,0 | 6,4 ± 0.0 | 6,7 ± 0.0 | 3,6 | 5,4 | 5,3 |
| Durchschnittliche Kettenlänge [P-Untereinheiten]: | | | | | | | |
| nach Enzym-Assay | 42,3 ± 2,2 | 11,3 ± 0,2 | 32,6 ± 0,6 | 12,3 ± 0,2 | 19,2 | 20,1 | 42,0 |
| nach PAGE | 122 ± 2 | 76 ± 0 | 110 ± 1 | 72 ± 1 | 90 | 85 | 152 |
| PolyP-Reinheit [% (w/w)] [a]: | | | | | | | |
| Lineares PolyP inkl. Gegenionen in Exsikkator-Trockenmasse | 85,0 ± 1,3 | 79,3 ± 0,1 | 81,8 ± 1,2 | 78,4 ± 0,2 | 89,0 | 87,5 | 85,5 |
| Lineares PolyP inkl. Gegenionen und Kristallwasser in Exsikkator-Trockenmasse | 91,7 ± 1,6 | 85,0 ± 0,2 | 88,0 ± 1,2 | 83,0 ± 0,3 | 89,2 | 87,5 | 85,8 |

(fortgesetzt)

| Verunreinigungen im PolyP [% (w/w) [a]: | | | | | | | |
|---|---|---|---|---|---|---|---|
| Wasser (GdQ: < 0,1 %) | 6,7 ± 0,3 | 5,7 ± 0,2 | 6,2 ± 0,0 | 4,6 ± 0,1 | 0,2 | n. n. | 0,3 |
| Unlösliche Stoffe (GdQ: < 0,01 %) | n. n. | n. n. | 6,2 ± 0,5 | n. n. | n. n. | n. n. | n. n. |
| Chlorid (als $Cl^-$, GdQ: < 0,75 %) | n. n. | n. n. | n. n. | n. n. | n. n. | n. n. | n. n. |
| Zyklisches PolyP (als $MPO_3$) | 0,8 ± 0,1 | 2,0 ± 0,2 | 0,7 ± 0,0 | 1,5 ± 0,0 | 7,8 | 10,3 | 5,2 |
| $P_i$ (als $PO_4^{3-}$, GdQ: < 0,01 %) | n. n. | 1,8 ± 0,2 | n. n. | 0,6 ± 0,1 | n. n. | n. n. | 0,7 |
| Nukleinsäure (als RNS, GdQ: < 0.05 %) | 0,2 ± 0,0 | 1,7 ± 0,1 | 0,1 ± 0,0 | 1,6 ± 0,0 | n. n. | n. n. | n. n. |
| Arsen, Cadmium, Kalzium, Chrom, Kupfer, Eisen, Blei, Nickel, Vanadium (GdQ: < 0,25 %) | n. n. | n. n. | n. n. | n. n. | n. n. | n. n. | n. n. |
| Ausbeute präparative Extraktion [% (mol/mol)] [b]: | | | | | | | |
| individuell für beide PolyP-Fraktionen | 15,9 ± 1,8 | 64,7 ± 2,4 | 21,3 ± 0,8 | 54,5 ± 0,6 | n. a. | n. a. | n. a. |
| Summe beider PolyP-Fraktionen | 80,6 ± 0,6 | | 75,8 ± 0,2 | | | | |

Alle PolyPs erschienen als feinkörniges weißes Pulver, mit Ausnahme des PolyP P100, das als große weiße Flocken geliefert wurde. Die flockige Form kann zu einer Heterogenität zwischen PolyP-Zubereitungen führen, wenn nur wenige Flocken pro Zubereitung gelöst werden. Das physische Erscheinungsbild des Bio-PolyPs war wie gewünscht. Die Pulverform ist vorteilhaft in Bezug auf Versandkosten, Lagerstabilität und Handling.

Die drei handelsüblichen PolyPs enthielten wie erwartet nur Natrium als Gegenion. Die Natrium-Bio-PolyPs enthielten etwa die gleichen molaren Anteile an Natrium, Kalium und Magnesium wie die Kationen. Das bedeutet, dass die Zelle hauptsächlich Kalium und Magnesium als anorganische Kationen enthielt. Das Kalium-Bio-PolyP enthielt Kalium und Magnesium in einem Molverhältnis von 1,7:1 und 2,9:1 (K:Mg) für die lang- bzw. kurzkettigen Varianten. Das Molverhältnis von Natrium plus Kalium zu Magnesium (Na+K:Mg) im Polymer ist in Tabelle 1 dargestellt. Dieser Wert war ein guter Indikator für die Geschwindigkeit der Auflösung, da PolyP mit nur Magnesium als Gegenion nicht wasserlöslich ist.

Die PolyP-Reinheit wird an zwei Stellen in Tabelle 1 angegeben: als lineares PolyP in Exsikkator-Trockenmasse und als Summe aus linearem PolyP und Kristallwasser in Exsikkator-Trockenmasse. Die chemische Synthese von PolyP beinhaltet das Erwärmen des Monomers bei mehreren hundert Grad für mehrere Stunden. Kein Wunder, dass die chemisch hergestellten PolyPs nur ≤ 0,3 % Wasser enthielten. Die Bio-PolyPs enthielten 4,6 bis 6,7 % Kristallwasser. Vergleicht man den Wassergehalt der lang- und kurzkettigen Varianten, so zeigt sich, dass der Wassergehalt für das Natrium um 1,0 Prozentpunkte und für das Kalium-PolyP um 1,6 Prozentpunkte höher war. Diese Ergebnisse bestätigen sowohl die Gesamthydrophilie von PolyP als auch, dass die Hydrophilie mit zunehmender Kettenlänge zunimmt. Der Wassergehalt stellt bei der Anwendung des Bio-PolyP kein Problem dar. Da Kristallwasser nicht als Verunreinigung im PolyP betrachtet wurde, wurde die PolyP-Reinheit auch einschließlich des Kristallwassers berichtet. Die Reinheit (inkl. Kristallwasser) der chemisch hergestellten PolyPs betrug zwischen 85,8 und 89,2 %. Die Reinheiten des Bio-PolyP (inkl. Kristallwasser) lagen zwischen 83,0 und 91,7 %. Die Bio-PolyP-Reinheit war - wie gewünscht - vergleichbar mit chemisch hergestelltem PolyP. Bei langkettigem Natrium-Bio-PolyP war die Reinheit sogar 2,5 Prozentpunkte höher als beim reinsten chemisch hergestellten PolyP.

Die durchschnittliche PolyP-Kettenlänge von Budit 4 und dem PolyP von Roth betrug ca. 20 P-Untereinheiten. Das PolyP P100 maß 42 P-Untereinheiten, was die derzeit längste verfügbare Kettenlänge ist, wenn man sich die massenchemische Synthese von festem, wasserlöslichem PolyP ansieht. Die durchschnittliche PolyP-Kettenlänge des langkettigen Natrium-Bio-PolyP war gleich der von P100. Im Vergleich dazu war das langkettige Kalium-Bio-PolyP kürzer (32 P-Untereinheiten). Die Kettenlängen der kurzkettigen Natrium- und Kalium-Bio-PolyPs waren etwa 12 P-Untereinheiten, was der Kettenlänge von Budit 7 (einem weiteren handelsüblichen) Natrium-PolyP der Firma Chemische Fabrik Budenheim entsprach. Bio-PolyP kann mit einer Vielzahl von PolyP-Kettenlängen hergestellt werden, was viele Anwendungen ermöglichen soll.

Die PolyP-Kettenlängenverteilung wurde mit PAGE analysiert (Abbildung 3). Die Bio-PolyP-Chargen wurden nebeneinander auf das Gel aufgetragen und zeigten die gleiche Kettenlängenverteilung, was - zusammen mit dem geringen Standardfehler der Mittel in den anderen Parametern - eine gute Reproduzierbarkeit der präparativen PolyP-Extraktion zeigte. Die untere Grenze der Kettenlängenverteilung konnte mit PAGE nicht bestimmt werden, da alle Proben einige PolyP mit einer Kettenlänge von ca. < 15 P-Untereinheiten enthielten, die nicht mit Toluidinblau O gefärbt werden können. Infolgedessen erschien die durchschnittliche PolyP-Kettenlänge mit PAGE länger als die Ergebnisse des Enzym-Assays, der alle Kettenlängen umfasste. Die Obergrenze der Kettenlängenverteilung wurde mit PAGE exakt bestimmt. Obwohl das langkettige Natrium-PolyP und das PolyP P100 die gleiche durchschnittliche PolyP-Kettenlänge (42 P-Untereinheiten) hatten, enthielt P100 eine heterogenere Mischung von Kettenlängen (längste Kettenlängen 314 bzw. 700 P-Untereinheiten). Die subtilen Unterschiede in der durchschnittlichen PolyP-Kettenlänge zwischen den Varianten Natrium und Kalium-Bio-PolyP waren in der PAGE-Analyse nicht sichtbar. Budit 4 und das PolyP von Roth zeigten die gleiche Kettenlängenverteilung (bis zu 207 P-Untereinheiten). Die kurzkettigen Bio-PolyPs besaßen die engste Kettenlängenverteilung (bis zu 130 P-Untereinheiten).

Der pH-Wert der chemisch hergestellten PolyPs lag im Bereich von 3,6 bis 5,4, was typisch für chemisch hergestelltes langkettiges PolyP ist. Der pH-Wert der Bio-PolyPs war neutral. Dies bedeutete, dass das PolyP den pH-Wert, der vor der Ethanolfällung eingestellt wurde, während der gesamten Ethanolfällung, Trocknung, Mahlung und Auflösung beibehält. Der neutrale pH-Wert ist von großem Interesse für Prozesse, bei denen ein langkettiges PolyP mit einem neutralen pH-Wert erforderlich ist, da die chemische Synthese nur saures langkettiges PolyP liefern kann.

Die chemisch hergestellten PolyPs waren vollständig wasserlöslich und wurden innerhalb einer Minute in Wasser gelöst. Die Bio-PolyPs waren ebenfalls vollständig wasserlöslich, mit Ausnahme des lang-kettiges Kalium- Bio-PolyPs, das 6,2 % unlösliche Stoffe enthielt. Der hohe Magnesiumgehalt war wahrscheinlich der Grund für das Vorhandensein von unlöslichem Magnesium-polyP im langkettigen Bio-PolyP. Alle Bio-PolyPs benötigten 1-2 Stunden, um sich in Wasser aufzulösen, da sie etwas Magnesium enthielten. Diese langsame Löslichkeit könnte ein Problem für Anwendungen sein, bei denen sich das PolyP schnell auflösen muss.

Von den chemisch hergestellten PolyPs enthielt nur das P100 wenig $P_i$ (0,7 %). Auch die langkettigen Bio-PolyPs enthielten kein $P_i$. Die kurzkettigen Natrium- und Kalium-PolyPs enthielten 1,8 bzw. 0,6 % $P_i$. Das bedeutet, dass der größte Teil des $P_i$, der natürlich in der Zelle vorhanden war, während der Ethanolfällung nicht ausfiel.

Der Nukleinsäuregehalt der Bio-PolyPs lag zwischen 0,1 und 1,7 %. Dieser Parameter wurde spektrophotometrisch bestimmt und ist daher nicht spezifisch. Eine niedrige Konzentration an Nukleinsäure wurde sowohl durch einen niedrigen Gehalt an Nukleinsäure in den Zellen als auch durch die Entfernung von Nukleinsäure im HCl-Ausfällungsschritt erreicht. NaCl oder KCl wurden zur Unterstützung der Ethanolfällung verwendet. Beides fiel nicht aus, da in den Bio-PolyPs kein Chlorid nachgewiesen wurde. Arsen, Cadmium, Kalzium, Chrom, Kupfer, Eisen, Blei, Nickel und Vanadium wurden weder in den Bio-PolyPs noch in den chemisch hergestellten PolyPs nachgewiesen, was für ihre Anwendung als Lebensmittelzusatzstoffe unerlässlich ist.

Bei gleicher Konzentration zog Kalium bei der ersten Ethanolfällung mehr PolyP im Vergleich zu Natrium herunter. Dieser Befund wurde durch die Erträge der präparativen Extraktion bestätigt, die bei 15,9 % für lang-kettiges Natrium- Bio-PolyP und 21,3 % für lang-kettiges Kalium- Bio-PolyP lagen. Die Gesamtausbeute der präparativen Extraktion des Natrium-Bio-PolyP war hoch (80,6 %). Die Ausbeute der präparativen Extraktion des Kalium-PolyP war etwas geringer (75 %). Beide Erträge waren zufriedenstellend.

Für die biotechnologische Herstellung von Natrium-PolyP wurden 125,3 ml Wasser, 131,6 ml Ethanol, 0,21 g HCl, 0,28 g NaOH, 0,35 g NaCl und 5,4 g polyP-reiche Hefezelle Trockengewicht pro g gewonnenem PolyP benötigt. Zu den Nebenprodukten gehörten 129,6 ml Wasser, 131,6 ml Ethanol, 0,75 g NaCl und 4,37 g Trockenhefezellablagerungen pro g gewonnenes PolyP. Der Anstieg des Wasservolumens ist auf Zellwasser zurückzuführen. Für die biotechnologische Herstellung von Kalium-PolyP wurden 120,8 ml Wasser, 128,3 ml Ethanol, 0,20 g HCl, 0,43 g KOH, 0,42 g KCl und 5,2 g polyP-reiche Hefezelle Trockengewicht pro g gewonnenem PolyP benötigt. Zu den Nebenprodukten gehörten 125,0 Wasser, 128,3 ml Ethanol, 0,99 g KCl und 4,18 g Trockenhefezellablagerungen pro g gewonnenes PolyP.

## Schlussfolgerung

Ein hochskalierbarer Prozess zur Reinigung von PolyP aus polyP-reichem S. *cerevisiae* wurde entwickelt. In Kombination mit der Herstellung von polyP-reichem *S. cerevisiae* ermöglicht das Verfahren die biotechnologische Herstellung des neuartigen Produkts "wasserlösliches lebensmitteltaugliches Bio-PolyP". Das hier entwickelte Verfahren öffnet die Tür für das biotechnologische $P_i$-Recycling aus ungenutzten $P_i$-Abfallströmen zu einem hochwertigen organischen ("Bio") Produkt.

## Referenzen

**Ref.** [1] aF. M. Harold, Bacteriol. Rev. 1966, 30, 772-794; bI. S. Kulaev, V. M. Vagabov, T. V. Kulakovskaya, The biochemistry of inorganic polyphosphates, Second ed., John Wiley & Sons, Ltd, West Sussex (England), 2005**;** cN. N. Rao, M. R. Gomez-Garcia, A. Kornberg, Annu. Rev. Biochem. 2009, 78, 605-647.
**Ref.** [2] J. R. Van Wazer, K. A. Holst, J. Am. Chem. Soc. 1950, 72, 639-644.
**Ref.** [3] J. R. Van Wazer, Phosphorus and its compounds. Volume I: Chemistry, Interscience publishers, Inc., New York (USA), 1958**.**
**Ref.** [4] aE. Thilo, W. Wieker, Zeitschrift Fur Anorganische Und Allgemeine Chemie 1957, 291, 164-185; bE. Thilo, Adv. Inorg. Chem. 1962, 4, 1-75.
**Ref.** [5] aM. Dürr, K. Urech, T. Boller, A. Wiemken, J. Schwencke, M. Nagy, Arch. Microbiol. 1979, 121, 169-175; bL. Jacobson, M. Halmann, J. Yariv, Biochem. J. 1982, 201, 473-479; cG. M. Roomans, Physiol. Plant. 1980, 48, 47-50.
**Ref.** [6] T. V. Kulakovskaya, V. M. Vagabov, I. S. Kulaev, Process Biochemistry 2012, 47, 1-10.
**Ref.** [7] Q. W. Shen, D. R. Swartz, Meat science 2010, 84, 364-364.
**Ref.** [8] J. E. Clark, H. G. Wood, Anal. Biochem. 1987, 161, 280-290.
**Ref.** [9] S. A. Smith, C. J. Baker, J. M. Gajsiewicz, J. H. Morrissey, Blood 2017, 130, 88-91.
**Ref.** [10] L. H. Cragg, H. Hammerschlag, Chem. Rev. 1946, 39, 79-135.
**Ref.** [11] aJ. J. Christ, L. M. Blank, Anal. Biochem. 2018, 548, 82-90; bT. Shiba, in Inorganic polyphosphates in eukaryotic cells (Eds.: T. Kulakovskaya, E. Pavlov, E. N. Dedkova), Springer International Publishing, Basel (Switzerland), 2016, pp. 139-158.
**Ref.** [12] J. J. Christ, L. M. Blank, FEMS yeast research 2019, 19.
**Ref.** [13] J. J. Christ, L. M. Blank, Anal. Biochem. 2018, 563, 71-78.
**Ref.** [14] J. J. Christ, S. Willbold, L. M. Blank, Anal. Chem. 2019, 91, 7654-7661.
**Ref.** [15] S. A. Smith, Y. Wang, J. H. Morrissey, Electrophoresis 2018, 39, 2454-2459.
**Ref.** [16] A. Kuroda, N. Takiguchi, T. Gotanda, K. Nomura, J. Kato, T. Ikeda, H. Ohtake, Biotechnol. Bioeng. 2002, 78, 333-338.
**Ref.** [17] A. Kuninaka, M. Fujimoto, K. Uchida, H. Yoshino, Agric. Biol. Chem. 1980, 44, 1821-1827.
**Ref.** [18] aN. Takiguchi, A. Kuroda, H. Ohtake, S. Tsuneda, in Phosphorus Recovery and Recycling, First ed. (Eds.: H. Ohtake, S. Tsuneda), Springer Singapore, Singapore, 2019, pp. 515-526; bR. Hirota, A. Kuroda, J. Kato, H. Ohtake, J. Biosci. Bioeng. 2010, 109, 423-432.
**Ref.** [19] S. Omelon, W. Habraken, in Inorganic polyphosphates in eukaryotic cells (Eds.: T. Kulakovskaya, E. Pavlov, E. N. Dedkova), Springer International Publishing, Basel (Switzerland), 2016, pp. 177-205.
**Ref.** [20] aE. C. De Oliveira Lima, G. B. Alcantara, F. C. Damasceno, J. M. M. Neto, F. Galambeck, Quim. Nova. 2010, 33, 1991-1995; bJ. R. Van Wazer, J. Am. Chem. Soc. 1950, 72, 647-655.
**Ref.** [21] aL. K. Seidlmayer, M. R. Gomez-Garcia, T. Shiba, G. A. Porter, Jr., E. V. Pavlov, D. M. Bers, E. N. Dedkova, Arch. Biochem. Biophys. 2019, 662, 177-189; bT. Shiba, Y. Takahashi, T. Uematsu, Y. Kawazoe, K. Ooi, K. Nasu, H. Itoh, H. Tanaka, M. Yamaoka, M. Shindoh, T. Kohgo, Key Engineering Materials 2004, 254-256, 1119-1122.
**Ref.** [22] N. Gilbert, Nature 2009, 461, 716-718.
**Ref.** [23] aL. Carraresi, S. Berg, S. Bröring, Journal of Cleaner Production 2018, 183, 87-101; bK. R. Herrmann, A. J. Ruff, B. Infanzon, U. Schwaneberg, Appl. Microbiol. Biotechnol. 2019, 103, 6435-6448.

## Patentansprüche

**1.** Verfahren zur Gewinnung von Polyphosphat aus polyphosphat-haltigen Hefezellen, umfassend

(1) Aufschließen von polyphosphathaltigen Hefezellen nach deren Ernte durch Einfrieren und Auftauen;
(2) Inkubieren einer wässrigen Lösung, enthaltend die aufgeschlossenen polyphosphat-haltigen Hefezellen bei einer Temperatur von ca. 70°C bis ca. 80°C für ca. 10 Minuten, wobei circa bedeutet innerhalb 20%;
(3) Entfernen von Zelldebris aus der wässrigen Lösung;
(4) Entfernen von Nukleinsäuren und Proteinen aus der wässrigen Lösung durch Zugabe einer Säure;
(5) Neutralisieren der verbleibenden wässrigen Lösung; und
(6) Gewinnen von Polyphosphat aus der wässrigen Lösung durch Fällung mittels eines Alkohols.

**2.** Verfahren nach Anspruch 1, wobei im Schritt (6) die Fällung mittels Alkohol eine fraktionierte Fällung ist.

**3.** Verfahren nach Anspruch 2, wobei die fraktionierte Fällung durch aufeinander folgende Fällungen mit ansteigendem Volumen des Alkohols im Bezug auf das Gesamtvolumen der wässrigen Lösung erfolgt.

**4.** Verfahren nach Anspruch 3, wobei nach dem Gewinnen von langkettigem Polyphosphat mit einer durchschnittlichen Kettenlänge von 32-44 P-Einheiten ein zweites Volumen des Alkohols zugegeben wird, wobei das zweite Volumen des Alkohols größer ist als das erste Volumen des Alkohols.

**5.** Verfahren nach einem der Ansprüche 1 bis 4, wobei im Schritt (5) eine Base verwendet wird, die ein einwertiges Kation als Gegenion enthält.

**6.** Verfahren nach einem der Ansprüche 1 bis 5, wobei im Schritt (5) eine Base verwendet wird, die

(a) Natrium, oder
(b) Kalium

als Gegenion enthält.

**7.** Zusammensetzung, enthaltend getrocknetes Polyphosphat, wobei das getrocknete Polyphosphat

(a) einen Reinheitsgrad von zumindest 80% aufweist, definiert als Gehalt von linearem Polyphosphat und Kristallwasser in der Trockensubstanz, wobei das Polyphosphat Natriumionen und/oder Kaliumionen und Magnesiumionen als Gegenionen enthält,
(b) einen Reinheitsgrad von linearem Polyphosphat von zumindest 80% aufweist, wobei das Polyphosphat Natriumionen und/oder Kaliumionen und Magnesiumionen als Gegenionen enthält,
(c) zumindest 20% (mol/mol) Natriumionen und/oder zumindest 20% (mol/mol) Kaliumionen und bis zu 50% (mol/mol) Magnesiumionen als Gegenionen enthält,
(d) eine durchschnittliche Kettenlänge von 10-125 P-Einheiten, vorzugsweise 11-44 P-Einheiten, aufweist, wobei die durchschnittliche Kettenlänge enzymatisch bestimmt wird, und
(e) einen Anteil an zyklischem Polyphosphat von nicht mehr als 5%, vorzugsweise nicht mehr als 2%, bezogen auf die Trockensubstanz aufweist.

**8.** Zusammensetzung nach Anspruch 7, wobei das getrocknete Polyphosphat

(a) einen Reinheitsgrad von zumindest 85% aufweist, definiert als Gehalt von linearem Polyphosphat und Kristallwasser in der Trockensubstanz, wobei das Polyphosphat Natriumionen, Kaliumionen und Magnesiumionen als Gegenionen enthält,
(b) einen Reinheitsgrad von linearem Polyphosphat von zumindest 85% aufweist, wobei das Polyphosphat Natriumionen und/oder Kaliumionen und Magnesiumionen als Gegenionen enthält,
(c) zumindest 20% (mol/mol) Natriumionen, zumindest 20% (mol/mol) Kaliumionen und bis zu 50% (mol/mol) Magnesiumionen als Gegenionen enthält,
(d) eine durchschnittliche Kettenlänge von 10-125 P-Einheiten, vorzugsweise 11-44 P-Einheiten, aufweist, wobei die durchschnittliche Kettenlänge enzymatisch bestimmt wird, und
(e) einen Anteil an zyklischem Polyphosphat von nicht mehr als 5%, vorzugsweise nicht mehr als 2%, bezogen auf die Trockensubstanz aufweist.

**9.** Zusammensetzung nach Anspruch 7, wobei das getrocknete Polyphosphat

(a) einen Reinheitsgrad von zumindest 83% aufweist, definiert als Gehalt von linearem Polyphosphat und Kristallwasser in der Trockensubstanz, wobei das Polyphosphat Kaliumionen und Magnesiumionen als Gegenionen enthält,
(b) einen Reinheitsgrad von linearem Polyphosphat von zumindest 83% aufweist, wobei das Polyphosphat Natriumionen und/oder Kaliumionen und Magnesiumionen als Gegenionen enthält,
(c) zumindest 70% (mol/mol) Kaliumionen und bis zu 30% (mol/mol) Magnesiumionen als Gegenionen enthält,
(d) eine durchschnittliche Kettenlänge von 12 P-Einheiten aufweist, wobei die durchschnittliche Kettenlänge enzymatisch bestimmt wird, und/oder
(e) einen Anteil an zyklischem Polyphosphat von nicht mehr als 5%, vorzugsweise nicht mehr als 2%, bezogen auf die Trockensubstanz aufweist.

**10.** Zusammensetzung nach einem der Ansprüche 7 bis 9, wobei die enzymatische Bestimmung der durchschnittlichen Kettenlänge von P-Einheiten des Polyphosphats colorimetrisch quantitativ unter Verwendung von Exopolyphosphatase, anorganischer Pyrophosphatase und einem Detektionsmittel durchgeführt wird, oder
wobei die enzymatische Bestimmung der durchschnittlichen Kettenlänge von P-Einheiten des Polyphosphats fluorometrisch quantitativ unter Verwendung von Exopolyphosphatase, ATP Sulfurlyase, Hexokinase und Glucose-6-phosphat-Dehydrogenase durchgeführt wird.

**11.** Zusammensetzung nach einem der Ansprüche 7 bis 10, wobei eine wässrige 1% (w/v) Lösung davon einen pH-Wert zwischen 6 und 8 aufweist.

**12.** Zusammensetzung nach einem der Ansprüche 7 bis 11, zur Verwendung als Medikament.

**13.** Lebensmittel, Lebensmittelzusatzstoff, Nahrungsergänzungsmittel, Futtermittel, Düngemittel, Lebensmittel-, Lebensmittelzusatzstoff-, Nahrungsergänzungsmittel-, Futtermittel-, Düngemittel-Zwischenprodukt oder pharmazeutische Zusammensetzung enthaltend eine Zusammensetzung nach einem der Ansprüche 7 bis 11.

**14.** Verwendung der Zusammensetzung nach einem der Ansprüche 7 bis 11 bei der Herstellung eines Lebensmittels, eines Lebensmittelzusatzstoffs, eines Nahrungsergänzungsmittels, eines Futtermittels, eines Düngemittels, eines Lebensmittel-, Lebensmittelzusatzstoff-, Nahrungsergänzungsmittel-, Futtermittel-, Düngemittel-Zwischenproduktes, einer pharmazeutischen Zusammensetzung oder einer Mischung davon.

## Claims

**1.** A method for obtaining polyphosphate from polyphosphate-containing yeast cells, comprising:

(1) disrupting polyphosphate-containing yeast cells after their harvest by freezing and thawing;
(2) incubating an aqueous solution containing the disrupted polyphosphate-containing yeast cells at a temperature of about 70°C to about 80°C for about 10 minutes, wherein about means within 20%;
(3) removing cell debris from the aqueous solution;
(4) removing nucleic acids and proteins from the aqueous solution by addition of an acid;
(5) neutralizing the remaining aqueous solution; and
(6) obtaining polyphosphate from the aqueous solution by precipitation using an alcohol.

**2.** The method according to claim 1, wherein in step (6) the precipitation using alcohol is a fractional precipitation.

**3.** The method according to claim 2, wherein the fractional precipitation is carried out by successive precipitations with increasing volume of the alcohol relative to the total volume of the aqueous solution.

**4.** The method according to claim 3, wherein after obtaining long-chain polyphosphate with an average chain length of 32-44 P-units, a second volume of the alcohol is added, wherein the second volume of the alcohol is greater than the first volume of the alcohol.

**5.** The method according to any one of claims 1 to 4, wherein in step (5) a base is used which contains a monovalent cation as counterion.

**6.** The method according to any one of claims 1 to 5, wherein in step (5) a base is used which contains

(a) sodium, or
(b) potassium

as a counterion.

**7.** A composition comprising a dried polyphosphate, wherein the dried polyphosphate:

(a) has a purity of at least 80%, defined as the content of linear polyphosphate and crystal water in the dry matter, wherein the polyphosphate contains sodium ions and/or potassium ions and magnesium ions as counterions,
(b) has a purity of linear polyphosphate of at least 80%, wherein the polyphosphate contains sodium ions and/or potassium ions and magnesium ions as counterions,
(c) contains at least 20% (mol/mol) sodium ions and/or at least 20% (mol/mol) potassium ions and up to 50% (mol/mol) magnesium ions as counterions,
(d) has an average chain length of 10-125 P-units, preferably 11-44 P-units, wherein the average chain length is enzymatically determined, and
(e) has a proportion of cyclic polyphosphate of not more than 5%, preferably not more than 2%, based on the dry matter.

**8.** The composition according to claim 7, wherein the dried polyphosphate:

(a) has a purity of at least 85%, defined as the content of linear polyphosphate and crystal water in the dry matter, wherein the polyphosphate contains sodium ions, potassium ions and magnesium ions as counterions,
(b) has a purity of linear polyphosphate of at least 85%, wherein the polyphosphate contains sodium ions and/or potassium ions and magnesium ions as counterions,
(c) contains at least 20% (mol/mol) sodium ions, at least 20% (mol/mol) potassium ions and up to 50% (mol/mol) magnesium ions as counterions,
(d) has an average chain length of 10-125 P-units, preferably 11-44 P-units, wherein the average chain length is enzymatically determined, and
(e) has a proportion of cyclic polyphosphate of not more than 5%, preferably not more than 2%, based on the dry matter.

**9.** The composition according to claim 7, wherein the dried polyphosphate:

(a) has a purity of at least 83%, defined as the content of linear polyphosphate and crystal water in the dry matter, wherein the polyphosphate contains potassium ions and magnesium ions as counterions,
(b) has a purity of linear polyphosphate of at least 83%, wherein the polyphosphate contains sodium ions and/or potassium ions and magnesium ions as counterions,
(c) contains at least 70% (mol/mol) potassium ions and up to 30% (mol/mol) magnesium ions as counterions,
(d) has an average chain length of 12 P-units, wherein the average chain length is enzymatically determined, and/or
(e) has a proportion of cyclic polyphosphate of not more than 5%, preferably not more than 2%, based on the dry matter.

**10.** The composition according to any one of claims 7 to 9, wherein the enzymatic determination of the average chain length of P-units of the polyphosphate is carried out colorimetrically and quantitatively using exopolyphosphatase, inorganic pyrophosphatase and a detection agent, or
wherein the enzymatic determination of the average chain length of P-units of the polyphosphate is carried out fluorometrically and quantitatively using exopolyphosphatase, ATP sulfurylase, hexokinase and glucose-6-phosphate dehydrogenase.

**11.** The composition according to any one of claims 7 to 10, wherein an aqueous 1% (w/v) solution thereof has a pH value between 6 and 8.

**12.** The composition according to any one of claims 7 to 11, for use as a medicament.

**13.** A foodstuff, food additive, nutritional supplement, feed, fertilizer, foodstuff intermediate, food additive intermediate, nutritional supplement intermediate, feed intermediate, fertilizer intermediate or pharmaceutical composition comprising a composition according to any one of claims 7 to 11.

**14.** A use of the composition according to any one of claims 7 to 11 in the manufacture of a foodstuff, a food additive, a nutritional supplement, a feed, a fertilizer, a foodstuff intermediate, a food additive intermediate, a nutritional supplement intermediate, a feed intermediate, a fertilizer intermediate, a pharmaceutical composition or a mixture thereof.

## Revendications

**1.** Un procédé d'obtention de polyphosphate à partir de cellules de levure contenant du polyphosphate, comprenant:

(1) la lyse de cellules de levure contenant du polyphosphate après leur récolte par congélation et décongélation;
(2) l'incubation d'une solution aqueuse contenant les cellules de levure contenant du polyphosphate lysées à une température d'environ 70°C à environ 80°C pendant environ 10 minutes, "environ" signifiant dans une plage de 20 %;
(3) l'élimination des débris cellulaires de la solution aqueuse;
(4) l'élimination des acides nucléiques et des protéines de la solution aqueuse par ajout d'un acide;
(5) la neutralisation de la solution aqueuse restante; et
(6) l'obtention de polyphosphate à partir de la solution aqueuse par précipitation à l'aide d'un alcool.

**2.** Le procédé selon la revendication 1, dans lequel, à l'étape (6), la précipitation à l'aide d'un alcool est une précipitation fractionnée.

**3.** Le procédé selon la revendication 2, dans lequel la précipitation fractionnée est effectuée par des précipitations successives avec un volume croissant de l'alcool par rapport au volume total de la solution aqueuse.

**4.** Le procédé selon la revendication 3, dans lequel, après l'obtention de polyphosphate à longue chaîne ayant une longueur moyenne de chaîne de 32 à 44 unités P, un deuxième volume de l'alcool est ajouté, le deuxième volume de l'alcool étant supérieur au premier volume de l'alcool.

**5.** Le procédé selon l'une des revendications 1 à 4, dans lequel, à l'étape (5), une base est utilisée qui contient un cation monovalent comme contre-ion.

**6.** Le procédé selon l'une des revendications 1 à 5, dans lequel, à l'étape (5), une base est utilisée qui contient

a) du sodium, ou
b) du potassium

comme contre-ion.

**7.** Une composition contenant du polyphosphate séché, ledit polyphosphate séché

a) présentant un degré de pureté d'au moins 80 %, défini comme la teneur en polyphosphate linéaire et en eau de cristallisation dans la matière sèche, ledit polyphosphate contenant des ions sodium et/ou des ions potassium et des ions magnésium comme contre-ions,
b) présentant un degré de pureté en polyphosphate linéaire d'au moins 80 %, ledit polyphosphate contenant des ions sodium et/ou des ions potassium et des ions magnésium comme contre-ions,
c) contenant au moins 20 % (mol/mol) d'ions sodium et/ou au moins 20 % (mol/mol) d'ions potassium et jusqu'à 50 % (mol/mol) d'ions magnésium comme contre-ions,
d) présentant une longueur moyenne de chaîne de 10 à 125 unités P, de préférence de 11 à 44 unités P, ladite longueur moyenne étant déterminée de manière enzymatique, et
e) présentant une teneur en polyphosphate cyclique ne dépassant pas 5 %, de préférence ne dépassant pas 2 %, par rapport à la matière sèche.

**8.** La composition selon la revendication 7, dans laquelle le polyphosphate séché

a) présente un degré de pureté d'au moins 85 %, défini comme la teneur en polyphosphate linéaire et en eau de cristallisation dans la matière sèche, ledit polyphosphate contenant des ions sodium, des ions potassium et des ions magnésium comme contre-ions,
b) présente un degré de pureté en polyphosphate linéaire d'au moins 85 %, ledit polyphosphate contenant des ions sodium et/ou des ions potassium et des ions magnésium comme contre-ions,
c) contient au moins 20 % (mol/mol) d'ions sodium, au moins 20 % (mol/mol) d'ions potassium et jusqu'à 50 % (mol/mol) d'ions magnésium comme contre-ions,
d) présente une longueur moyenne de chaîne de 10 à 125 unités P, de préférence de 11 à 44 unités P, ladite longueur moyenne étant déterminée de manière enzymatique, et
e) présente une teneur en polyphosphate cyclique ne dépassant pas 5 %, de préférence ne dépassant pas 2 %, par rapport à la matière sèche.

**9.** La composition selon la revendication 7, dans laquelle le polyphosphate séché

a) présente un degré de pureté d'au moins 83 %, défini comme la teneur en polyphosphate linéaire et en eau de cristallisation dans la matière sèche, ledit polyphosphate contenant des ions potassium et des ions magnésium comme contre-ions,
b) présente un degré de pureté en polyphosphate linéaire d'au moins 83 %, ledit polyphosphate contenant des ions sodium et/ou des ions potassium et des ions magnésium comme contre-ions,
c) contient au moins 70 % (mol/mol) d'ions potassium et jusqu'à 30 % (mol/mol) d'ions magnésium comme contre-ions,
d) présente une longueur moyenne de chaîne de 12 unités P, ladite longueur moyenne étant déterminée de manière enzymatique, et/ou
e) présente une teneur en polyphosphate cyclique ne dépassant pas 5 %, de préférence ne dépassant pas 2 %, par rapport à la matière sèche.

**10.** La composition selon l'une des revendications 7 à 9, dans laquelle la détermination enzymatique de la longueur moyenne des chaînes d'unités P du polyphosphate est réalisée de manière colorimétrique et quantitative à l'aide d'une exopolyphosphatase, d'une pyrophosphatase inorganique et d'un agent de détection, ou
dans laquelle la détermination enzymatique de la longueur moyenne des chaînes d'unités P du polyphosphate est réalisée de manière fluorométrique et quantitative à l'aide d'une exopolyphosphatase, d'une ATP sulfurylase, d'une hexokinase et d'une glucose-6-phosphate déshydrogénase.

**11.** La composition selon l'une des revendications 7 à 10, dans laquelle une solution aqueuse à 1 % (p/v) de celle-ci présente une valeur de pH comprise entre 6 et 8.

**12.** La composition selon l'une des revendications 7 à 11, pour une utilisation en tant que médicament.

**13.** Un aliment, un additif alimentaire, un complément nutritionnel, un aliment pour animaux, un engrais, un intermédiaire alimentaire, un intermédiaire d'additif alimentaire, un intermédiaire de complément nutritionnel, un intermédiaire d'aliment pour animaux, un intermédiaire d'engrais ou une composition pharmaceutique contenant une composition selon l'une des revendications 7 à 11.

**14.** Une utilisation de la composition selon l'une des revendications 7 à 11 dans la fabrication d'un aliment, d'un additif alimentaire, d'un complément nutritionnel, d'un aliment pour animaux, d'un engrais, d'un intermédiaire alimentaire, d'un intermédiaire d'additif alimentaire, d'un intermédiaire de complément nutritionnel, d'un intermédiaire d'aliment pour animaux, d'un intermédiaire d'engrais, d'une composition pharmaceutique ou d'un mélange de ceux-ci.

**Abbildung 1**

A)
Rückgewinnung [%]
100
80
60
40
20
0
ohne NaCl
mit NaCl
2 vol. Ethanol
ohne NaCl
mit NaCl
2 vol. Propanol
ohne NaCl
mit NaCl
2 vol. Aceton
B)
Rückgewinnung [%]
NaCl
KCl
0
100
200
300
400
500
600
700
800
NaCl oder KCl Konzentration [mM]
C)
Rückgewinnung [%]
100 mM NaCl
100 mM KCl
0
0.5
1
1.5
2
2.5
3
Ethanolkonzentration [Volumen]
D)
Wassergehalt [% (w/w)]
60
50
40
30
20
10
0
Natrium PolyP-Gel
Kalium PolyP-Gel
1
2
3
4
5
6
7
Trockenzeit [Tage]

**Abbildung 2**

A)
Extraktionseffizienz (•) [%]
Kettenlänge (Δ) [P-Untereinheiten]
ml Wasser pro g feuchte Zellmasse
B)
Extraktionseffizienz (•) [%]
Kettenlänge (Δ) [P-Untereinheiten]
Inkubationszeit während der Hitzebehandlung [Min]
C)
Extraktionseffizienz (•) [%]
Kettenlänge (Δ) [P-Untereinheiten]
Temperatur während der Hitzebehandlung [°C]
D)
Extraktionseffizienz (•) [%]
Kettenlänge (Δ) [P-Untereinheiten]
NaOH-Konzentration währed der Hitzebehandlung [mM]

**Abbildung 2 (Fortsetzung)**

E)
Extraktionseffizienz (•) [%]
Kettenlänge (Δ) [P-Untereinheiten]
NaCl-Konzentration während der Hitzebehandlung [mM]
F)
Extraktionseffizienz (•) [%]
Kettenlänge (Δ) [P-Untereinheiten]
Verhältnis polyP zu Protein (◇)
HCl-Konzentration [mM]
G)
Extraktionseffizienz [%]
fraction 1
fraction 2
Ethanolvolumen für Fraktion 1 [vol]
H)
Kettenlänge (Δ) [P-Untereinheiten]
fraction 1
fraction 2
Ethanolvolumen für Fraktion 1 [vol]

Abbildung 3

Langes Na-bio-polyP (Charge 1)
(Charge 2)
Kurzes Na-bio-polyP (Charge 1)
(Charge 2)
Langes K-bio-polyP (Charge 1)
(Charge 2)
Kuzres K-bio-polyP (Charge 1)
(Charge 2)
Budit 4
PolyP von Roth
P100
Länge
840
649
524
477
428
374
314
246
207
162
107

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- GB 1161693 A
- WO 2013011996 A


### In der Beschreibung aufgeführte Nicht-Patentliteratur


- **SHEN et al.** *Meat Science*, 2010, vol. 84, 364
- **LISS** ; **LANGEN**. *Archiv für Mikrobiologie*, 1962, vol. 41, 383-392
- **CHRIST** ; **BLANK**. *FEMS Yeast Research*, 2019
- **CHRIST** ; **BLANK**. *Anal Biochem*, 2018, vol. 548, 82-90
- **F. M. HAROLD**. *Bacteriol. Rev.*, 1966, vol. 30, 772-794
- **S. KULAEV** ; **V. M. VAGABOV** ; **T. V. KULAKOVSKAYA**. The biochemistry of inorganic polyphosphates. John Wiley & Sons, Ltd, 2005
- **N. RAO** ; **M. R. GOMEZ-GARCIA** ; **A. KORNBERG**. *Annu. Rev. Biochem.*, 2009, vol. 78, 605-647
- **J. R. VAN WAZER** ; **K. A. HOLST**. *J. Am. Chem. Soc.*, 1950, vol. 72, 639-644
- **J. R. VAN WAZER**. Phosphorus and its compounds. Volume I: Chemistry. Interscience publishers, Inc., 1958
- **M. DÜRR** ; **K. URECH** ; **T. BOLLER** ; **A. WIEMKEN** ; **J. SCHWENCKE** ; **M. NAGY**. *Arch. Microbiol.*, 1979, vol. 121, 169-175
- **L. JACOBSON** ; **M. HALMANN** ; **J. YARIV**. *Biochem. J.*, 1982, vol. 201, 473-479
- **G. M. ROOMANS**. *Physiol. Plant.*, 1980, vol. 48, 47-50
- **S. KULAEV** ; **V. M. VAGABOV** ; **T. V. KULAKOVSKAYA**. The biochemistry of inorganic polyphosphates. John Wiley & Sons, Ltd, 2005, vol. 6
- **T. V. KULAKOVSKAYA** ; **V. M. VAGABOV** ; **I. S. KULAEV**. *Process Biochemistry*, 2012, vol. 47, 1-10
- **Q. W. SHEN** ; **D. R. SWARTZ**. *Meat science*, 2010, vol. 84, 364-364
- **J. E. CLARK** ; **H. G. WOOD**. *Anal. Biochem.*, 1987, vol. 161, 280-290
- **S. A. SMITH** ; **C. J. BAKER** ; **J. M. GAJSIEWICZ** ; **J. H. MORRISSEY**. *Blood*, 2017, vol. 130, 88-91
- **L. H. CRAGG** ; **H. HAMMERSCHLAG**. *Chem. Rev.*, 1946, vol. 39, 79-135
- **J. J. CHRIST** ; **L. M. BLANK**. *Anal. Biochem.*, 2018, vol. 548, 82-90
- **T. SHIBA**. Inorganic polyphosphates in eukaryotic cells. Springer International Publishing, 2016, 139-158
- **J. J. CHRIST** ; **L. M. BLANK**. *FEMS yeast research*, 2019, vol. 19
- **A. KURODA** ; **N. TAKIGUCHI** ; **T. GOTANDA** ; **K. NOMURA** ; **J. KATO** ; **T. IKEDA** ; **H. OHTAKE**. *Biotechnol. Bioeng.*, 2002, vol. 78, 333-338
- **N. TAKIGUCHI** ; **A. KURODA** ; **H. OHTAKE** ; **S. TSUNEDA**. Phosphorus Recovery and Recycling. Springer Singapore, 2019, 515-526
- **R. HIROTA** ; **A. KURODA** ; **J. KATO** ; **H. OHTAKE**. *J. Biosci. Bioeng.*, 2010, vol. 109, 423-432
- **CHRIST JJ** ; **WILLBOLD S** ; **BLANK LM**. *Analytical Chemistry*, 2019, vol. 91 (12), 7654-7661
- **CHRIST** ; **BLANK**. *Anal Biochem*, 2018, vol. 563, 71-78
- **LISS** ; **LANGEN**. *Arch. Mikrobiol.*, 1962, vol. 41, 383-392
- **PROTOKOLL VON BRU et al.** *Microbial Cell*, 2016, vol. 4, 6-15
- **BRU et al.** *Microbial Cell*, 2016, vol. 4, 6-15
- **S. KULAEV** ; **V. M. VAGABOV** ; **T. V. KULAKOVSKAYA**. The biochemistry of inorganic polyphosphates. John Wiley & Sons, Ltd, 2005, 602-604
- **S. OMELON** ; **W. HABRAKEN**. Inorganic polyphosphates in eukaryotic cells. Springer International Publishing, 2016, 177-205
- **E. C. DE OLIVEIRA** ; **LIMA, G. B. ALCANTARA** ; **F. C. DAMASCENO** ; **J. M. M. NETO** ; **F. GALAMBECK**. *Quim. Nova.*, 2010, vol. 33, 1991-1995
- **J. R. VAN WAZER**. *J. Am. Chem. Soc.*, 1950, vol. 72, 647-655
- **L. K. SEIDLMAYER** ; **M. R. GOMEZ-GARCIA** ; **T. SHIBA** ; **G. A. PORTER, JR.** ; **E. V. PAVLOV** ; **D. M. BERS** ; **E. N. DEDKOVA**. *Arch. Biochem. Biophys.*, 2019, vol. 662, 177-189

- **T. SHIBA** ; **Y. TAKAHASHI** ; **T. UEMATSU** ; **Y. KAWAZOE** ; **K. OOI** ; **K. NASU** ; **H. ITOH** ; **H. TANAKA** ; **M. YAMAOKA** ; **M. SHINDOH**. *Key Engineering Materials*, 2004, vol. 254-256, 1119-1122
- **S. KULAEV** ; **V. M. VAGABOV** ; **T. V. KULAKOVSKAYA**. The biochemistry of inorganic polyphosphates. John Wiley & Sons, Ltd, 2005, 671
- **N. GILBERT**. *Nature*, 2009, vol. 461, 716-718
- **L. CARRARESI** ; **S. BERG** ; **S. BRÖRING**. *Journal of Cleaner Production*, 2018, vol. 183, 87-101
- **K. R. HERRMANN** ; **A. J. RUFF** ; **B. INFANZON** ; **U. SCHWANEBERG**. *Appl. Microbiol. Biotechnol.*, 2019, vol. 103, 6435-6448
- **CHRIST** ; **BLANK**. *FEMS Yeast Res.*, 2019, vol. 19 (3)
- **CHRIST et al.** *Anal Chem*, 2019, vol. 91, 7654-7661
- **J. J. CHRIST** ; **L. M. BLANK**. *Anal. Biochem.*, 2018, vol. 563, 71-78
- **J. J. CHRIST** ; **S. WILLBOLD** ; **L. M. BLANK**. *Anal. Chem.*, 2019, vol. 91, 7654-7661
- **S. A. SMITH** ; **Y. WANG** ; **J. H. MORRISSEY**. *Electrophoresis*, 2018, vol. 39, 2454-2459
- **A. KUNINAKA** ; **M. FUJIMOTO** ; **K. UCHIDA** ; **H. YOSHINO**. *Agric. Biol. Chem.*, 1980, vol. 44, 1821-1827
- **N. N. RAO** ; **M. R. GOMEZ-GARCIA** ; **A. KORNBERG**. *Annu. Rev. Biochem.*, 2009, vol. 78, 605-647
- **E. THILO** ; **W. WIEKER**. *Zeitschrift Fur Anorganische Und Allgemeine Chemie*, 1957, vol. 291, 164-185
- **E. THILO**. *Adv. Inorg. Chem.*, 1962, vol. 4, 1-75
- **E. C. DE OLIVEIRA LIMA** ; **G. B. ALCANTARA** ; **F. C. DAMASCENO** ; **J. M. M. NETO** ; **F. GALAMBECK**. *Quim. Nova.*, 2010, vol. 33, 1991-1995
- **K. SEIDLMAYER** ; **M. R. GOMEZ-GARCIA** ; **T. SHIBA** ; **G. A. PORTER, JR.** ; **E. V. PAVLOV** ; **D. M. BERS** ; **E. N. DEDKOVA**. *Arch. Biochem. Biophys.*, 2019, vol. 662, 177-189